(19) Europäisches Patentamt
European Patent Office
Office européen des brevets

(11) EP 2 135 525 A2

(12) DEMANDE DE BREVET EUROPEEN

(43) Date de publication:
**23.12.2009 Bulletin 2009/52**

(51) Int Cl.:
***A45D 40/26*** *(2006.01)* ***A61K 8/18*** *(2006.01)*

(21) Numéro de dépôt: **09162143.3**

(22) Date de dépôt: **08.06.2009**

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **10.06.2008 FR 0853853**

(71) Demandeur: **L'Oréal**
**75008 Paris (FR)**

(72) Inventeurs:
- **Jager Lezer, Nathalie**
**91370, Verrieres-le-Buisson (FR)**
- **Arditty, Stéphane**
**91160, Ballainvilliers (FR)**

(74) Mandataire: **Bernardi Tedesco, Céline Michèle Claude**
**L'Oréal**
**D.I.P.I.**
**25-29 Quai Aulagnier**
**92665 Asnieres-sur-Seine Cedex (FR)**

# (54) Ensemble de maquillage et/ou de soin des cils

(57) La présente invention concerne un ensemble de maquillage et/ou de soin des cils **caractérisé en ce qu**'il comprend :
- au moins une composition de maquillage et/ou de soin des cils comprenant des particules sous forme de plaquettes, au moins un tensioactif non ionique et/ou au moins un tensioactif ionique et/ou au moins un tensioactif polymérique, ou au moins un agent rhéologique de phase grasse,
- et au moins un applicateur comprenant un élément d'application et une source vibrante permettant de faire vibrer l'élément d'application.

EP 2 135 525 A2

## Description

La présente invention concerne un ensemble de maquillage et/ou de soin des matières kératiniques, notamment des fibres kératiniques, notamment des cils comprenant :

- au moins une composition cosmétique de maquillage et/ou de soin des cils comprenant des particules de forme plaquettaire, au moins un tensioactif non ionique et/ou au moins un tensioactif ionique et/ou au moins un tensioactif polymérique, ou au moins une huile structurée par au moins un agent rhéologique de phase grasse,
- au moins un applicateur comprenant un élément d'application, et des moyens pour, préalablement, simultanément ou postérieurement à son application sur lesdites fibres, faire vibrer ladite composition.

Il existe un besoin d'obtenir de bons effets de maquillage sur les cils et notamment de disposer d'un mascara donnant une bonne qualité de dépôt, une bonne séparation, et/ou un effet épaississant, afin d'atteindre un résultat plus ou moins naturel selon les souhaits de la consommatrice.

Il existe en particulier un besoin pour fournir des formules dont on peut, préalablement et/ou simultanément à l'application, modifier la rhéologie, notamment en en abaissant la viscosité, afin de permettre l'obtention d'un dépôt plus lisse et une meilleure définition du maquillage.

A l'aide d'un applicateur de type brosse ou peigne classique, il peut s'avérer délicat d'obtenir ce résultat.

La demande de brevet publiée sous le N° US2006/0032512 décrit un système vibrant pour appliquer du mascara sur les cils et dont un des effets revendiqués réside dans l'aptitude du système à modifier la rhéologie du mascara appliqué. Cette demande de brevet est totalement silencieuse sur les caractéristiques de la composition susceptibles, en combinaison avec un applicateur vibrant, de conduire à une telle modification de rhéologie. A l'expérience, il s'avère que toutes les compositions de mascara n'ont pas une rhéologie sensible à la vibration. En outre, dans cette demande de brevet, les modifications de viscosité dont il est fait état, et qui sont généralement inférieures à 10% ne peuvent pas, compte tenu de la méthode de mesure, être vraiment considérées comme étant significatives.

Les inventeurs ont maintenant constaté qu'un maquillage plus important du point de vue définition ou charge des cils peut être obtenu grâce à l'ensemble selon l'invention.

L'invention vise donc un moyen pour permettre d'atteindre facilement cet objectif de maquillage.

La présente invention a donc pour objet, selon un de ses aspects, un ensemble de maquillage et/ou de soin des matières kératiniques, en particulier des fibres kératiniques, notamment des cils ou des sourcils, comprenant :

- un récipient délimitant au moins un compartiment contenant au moins une composition de maquillage et/ou de soin desdites matières, en particulier des cils ou des sourcils, comprenant des particules de forme plaquettaire,
- au moins un applicateur comprenant un élément d'application pour appliquer la composition sur lesdites matières et
- des moyens pour, préalablement, simultanément ou postérieurement à son application sur lesdites matières kératiniques, faire vibrer ladite composition de maquillage.

La présente invention a également pour objet un ensemble de maquillage et/ou de soin des matières kératiniques, en particulier des fibres kératiniques, notamment des cils ou des sourcils, comprenant :

- un récipient délimitant au moins un compartiment contenant au moins une composition de maquillage et/ou de soin desdites matières, en particulier des cils ou des sourcils, comprenant au moins un tensioactif non ionique et/ou au moins un tensioactif ionique et/ou au moins un tensioactif polymérique,
- au moins un applicateur comprenant un élément d'application pour appliquer la composition sur lesdites matières et
- des moyens pour, préalablement, simultanément ou postérieurement à son application sur lesdites matières kératiniques, faire vibrer ladite composition de maquillage.

La présente invention a également pour objet un ensemble de maquillage et/ou de soin des matières kératiniques, en particulier des fibres kératiniques, notamment des cils ou des sourcils, comprenant :

- un récipient délimitant au moins un compartiment contenant au moins une composition de maquillage et/ou de soin desdites matières, en particulier des cils ou des sourcils, comprenant au moins une huile structurée par au moins un agent rhéologique de phase grasse et moins de 10% en poids par rapport au poids total de la composition de cire, de préférence moins de 5% en poids,
- au moins un applicateur comprenant un élément d'application pour appliquer la composition sur lesdites matières et
- des moyens pour, préalablement, simultanément ou postérieurement à son application sur lesdites matières kératiniques, faire vibrer ladite composition de maquillage. De préférence, selon cette alternative, la composition est exempte de cire.

La présente invention a également pour objet un ensemble de maquillage et/ou de soin des matières kératiniques, en particulier des fibres kératiniques, notamment des cils ou des sourcils, comprenant :

- un récipient délimitant au moins un compartiment contenant au moins une composition de maquillage et/ou de soin desdites matières, en particulier des cils ou des sourcils, comprenant au moins une huile structurée par au moins un agent rhéologique de phase grasse, ledit agent rhéologique de phase grasse étant choisi parmi les familles chimiques listées ci-après,
- au moins un applicateur comprenant un élément d'application pour appliquer la composition sur lesdites matières et
- des moyens pour, préalablement, simultanément ou postérieurement à son application sur lesdites matières kératiniques, faire vibrer ladite composition de maquillage. Selon cette alternative, de préférence, la composition utilisée contient moins de 10% en poids par rapport au poids total de la composition de cire, de préférence moins de 5% en poids, de préférence est exempte de cire.

Ces moyens sont de préférence constitués par une source vibrante permettant, préalablement, simultanément ou postérieurement à l'application sur les fibres de la composition, de faire vibrer cette dernière.

Avantageusement, la composition est une composition, notamment de mascara, à appliquer sur les cils, ladite composition étant soumise aux vibrations pendant et/ou postérieurement à son application de manière à conférer une bonne définition et/ou un bon effet chargeant aux cils.

Selon un mode de réalisation avantageux, la composition est mise en vibration au moyen de l'élément d'application lui-même, ce dernier étant couplé à une source vibrante. Aussi, avantageusement, la source vibrante est couplée audit élément d'application de manière à permettre la vibration de ce dernier avant application de la composition sur les fibres, lors de l'application de la composition sur les fibres, ou après.

Alternativement, l'application de la composition sur les cils et sa vibration sont réalisées au moyen de deux outils distincts, l'un assurant l'application de la composition sur les cils ou les sourcils, l'autre la vibration du dépôt ainsi réalisé.

La composition est de préférence un produit destiné à être appliqué sur les cils, par exemple un mascara.

Les inventeurs ont observé que sous l'effet de la vibration, la viscosité de la composition varie. En effet, la composition se retrouve soit plus épaisse, soit plus fluide. Dans le premier cas, ie quand la composition se trouve épaissie (viscosité augmentée), elle permet d'obtenir un bon épaississement des cils, et donc un bon effet de charge. Dans le second cas, ie quand la composition se retrouve fluidifiée (viscosité diminuée), elle permet d'obtenir un dépôt plus lisse, et une meilleure définition du maquillage, donc une meilleure qualité de dépôt et une meilleure séparation des cils.

L'application sur les cils ou les sourcils de la composition, couplée à une mise en vibration de la composition, soit simultanément à son dépôt, soit postérieurement, permet donc de faire varier la viscosité de cette dernière.

En particulier, l'ensemble selon l'invention permet d'obtenir un dépôt lisse et homogène, facile à appliquer, qui sépare et gaine les cils, et/ou qui donne un bon effet chargeant.

Selon un deuxième aspect, la présente invention concerne un procédé de maquillage et/ou de soin non thérapeutique des matières kératiniques, en particulier des fibres kératiniques, en particulier des cils ou des sourcils, qui consiste à faire vibrer simultanément ou postérieurement à son application sur lesdites matières kératiniques, une composition comprenant :

- des particules de forme plaquettaire, et/ou
- au moins un tensioactif non ionique et/ou au moins un tensioactif ionique et/ou au moins un tensioactif polymérique, et/ou
- au moins une huile structurée par au moins un agent rhéologique de phase grasse.

Le procédé selon l'invention peut donc consister à :

- appliquer sur lesdites matières kératiniques au moins une couche d'une composition de maquillage et/ou de soin,
- faire vibrer ladite composition préalablement, et/ou simultanément et/ou postérieurement à son application sur lesdites matières,
- ledit procédé étant **caractérisé en ce que** la composition comprend des particules de forme plaquettaire, et/ou au moins un tensioactif non ionique et/ou au moins un tensioactif ionique et/ou au moins un tensioactif polymérique, et/ou au moins une huile structurée par au moins un agent rhéologique de phase grasse.

De préférence, la composition est une composition de maquillage des cils, et elle est soumise aux vibrations pendant et/ou postérieurement à son application sur les cils de manière à conférer à ces derniers une bonne définition et/ou un bon effet chargeant avant que la composition ne sèche complètement.

La composition ainsi appliquée et mise en mouvement par un organe vibrant, permet d'obtenir dépôt lisse et homogène, facile à appliquer, qui sépare et gaine les cils, et/ou qui donne un bon effet chargeant.

Les compositions conformes à l'invention comprennent un milieu physiologiquement acceptable, notamment cosmétiquement acceptable, c'est-à-dire un milieu compatible en particulier avec les cils et la zone oculaire.

Par "cosmétiquement acceptable", on entend, dans le cadre de la présente invention, un composé dont l'utilisation est compatible avec une application sur les cils.

L'expression "comportant un" doit être considérée comme étant synonyme de l'expression "comportant au moins un", et l'expression "compris entre" doit être considérée comme incluant les bornes, sauf si le contraire est spécifié.

Toutes les teneurs en composants sont exprimées en matière sèche.

Les termes "moyen d'application" ou "applicateur" sont employés indifféremment dans la suite de la description.

La composition selon l'invention comprend, selon une première alternative, préférée, des particules de forme plaquettaire.

Par particule plaquettaire ou plaquette, on entend une particule dont l'une des dimensions est très inférieure aux deux autres. De telles plaquettes comportent le plus souvent une épaisseur E bien inférieure à leur longueur L1 ou largeur L2.

De préférence, le rapport E/L1 et E/L2 est inférieur ou égal à 0,5, de préférence inférieur ou égal à 0,3, de préférence inférieur ou égal 0,1.

Ces particules de forme plaquettaire sont notamment choisies parmi certains gélifiants et certaines charges, lesdites charges pouvant être non colorées ou colorées (ex : particules réfléchissantes).

Elles peuvent être présentes dans la composition en quantité allant de 0,5 à 60 % en poids par rapport au poids total de la composition, de préférence en quantité allant de 1 à 50 % en poids, de préférence en quantité allant de 1,5 à 25 % en poids.

En particulier, on utilisera une quantité allant de 1 à 7% en poids, de préférence 3 à 4% en poids par rapport au poids total de ladite composition, pour des particules de forme plaquettaire de longueur L1 moyenne inférieure à 500nm. C'est le cas notamment des argiles, telles que les hectorites, les bentonites, les silicates de magnésium, les montmorillonites décrites ci-après.

Dans le cas de particules de forme plaquettaire de longueur L1 moyenne supérieure à 500nm, on utilisera avantageusement une quantité desdites particules allant de 15 à 35% en poids, de préférence de 20 à 25% en poids par rapport au poids total de ladite composition. C'est le cas notamment des charges et particules réfléchissantes décrites ci-après, et en particulier du talc, mica, kaolin, lauroyl-lysine, particules réfléchissantes comportant une substrat de mica synthétique revêtu d'oxyde de titane et/ou d'oxydes de fer.

Parmi les gélifiants utilisables, on peut citer les argiles, lipophiles ou hydrophiles.

On entend par argile hydrophile une argile apte à gonfler dans l'eau ; cette argile gonfle dans l'eau et forme après hydratation une dispersion colloïdale.

Ces argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence. Les argiles sont des silicates contenant un cation pouvant être choisi parmi les cations de calcium, de magnésium, d'aluminium, de sodium, de potassium, de lithium et leurs mélanges. A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique.

Comme argile hydrophile, on peut citer les smectites telles que les saponites, les hectorites, les montmorillonites, les bentonites, la beidellite.

Comme argile hydrophile, on peut citer les hectorites synthétiques (appelées aussi laponites) comme les produits vendus par la société Laporte sous le nom Laponite XLG, Laponite RD, Laponite RDS (ces produits sont des silicates de sodium et de magnésium et en particulier des silicates de sodium, de lithium et de magnésium) ; les bentonites comme le produit vendu sous la dénomination Bentone HC par la société RHEOX ; les silicates de magnésium et d'aluminium notamment hydratés comme les produits vendus par la société Vanderbilt Company sous le nom Veegum ultra, Veegum HS, Veegum DGT, ou encore les silicates de calcium et notamment celui sous forme synthétique vendu par la société sous le nom de Micro-cel C.

On entend par argile lipophile une argile apte à gonfler dans un milieu lipophile; cette argile gonfle dans le milieu et forme ainsi une dispersion colloïdale.

Comme argiles lipophiles, on peut citer par exemple les argiles modifiées telles que le silicate de magnésium modifié (Bentone gel VS38 de RHEOX), les hectorites modifiées par un chlorure d'ammonium d'acide gras en $C_{10}$ à $C_{22}$, comme l'hectorite modifiée par le chlorure de distéaryl diméthyl ammonium (nom CTFA : Disteardimonium hectorite) commercialisé sous la dénomination « Bentone 38 CE » par la société RHEOX ou Bentone 38V® par la société ELEMENTIS.

Parmi les charges utilisables, on peut citer celles bien connues de l'homme du métier et couramment utilisées

dans les compositions cosmétiques.

Les charges peuvent être minérales ou organiques, lamellaires ou plaquettaires.

On peut citer le talc, le mica, le sulfate de baryum, le kaolin, la lauroyl-lysine, l'amidon, le nitrure de bore, le carbonate de calcium précipité, le carbonate et l'hydrocarbonate de magnésium, l'hydroxyapatite, la montmorillonite (comme le Gel White H de Rookwood Additives), des particules de cire de polytétrafluoroéthylène (PTFE) (comme Ceridust 9205 F de Clariant, ou Fluoropure 103 C de Shamrock Technologies), le sulfate de calcium (comme Prestia PR306 de Lafarge Prestia), la poudre de pierre ponce (comme O-D ponce décontaminée d'Eyraud), l'oxychlorure de bismuth, la poudre d'oxychlorure de bismuth et d'oxyde de zinc (comme Pearl II UCR de Farmaquimia), la perlite (comme Optima 1430 OR de World Minerals), des particules de verre notamment de taille d'environ 10 microns et d'épaisseur d'environ 0,4 microns, comme celles commercialisées sous les références MTD010FYX(6001) ou MTD010FYX(6009) par Nippon Sheet Glass, de taille d'environ 25 microns et d'épaisseur d'environ 0,4 microns, comme celles commercialisées sous les références MTD025FYX(6002) ou MTD025FYX(6010) par Nippon Sheet Glass, des particules sol/gel de silice et dioxyde de titane (comme NLT30H2WA de Nippon Sheet Glass), des particules de mica et de dioxyde de titane comme Blancsealer de Merck, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium, ou encore des particules réfléchissantes multicouches plaquettaires, en particulier les particules réfléchissantes comportant un substrat de mica synthétique revêtu de dioxyde de titane et/ou d'oxyde de fer noir, et leurs mélanges.

Les particules de sulfate de baryum mentionnées ci-dessus peuvent être enrobées d'acide aminé N-acylé tels que ceux comprenant un groupe acyle ayant de 8 à 22 atomes de carbones, comme par exemple un groupe 2-éthyl hexanoyle, caproyle, lauroyle, myristoyle, palmitoyle, stéaroyle, cocoyle. L'acide aminé peut être par exemple la lysine, l'acide glutamique, l'alanine. De préférence, les particules de sulfate de baryum sont enrobées de lauroyl lysine.

Les particules réfléchissantes multicouches plaquettaires permettent de rendre la réflexion plus directionnelle.

Les particules réfléchissantes sont par exemple à reflet métallique et comportent avantageusement au moins une couche conductrice de l'électricité en surface, formée d'au moins un métal ou oxyde métallique.

Les particules réfléchissantes à reflet métallique, quelle que soit leur forme, comportent par exemple au moins une couche ayant de préférence une épaisseur uniforme, notamment d'un matériau réfléchissant, avantageusement un composé métallique.

Les particules réfléchissantes selon l'invention peuvent par exemple comporter un substrat naturel ou synthétique, notamment un substrat synthétique au moins partiellement enrobé par au moins une couche d'un matériau réfléchissant, notamment au moins une couche d'au moins un composé métallique tel qu'un métal ou alliage. Le substrat peut être monomatière, multimatériau, organique et/ou inorganique. Plus particulièrement, le substrat peut être choisi parmi les verres, les céramiques, le graphite, les oxydes métalliques, les alumines, les silices, les silicates, notamment les aluminosilicates et les borosilicates, le mica synthétique et leurs mélanges, cette liste n'étant pas limitative.

On peut citer comme particules réfléchissantes selon l'invention les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, et les pigments nacrés à base d'oxychlorure de bismuth.

A titre d'exemple de particules réfléchissantes comportant un substrat minéral enrobé d'une couche de métal, on peut citer les particules comportant un substrat de borosilicate enrobé d'argent. Des particules à substrat de verre revêtu d'argent, en forme de plaquettes, sont vendues sous la dénomination de MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société. Des particules à substrat de verre revêtu de d'oxyde de titane sont vendues sous la dénomination METASHINE MC1120RY par la société Nippon Sheet Glass ou REFLECKS Dimensions Glittering Gold G230S par la société ENGELHARD. Des particules à substrat de verre revêtu de d'oxyde de titane à reflets argentés sont vendues sous la dénomination METASHINE MC1020RS par la société Nippon Sheet Glass ou REFLECKS Dimensions Luminous White G130M par la société ENGELHARD.

Les particules réfléchissantes à reflet métallique, qu'elles soient de forme lamellaire ou plaquettaire, peuvent également être choisies parmi les particules à substrat synthétique enrobé au moins partiellement d'au moins une couche d'au moins un oxyde métallique, choisi par exemple parmi les oxydes de titane, notamment $Ti0_2$, de fer notamment $Fe_20_3$, d'étain, de chrome, le sulfate de baryum et les matériaux suivants : $MgF_2$, $CrF_3$, ZnS, ZnSe, $Si0_2$, $Al_20_3$, Mg0, $Y_20_3$, $Se0_3$, Si0, $Hf0_2$, $Zr0_2$, $Ce0_2$, $Nb_20_5$, $Ta_20_5$, $MoS_2$ et leurs mélanges.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS® par la société ENGELHARD. On peut citer également les particules de mica recouvertes de dioxyde de titane et d'oxyde de fer comme FLAMENCO SATIN GOLD 260 M d'Engelhard, les particules de silice recouvertes d'oxyde de titane comme Xirona Kiwi Rose de Merck.

Comme autres exemples de particules comportant un substrat de mica synthétique revêtu de dioxyde de titane et/ou d'oxyde de fer noir, on peut citer notamment les particules de mica-oxyde de titane-oxyde de fer noir (58% CI77019 + 18% CI77891 + 24% CI77499) commercialisées sous la dénomination COLORONA PATINA SILVER par la société MERCK ; les particules de mica-oxyde de titane (52,5% CI77019 + 47,5% CI77891) commercialisées sous la dénomination FLAMENCO BLUE 620C par la société BASF PERSONAL CARE INGREDIENTS ; les particules de mica-oxyde de fer (52% CI77019 + 48% CI77499) commercialisées sous la dénomination COLORINA BLACKLSTAR RED par la société MERCK ; les particules de mica-oxyde de titane-oxyde d'étain-oxyde de fer (48% CI77019 + 36% CI77891 +1% CI77861 + 15% CI77499) commercialisées sous la dénomination FLAMENCO TWILIGHT GOLD 230ZB par la société BASF PERSONAL CARE INGREDIENTS.

Selon un mode préféré, on utilisera les particules de mica-oxyde de titane-oxyde de fer noir (58% CI77019 + 18% CI77891 + 24% CI77499) commercialisées sous la dénomination COLORONA PATINA SILVER par la société MERCK.

Avantageusement, la quantité desdites particules réfléchissantes de type mica-oxyde de titane-oxyde de fer noir pourra aller de 15 à 35% en poids, de préférence de 20 à 25% en poids par rapport au poids total de la composition de l'invention.

Comme autres exemples de particules réfléchissantes à reflet métallique lamellaires ou plaquettaires présentant en surface un composé métallique ou incluant au moins un composé métallique enrobé, on peut citer les particules proposées sous les dénominations METASHINE® ME 2040 PS, METASHINE® MC5090 PS ou METASHINE® MC280GP (2523) par la société NIPPON SHEET GLASS, SILVER FLAKE® JV6 ou GOLD POWDER® A1570 par la société ENGELHARD, STARLIGHT REFLECTIONS FXM® par la société ENERGY STRATEGY ASSOCIATES INC. BRIGHT SILVER® 1 E 0.008X0.008 par la société MEADOWBROOK INVENTIONS, ULTRAMIN® (ALUMINIUM POUDRE FINE LIVING), et COSMETIC METALLIC POWDER VISIONAIRE BRIGHT SILVER SEA®, COSMETIC METALLIC POWDER VISIONAIRE NATURAL GOLD® (60314) ou COSMETIC METALLIC POWDER VISIONAIRE HONEY® (60316) par la société ECKART, ainsi que XIRONA SILVER de Merck, RONASTAR AQUA ou RONASTAR RED de Merck.

Les particules réfléchissantes à reflet métallique peuvent réfléchir le spectre visible de manière sensiblement uniforme, comme c'est le cas par exemple de particules revêtues d'un métal tel que l'argent ou l'aluminium, ou non, ce qui peut alors conduire par exemple à une reflet métallique ayant un ton non neutre, jaune, rose, rouge, bronze, orangé, brun, or et/ou cuivré, selon la nature par exemple du composé métallique en surface.

La composition selon l'invention comprend, selon une seconde alternative, au moins un tensioactif non ionique et/ou au moins un tensioactif ionique et/ou au moins un tensioactif polymérique.

De préférence, la composition comprend au moins un tensioactif non ionique. De préférence, la composition comprend au moins un tensioactif non ionique de HLB supérieure ou égale à 8 à 25 °C. De préférence, la composition comprend à la fois au moins un tensioactif non ionique et au moins un tensioactif ionique, de préférence anionique.

Selon l'invention, on utilise généralement un tensioactif choisi de manière appropriée pour l'obtention d'une émulsion cire dans eau ou huile-dans-eau. En particulier, on peut utiliser un émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Ces tensioactifs peuvent être choisis parmi des tensioactifs non ioniques, anioniques, cationiques, amphotériques ou encore des émulsionnants tensioactifs. On peut se reporter au document "Encyclopedia of Chemical Technology, KIRK-OTHMER", volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.

Les tensioactifs utilisés préférentiellement dans la composition selon l'invention sont choisis parmi :

a) les tensioactifs non ioniques de HLB supérieur ou égal à 8 à 25 °C, utilisés seuls ou en mélange; on peut citer notamment :

- les esters et éthers d'oses tels que le mélange de cétylstéaryl glucoside et d'alcools cétylique et stéarylique comme le Montanov 68 de Seppic;
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30"), l'éther oxyéthyléné de l'alcool stéarylique à 20 groupes oxyéthylénés (nom CTFA "Steareth-20"), et l'éther oxyéthyléné du mélange d'alcools gras en C12-C15 comportant 7 groupes oxyéthylénés (nom CTFA "C12-15 Pareth-7") commercialisé sous la dénomination NEODOL 25-7® par SHELL CHEMICALS,
- les esters d'acide gras (notamment d'acide en C8-C24, et de préférence en C16-C22) et de polyéthylène glycol

(pouvant comprendre de 1 à 150 motifs d'éthylèneglycol) tels que le stéarate de PEG-50 et le monostéarate de PEG-40 commercialisé sous le nom MYRJ 52P® par la société ICI UNIQUEMA,

- les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,
- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 20 vendu sous la dénomination Tween 20® par la société CRODA, le polysorbate 60 vendu sous la dénomination Tween 60® par la société CRODA,
- la diméthicone copolyol, telle que celle vendue sous la dénomination Q2-5220® par la société DOW CORNING,
- la diméthicone copolyol benzoate (FINSOLV SLB 101® et 201® de la société FINTEX),
- les copolymères d'oxyde propylène et d'oxyde d'éthylène, également appelés polycondensats OE/OP,
- et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H-(O-CH_2-CH_2)_a-(O-CH(CH_3)-CH_2)_b-(O-CH_2-CH_2)_a-OH,$$

formule dans laquelle a va de 2 à 120, et b va de 1 à 100.

Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC® comme les SYNPERONIC PE/ L44® et SYNPERONIC PE/F127® par la société ICI.

b) les tensioactifs non ioniques de HLB inférieur à 8 à 25 °C, éventuellement associés à un ou plusieurs agents tensioactif non ioniques de HLB supérieur à 8 à 25 °C, tels que cités ci-dessus tels que :

- les esters et éthers d'oses tels que les stéarate de sucrose, cocoate de sucrose, stéarate de sorbitan et leurs mélanges comme l'Arlatone 2121® commercialisé par la société ICI ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool stéarylique à 2 groupes oxyéthylénés (nom CTFA "Steareth-2") ;
- les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
- les lécithines, telles que les lécithines de soja (comme Emulmetik 100 J de Cargill, ou Biophilic H de Lucas Meyer) ;
- le mélange de cyclométhicone/diméthicone copolyol vendu sous la dénomination Q2-3225C® par la société DOW CORNING.

c) les tensioactifs anioniques tels que :

- les sels d'acides gras en C16-C30 notamment ceux dérivant des amines, comme le stéarate de triéthanolamine et/ou le stéarate d'amino-2-méthyl-2-propane di-ol-1,3 ;
- les sels d'acides gras polyoxyéthylénés notamment ceux dérivant des amines ou les sels alcalins, et leurs

mélanges ;

- les esters phosphoriques et leurs sels tels que le "DEA oleth-10 phosphate" (Crodafos N 10N de la société CRODA) ou le phosphate de monocétyle monopotassique (Amphisol K de Givaudan) ;
- les sulfosuccinates tels que le "Disodium PEG-5 citrate lauryl sulfosuccinate" et le "Disodium ricinoleamido MEA sulfosuccinate" ;
- les alkyléthersulfates tels que le lauryl éther sulfate de sodium ;
- les iséthionates ;
- les acylglutamates tels que le "Disodium hydrogenated tallow glutamate" (AMISOFT HS-21 R® commercialisé par la société AJINOMOTO) et le sodium stearoyl glutamate (AMISOFT HS-11 PF® commercialisé par la société AJINOMOTO) et leurs mélanges ;
- les dérivés de soja comme le potassium soyate ;
- les citrates, comme le Glyceryl stearate citrate (Axol C 62 Pellets de Degussa) ;
- les dérivés de proline, comme le Sodium palmitoyl proline (Sepicalm VG de Seppic), ou le Mélange de Sodium palmitoyl sarcosinate, Magnesium palmitoyl glutamate, Palmitic acid et Palmitoyl proline (Sepifeel One de Seppic) ;
- les lactylates, comme le Sodium stearoyl lactylate (Akoline SL de Karlshamns AB) ;
- les sarcosinates, comme le sodium palmitoyl sarcosinate (Nikkol sarcosinate PN) ou le mélange de Stéaroyl sarcosine et Myristoyl sarcosine 75/25 (Crodasin SM de Croda);
- les sulfonates, comme le Sodium C14-17 alkyl sec sulfonate (Hostapur SAS 60 de Clariant) ;
- les glycinates, comme le sodium cocoyl glycinate (Amilite GCS-12 d'Ajinomoto).

Convient tout particulièrement à l'invention, le stéarate de triéthanolamine. Ce dernier est généralement obtenu par simple mélange de l'acide stéarique et de la triéthanolamine.

Les compositions conformes à l'invention peuvent également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL.

Le tensioactif utilisable peut également être un tensioactif polymérique, notamment un polymère thermogélifiant.

Les polymères thermogélifiants selon l'invention sont hydrosolubles et comprennent des unités hydrosolubles et des unités présentant dans l'eau une température inférieure critique de démixtion LCST, la température de démixtion par chauffage en solution aqueuse desdites unités à LCST étant de 5 à 40°C pour une concentration massique dans l'eau de 1 % desdites unités et la concentration dudit polymère dans ladite composition étant telle que sa température de gélification soit dans la plage de 5 à 40°C.

On entend par « polymère hydrosoluble » généralement un polymère soluble dans l'eau, à une température de 5 à 80°C, à raison d'au moins 10 g/l, de préférence d'au moins 20 g/l. Toutefois, on entend également par polymère hydrosoluble » un polymère ne possédant pas obligatoirement la solubilité ci-dessus mentionnée, mais qui en solution aqueuse à 1 % en poids, de 5 à 80°C, permet l'obtention d'une solution macroscopiquement homogène et transparente, c'est-à-dire ayant une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %. Par « unités hydrosolubles », on entend généralement que ces unités sont des unités solubles dans l'eau, à une température de 5 à 80°C, à raison d'au moins 10 g/l, de préférence d'au moins 20 g/l. Toutefois, on entend également par unités hydrosolubles des unités ne possédant pas obligatoirement la solubilité ci-dessus mentionnée, mais qui en solution aqueuse à 1 % en poids, de 5 à 80°C, permettent l'obtention d'une solution macroscopiquement homogène et transparente, c'est-à-dire ayant une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %. Ces unités hydrosolubles ne présentent pas de température de démixtion par chauffage de type LCST.

A ce propos, il est utile de rappeler que par unités à LCST, on entend de préférence des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère constitué uniquement d'unités à LCST est appelée « LCST » (Lower Critical Solution Température). Pour chaque concentration en polymère à LCST, une température de démixtion par chauffage est observée. Elle est supérieure à la LCST qui est le point minimum de la courbe. En dessous de cette température, le polymère est soluble dans l'eau, au-dessus de cette température, le polymère perd sa solubilité dans l'eau.

Ces unités à LCST du polymère ont, selon l'invention, de préférence, une température de démixtion par chauffage de 5 à 40°C pour une concentration massique dans l'eau de 1 % en poids desdites unités à LCST.

Plus préférentiellement, la température de démixtion par chauffage en solution aqueuse des unités à LCST du polymère est de 10 à 35°C pour une concentration massique dans l'eau de 1 % desdites unités à LCST. Plus préférentiellement, la concentration du polymère est telle que la température de gélification soit dans la plage de 10 à 35°C.

Le polymère ayant la structure décrite plus haut avec des unités hydrosolubles et des unités à LCST spécifiques définies ci-dessus présente en solution aqueuse, des propriétés de gélification au-delà d'une température critique, ou propriétés de thermogélification.

Ces propriétés de gélification par chauffage observées au-delà de la température de démixtion des chaînes à LCST sont décrites notamment dans les documents suivants :

[1] D. HOURDET et al., Polymer, 1994, vol. 35, n° 12, pages 2 624-2 630.
[2] F. L'ALLORET et al., Coll. Polym. Sci., 1995, vol. 273, n° 12, pages 1 163-1 173.
[3] F. L'ALLORET, Revue de l'Institut Français du Pétrole, 1997, vol. 52, n° 2, pages 117-128.

Elles sont dues à l'association des chaînes à LCST au sein de microdomaines hydrophobes au-delà de leur température de démixtion, formant ainsi des noeuds de réticulation entre les chaînes principales.

Ces propriétés gélifiantes sont observées lorsque la concentration en polymère est suffisante pour permettre des interactions entre des greffons à LCST portées par des macromolécules différentes. La concentration minimale nécessaire, appelée « concentration critique d'agrégation ou CAC », est évaluée par des mesures de rhéologie : il s'agit de la concentration à partir de laquelle la viscosité d'une solution aqueuse des polymères de l'invention devient supérieure à la viscosité d'une solution du polymère équivalent ne comportant pas de chaînes à LCST.

Au-delà de la CAC, les polymères de l'invention présentent de préférence des propriétés de gélification lorsque la température devient supérieure à une valeur critique appelée « température de gélification ou Tgel ». D'après les données de la littérature, un bon accord existe entre Tgel et la température de démixtion des chaînes à LCST, dans les mêmes conditions de concentrations. La température de gélification d'une solution aqueuse d'un polymère de l'invention est déterminée par des mesures de rhéologie : il s'agit de la température à partir de laquelle la viscosité de la solution d'un polymère de l'invention devient supérieure à la viscosité d'une solution du polymère équivalent ne comportant pas de chaînes à LCST.

Les polymères de l'invention de préférence se caractérisent par une température de gélification spécifique généralement de 5 à 40°C, de préférence de 10 à 35°C, pour une concentration massique dans l'eau, par exemple égale à 2 % en poids.

Les polymères utilisés dans l'invention peuvent être des polymères séquencés (ou blocs), ou des polymères greffés, qui comprennent, d'une part, des unités hydrosolubles et, d'autre part, des unités à LCST telles que définies ci-dessus.

On précise que, dans le présent texte, les unités hydrosolubles ou les unités à LCST des polymères mis en oeuvre selon l'invention sont définies comme n'incluant pas les groupes liant entre elles, d'une part, lesdites unités hydrosolubles et, d'autre part, lesdites unités à LCST.

Lesdits groupes de liaison sont issus de la réaction, lors de la préparation du polymère, des sites réactifs portés, d'une part, par les précurseurs desdites unités hydrosolubles et, d'autre part, par les précurseurs desdites unités à LCST.

Les polymères employés dans le cadre de l'invention peuvent donc être des polymères séquencés, comprenant par exemple des séquences constituées d'unités hydrosolubles alternées avec des séquences à LCST.

Ces polymères peuvent également se présenter sous la forme de polymères greffés dont le squelette est formé d'unités hydrosolubles, ledit squelette étant porteur de greffons constitués d'unités à LCST.

Lesdits polymères peuvent être partiellement réticulés.

Ces unités hydrosolubles sont en totalité ou en partie susceptibles d'être obtenues par polymérisation, notamment radicalaire, ou par polycondensation, ou encore sont constituées en totalité ou en partie par des polymères naturels ou naturels modifiés, existants.

A titre d'exemple, les unités hydrosolubles sont en totalité ou en partie susceptibles d'être obtenus par polymérisation, notamment radicalaire, d'au moins un monomère choisi parmi les monomères suivants :

- l'acide (méth)acrylique ;
- les monomères vinyliques de formule (I) suivante :

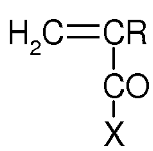

(I)

dans laquelle :

- R est choisi parmi H, $-CH_3$, $-C_2H_5$ ou $-C_3H_7$ ; et
- X est choisi parmi :

- les oxydes d'alkyle de type -OR' où R' est un radical hydrocarboné, linéaire

ou ramifié, saturé ou insaturé, ayant de 1 à 6 atomes de carbone, éventuellement substitué par au moins un atome d'halogène (iode, brome, chlore, fluor) ; un groupement sulfonique ($-SO_3$-), sulfate ($-SO_4$-), phosphate ($-PO_4H_2$) ; hydroxy (-OH) ; amine primaire (-NH2) ; amine secondaire ($-NHR_1$), tertiaire ($-NR_1R_2$) ou quaternaire ($-N+R_1R_2R_3$) avec $R_1$, $R_2$ et $R_3$ étant, indépendamment l'un de l'autre, un radical hydrocarboné, linéaire ou ramifié, saturé ou insaturé ayant 1 à 6 atomes de carbone, sous réserve que la somme des atomes de carbone de R' + $R_1$ + $R_2$ + $R_3$ ne dépasse pas 7 ; et

- les groupements $-NH_2$, $-NHR_4$ et $-NR_4R_5$ dans lesquels $R_4$ et $R_5$ sont indépendamment l'un de l'autre des radicaux hydrocarbonés, linéaires ou ramifiés, saturés

ou insaturés ayant 1 à 6 atomes de carbone, sous réserve que le nombre total d'atomes de carbone de R4 + R5 ne dépasse pas 7, lesdits $R_4$ et $R_5$ étant éventuellement substitués par un atome d'halogène (iode, brome, chlore, fluor) ; un groupement hydroxy (-OH) ; sulfonique ($-SO_3$-) ; sulfate ($-SO_4$-) ; phosphate ($-PO_4H_2$) ; amine primaire ($-NH_2$) ; amine secondaire ($-NHR_1$), tertiaire ($-NR_1R_2$) et/ou quaternaire ($-N+R_1R_2R_3$) avec R1, R2 et R3 étant, indépendamment l'un de l'autre, un radical hydrocarboné, linéaire ou ramifié, saturé ou insaturé ayant 1 à 6 atomes de carbone, sous réserve que la somme des atomes de carbone de $R_4$ + $R_5$ + $R_1$ + $R_2$ + $R_3$ ne dépasse pas 7 ;

- l'anhydride maléique ;
- l'acide itaconique ;
- l'alcool vinylique de formule $CH_2$=CHOH ;
- l'acétate de vinyle de formule $CH_2$=CH-$OCOCH_3$ ;
- les N-vinyllactames tels que la N-vinylpyrrolidone, la N-vinylcaprolactame et la N-butyrolactame ;
- les vinyléthers de formule $CH_2$=$CHOR_6$ dans laquelle $R_6$ est un radical hydrocarboné, linéaire ou ramifié, saturé ou insaturé, ayant de 1 à 6 carbones ;
- les dérivés hydrosolubles du styrène, notamment le styrène sulfonate ;
- le chlorure de diméthyldiallyl ammonium ; et
- la vinylacétamide.

Les polycondensats et les polymères naturels ou naturels modifiés qui peuvent constituer tout ou partie des unités hydrosolubles sont choisis parmi un ou plusieurs des composants suivants :

- les polyuréthanes hydrosolubles,
- la gomme de xanthane, notamment celle commercialisée sous les dénominations Keltrol T et Keltrol SF par Kelco ; ou Rhodigel SM et Rhodigel 200 de Rhodia ;
- les alginates (Kelcosol de Monsanto) et leurs dérivés tels que l'alginate de propylène glycol (Kelcoloid LVF de Kelco) ;
- les dérivés de cellulose et notamment la carboxyméthylcellulose (Aquasorb A500, Hercules), l'hydroxypropylcellulose, l'hydroxyéthylcellulose et l'hydroxyéthylcellulose quaternisée ;
- les galactomannanes et leurs dérivés, tels que la gomme Konjac, la gomme de guar, l'hydroxypropylguar, l'hydroxypropylguar modifié par des groupements méthylcarboxylate de sodium (Jaguar XC97-1, Rhodia), le chlorure de guar hydroxypropyl triméthyl ammonium.

On peut également citer la polyéthylène imine.

De préférence, les unités hydrosolubles ont une masse molaire allant de 1 000 g/mole à 5 000 000 g/mole lorsqu'elles constituent le squelette hydrosoluble d'un polymère greffé. Ces unités hydrosolubles ont de préférence une masse molaire allant de 500 g/mole à 100 000 g/mole lorsqu'elles constituent un bloc d'un polymère multiblocs.

On peut définir les unités à LCST des polymères utilisés dans l'invention, comme étant des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" (Lower Critical Solution Temperature). Pour chaque concentration en polymère, une température de démixtion par chauffage est observée ; elle est supérieure à la LCST qui est le point minimum de la courbe. En dessous de cette température, le polymère constituant l'unité à LCST est soluble dans l'eau ; au-dessus de cette température, le polymère constituant l'unité à LCST perd sa solubilité dans l'eau. Certains de ces polymères à LCST sont notamment décrits dans les articles suivants :

- TAYLOR et al, Journal of Polymer Science, part A : Polymer Chemistry, 1975, 13, 2 551 ;
- J. BAILEY et al, Journal of Applied Polymer Science, 1959, 1,56 ;
- HESKINS et al, Journal of Macromolecular Science, Chemistry A2, 1968, vol.8, 1441.

Par soluble dans l'eau à la température T, on entend que les unités présentent une solubilité à T, d'au moins 1 g/l, de préférence d'au moins 2 g/l.

La mesure de la LCST peut se faire visuellement : on détermine la température à laquelle apparaît le point de trouble de la solution aqueuse ; ce point de trouble se traduit par l'opacification de la solution, ou perte de transparence.

D'une manière générale, une composition transparente aura une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %. La transmittance peut être mesurée en plaçant un échantillon de 1 cm d'épaisseur dans le rayon lumineux d'un spectrophotomètre travaillant dans les longueurs d'onde du spectre lumineux.

Les unités à LCST des polymères utilisés dans l'invention peuvent être constituées par un ou plusieurs polymères choisis parmi les polymères suivants :

- les polyéthers tels que le polyoxyde de propylène (POP), les copolymères statistiques d'oxyde d'éthylène (OE) et d'oxyde de propylène (OP),
- les polyvinylméthyléthers,
- les dérivés polymériques et copolymériques N-substitués de l'acrylamide ayant une LCST, tels que le poly N-isopropylacrylamide (NIPAM) et le poly N-éthylacrylamide, et
- le polyvinylcaprolactame et les copolymères de vinyl caprolacatame.
  De préférence, les unités à LCST sont constituées de polyoxyde de propylène (POP)n où n est un nombre entier de 10 à 70, ou de copolymères statistiques d'oxyde d'éthylène (OE) et d'oxyde de propylène (OP), représentés par la formule :

$$(OE)m\ (OP)n$$

dans laquelle m est un nombre entier allant de 1 à 40, de préférence de 2 à 20, et n est un nombre entier allant de 10 à 60, de préférence de 20 à 50.

De préférence, la masse molaire de ces unités à LCST est de 500 à 5 300 g/mole, plus préférentiellement de 1 500 à 4 000 g/mole.

On a constaté que la répartition statistique des motifs OE et OP se traduit par l'existence d'une température inférieure critique de démixtion, au-delà de laquelle une séparation de phases macroscopique est observée. Ce comportement est différent de celui des copolymères (OE) (OP) à blocs, qui micellisent au-delà d'une température critique dite de micellisation (agrégation à l'échelle microscopique).

Les unités à LCST peuvent donc notamment être des poly oxydes de propylène tels que les Polyglycols P3000 et P4000 de Dow Chemical, des copolymères statistiques d'oxyde d'éthylène et d'oxyde de propylène, aminés, notamment monoaminés, diaminés ou triaminés. Ces polymères, avant réaction, sont porteurs de sites réactifs, dans ce cas de groupes aminés, réagissant avec les sites réactifs des polymères hydrosolubles, par exemple des groupes carboxyle, pour donner le polymère final mis en oeuvre dans l'invention. Dans le polymère final, les unités hydrosolubles sont liés aux unités à LCST par des groupes de liaison issus de la réaction des groupes ou sites réactifs portés respectivement par les unités à LCST et les précurseurs des unités hydrosolubles. Ces groupes de liaison seront, par exemple, des groupes amide, ester, éthers ou uréthanes.

Parmi ces polymères à LCST, commercialement disponibles, on peut citer les copolymères vendus sous la dénomination Jeffamine par HUNTSMAN, et notamment la Jeffamine XTJ-507 (M-2005), la Jeffamine D-2000 et la Jeffamine XTJ-509 (ou T-3000).

Les unités à LCST peuvent également être issues de copolymères OE/OP statistiques à extrémités OH, tels que ceux vendus sous la dénomination Polyglycols P41 et B11 par Clariant.

On peut aussi utiliser dans l'invention comme unités à LCST, les dérivés polymériques et copolymériques N-substitués de l'acrylamide ayant une LCST, ainsi que le polyvinyl caprolactame et les copolymères de vinylcaprolactame.

A titre d'exemples de dérivés polymériques et copolymériques N-substitués de l'acrylamide ayant une LCST, on peut citer le polyN-isopropylacrylamide, le poly N-éthylacrylamide et les copolymères de N-isopropylacrylamide (ou de N-éthylacrylamide) et d'un monomère vinylique choisi parmi les monomères ayant la formule (I) donnée ci-dessus, l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle.

La masse molaire de ces polymères est de préférence de 1 000 g/mole à 500 000 g/mole, de préférence de 2 000 à 50 000 g/mole.

La synthèse de ces polymères peut être réalisée par polymérisation radicalaire à l'aide d'un couple d'amorceurs tel que le chlorhydrate d'aminoéthanethiol, en présence de persulfate de potassium, afin d'obtenir des oligomères précurseurs ayant une extrémité réactive aminée.

A titre d'exemples de copolymères de vinylcaprolactame, on peut citer les copolymères de vinyl caprolactame et d'un monomère vinylique ayant la formule (I) donnée ci-dessus, ou d'un monomère choisi parmi l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

La masse molaire de ces polymères ou copolymères de vinylcaprolactame est généralement de 1 000 g/mole à 500 000 g/mole, de préférence de 2 000 à 50 000 g/mol.

La synthèse de ces composés peut être réalisée par polymérisation radicalaire à l'aide d'un couple d'amorceurs tel que le chlorhydrate d'aminoéthanethiol, en présence de persulfate de potassium, afin d'obtenir des unités à LCST ayant une extrémité réactive aminée.

La proportion massique des unités à LCST dans le polymère final est de préférence de 5 % à 70 %, notamment de 10 % à 60 %, et particulièrement de 20 % à 50 % en poids, par rapport au polymère final.

On a vu plus haut que la température de démixtion par chauffage desdites unités à LCST du polymère utilisé dans l'invention est de 5 à 40°C, de préférence de 10 à 35°C, pour une concentration massique dans l'eau, de 1 % en poids desdites unités à LCST.

Les polymères employés dans le cadre de l'invention peuvent être aisément préparés par l'homme du métier sur la base de ses connaissances générales, en utilisant des procédés de greffage, de copolymérisation ou de réaction de couplage.

Lorsque le polymère final se présente sous la forme d'un polymère greffé, notamment présentant un squelette hydrosoluble avec des chaînes latérales ou greffons à LCST, il est possible de le préparer par greffage des unités à LCST ayant au moins une extrémité réactive ou site réactif, notamment aminé(e), sur un polymère hydrosoluble formant le squelette, portant au minimum 10 % (en mole) de groupes réactifs tels que des fonctions acides carboxyliques. Cette réaction peut s'effectuer en présence d'un carbodiimide tel que le dicyclohexylcarbodiimide ou le chlorhydrate de 1-(3-diméthylaminopropyl)-3-éthyl carbodiimide, dans un solvant tel que la N-méthylpyrrolidone ou l'eau.

Une autre possibilité pour préparer des polymères greffés consiste à copolymériser par exemple un macromonomère à LCST (chaîne à LCST précédemment décrite avec une extrémité vinylique) et un monomère vinylique hydrosoluble tel que l'acide acrylique ou les monomères vinyliques ayant la formule (I).

Lorsque le polymère final se présente sous la forme d'un polymère séquencé ou à blocs, il est possible de le préparer par couplage entre des unités hydrosolubles et des unités à LCST, ces unités ayant à chaque extrémité des sites réactifs complémentaires.

Dans le cas des procédés de greffage et des procédés de couplage, les sites réactifs des unités à LCST peuvent être des fonctions amines notamment monoamines, diamines ou triamines, et des fonctions OH. Dans ce cas, les sites réactifs des unités hydrosolubles peuvent être des fonctions acides carboxyliques. Les groupes liant les unités hydrosolubles et les unités à LCST seront donc, par exemple, des groupes amide ou des groupes ester.

Les polymères thermogélifiants conformes à l'invention peuvent être choisis parmi ceux décrits dans les demandes de brevet et brevets suivants :

les demandes de brevet EP1307501, EP1355990, EP1355625, FR2856923, EP1493774 et WO04/006872 , les brevets US6,878,754 et US6,689,856 ; les demandes de brevet EP1407791,EP1416044, FR 2788008, WO 03/008462, FR2694939, EP0629649, US6645476, WO97/00275, WO98/06438,WO98/2948 WO98/48768, WO98/50005,WO00/07603,WO2/076392, FR2820976, WO00/35961,WO2/032560, EP0692506, US6870012, WO03/106536, WO00/38651,WO00/00222, WO01/41735, US2003/0099709, GB2408510.

Des polymères hydrosolubles thermogélifiants particulièrement intéressants peuvent être choisis parmi :

(1) les polyuréthanes comportant des groupement polyoxyde d'éthylène/polyoxypropylène/polyoxyde d'éthylène (ou POE-POP-POE) tels que ceux décrits dans les demandes EP-1407791 (exemple 1 décrit un polyuréthane issu de la polycondensation de Pluronic F-127 avec l'hexaméthylène diisocyanate), EP-A-692506 , FR-A-2840907, WO 03/106536, US-A-2005175573 , US-A-5702717.

De tels polyuréthanes sont obtenus de manière connus par polycondensation de diisocyanates et de diol triblocs POE-POP-POE thermosensibles et notamment décrits dans les demandes citées précédemment.

Comme diisocyanates, on peut citer les diisocyanates aliphatiques comme l'éthylène diisocyanate, l'hexamethylène diisocyanate, le décamethylène diisocyanate, et également le méthylène 4,4'-bis-dicyclohexyl diisocyanate, le diphénylméthane 4,4'-diisocyanate, le xylylène diisocyanate, le phenylène diisocyanate, le tolylène diisocyanate, le

dimethyl diphenylène diisocyanates.
Des diols triblocs POE-POP-POE utilisés peuvent répondre à la formule (I) suivant :

$$HO-(CH_2-CH_2-O)_x-(CH_2-CH(CH_3)-O)_y-(CH_2-CH_2-O)_x-H$$

avec $20<x<120$ et $20<y<120$
tels que les Pluronics, notamment le Pluronic F-127.
Le polyuréthane peut comprendre des groupements urées et/ou allophanates, comme décrit dans les demandes WO 03/106536 et US-A-5702717.
La polycondensation peut s'effectuer également en présence d'autres composés réactifs comme les diols comportant un ou plusieurs groupes acide carboxylique ou un groupe amine tertiaire (notamment amino méthyle)ou bien encore comme les polyoxyde d'éthylène monohydroxylés. Notamment, la polycondensation peut s'effectuer en présence d'eau. Le polyuréthane peut être linéaire ou ramifié.
(2) les copolymères multiblocs comprenant un bloc poly de N-isopropylamide et de -n-butylacrylate répartis de manière statistique et un bloc polyéthylèneglycol tels que ceux décrits dans les demandes EP-A-1407791. On peut en particulier utiliser le produit vendu sous la dénomination commerciale TGP-20 par la société MEBIOL.
(3) les copolymères d'acide acrylamidométhylpropane sulfonique (ou AMPS) tels que ceux décrits dans le brevet US6645476 et US6689856, ainsi que leurs sels (en particulier sels de sodium ou d'ammonium) et de macromonomère d'ester d'acide (méth)acrylique et d'alkyle en C2-C4 alcoxylés (en particulier oxyde d'éthylène (OE) et/ou oxyde de propylène (OP) (notamment à 1 à 500 motifs d'alkyle alcoxylé, plus préférentiellement de 3 à 50 et encore mieux 7 à 30 motifs) .
De tels macromonomères sont notamment choisis parmi les esters d'acide (méth)acrylique avec un éther de polyéthylène et propylène glycol ou encore un éther de polyglycol (8 à 25 OE) et d'alcool gras C10 à C22) notamment choisis parmi les Genapol C-080 ou UD-080, ou LA-070 ou LA-110 ou T-080 ou T-150 ou T-110 ou T-200 ou T-250 de chez Clariant.
De tels macromonomères peuvent également être issus de copolymères statistiques OE/OP aminés, notamment mono, di ou tri aminés de type Jeffamine de chez HUNTSMAN, et notamment la Jeffamine XTJ-507 (M-2005), la Jeffamine D-2000 et la Jeffamine XTJ-509 (ou T-3000).
De tels macromonomères peuvent également être issus de copolymères statistiques OE/OP à extrémités OH, tels que ceux vendus sous la dénomination Polyglycols P41 et B11 par Clariant. On utilisera plus particulièrement le copolymère d'acide polyacrylamido -2-méthylpropane sulfonique (AMPS) neutralisé par l'ammoniaque (40% en poids par rapport au poids total du polymère) et d'un macromonomère méthacrylate de polyéther (60 % en poids) dans lequel le polyéther est un copolymère statistique POE/POP comprenant 5,5 moles d'unités d'oxyde d'éthylène (OE) et 31 unités d'oxyde de propylène.
(4) les copolymères tels que décrits dans la demande de brevet EP1307501 constitués par un squelette d'acide polyacrylique (PAA) portant des chaînes latérales ou greffons constituées par des unités à LCST choisies parmi

(i) ceux du type de copolymères statistiques d'oxyde d'éthylène (OE) et d'oxyde de propylène (OP), représentés par la formule :

$$(OE)_m\,(OP)_n$$

dans laquelle m est un nombre entier allant de 1 à 40, de préférence de 2 à 20, et n est un nombre entier allant de 10 à 60, de préférence de 20 à 50 ; la masse molaire de ces unités à LCST étant de préférence de 500 à 5 300 g/mole, plus préférentiellement de 1 500 à 4 000 g/mole.

(ii) des polymères poly N-isopropylacrylamide dont la masse molaire est de préférence de 1 000 g/mole à 500 000 g/mole, plus préférentiellement de 2 000 à 50 000 g/mole.

L'agent tensioactif peut être présent en quantité allant de 0,1 à 30% en poids par rapport au poids total, de préférence en quantité allant de 0,5 à 20% en poids, de préférence en quantité allant de 1 à 15% en poids.
La composition selon l'invention comprend, selon une dernière alternative, au moins une huile structurée par au moins au moins un agent rhéologique de phase grasse.
L'agent rhéologique de phase grasse peut être choisi parmi :

- les polymères cristallins, de préférence choisis parmi les polymères semi-cristallins, les esters de dextrine et d'acide

gras, les polysaccharides modifiés hydrophobes, les copolymères d'oléfines cristallins et les polycondensats cristallins;

- les agents structurants lipophiles minéraux, comme les argiles lipophiles et les silices hydrophobes, comme la silice pyrogénée traitée hydrophobe,
- les polymères de type polyamide lipophiles,
- les polyurées et les polyuréthanes lipophiles,
- les polymères siliconés comprenant le cas échéant au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons, de préférence des groupes amides,
- les organogélateurs ;
- les polymères blocs ;
- les agents cristaux liquides cholestériques ;
- et leurs mélanges.

De préférence, l'agent rhéologique de phase grasse est choisi parmi les polymères semi-cristallins, les polymères de type polyamide lipophiles, et les polymères siliconés comprenant au moins un motif hydrocarboné comportant deux groupes capables d'établir des interactions hydrogènes choisis parmi les groupes amides.

On précise que, selon l'invention, dans le cas des associations d'un agent rhéologique de phase grasse avec une huile, on entend par « huile », un corps gras liquide à température ambiante.

On précise en outre que par « composé volatil » par exemple « huile volatile », on entend, au sens de l'invention, tout composé (ou milieu non aqueux) susceptible de s'évaporer au contact de la peau ou de la fibre kératinique en moins d'une heure, à température ambiante et pression atmosphérique. Le composé volatil est un composé cosmétique volatil, liquide à température ambiante, ayant notamment une pression de vapeur non nulle, à température ambiante et pression atmosphérique, notamment ayant une pression de vapeur allant de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1 300 Pa (0,01 à 10 mm de Hg).

Par opposition, on entend par « composé non volatil » par exemple « huile non volatile », un composé restant sur la peau ou la fibre kératinique à température ambiante et pression atmosphérique, au moins plusieurs heures et ayant notamment une pression de vapeur inférieure à $10^{-3}$ mm de Hg (0,13 Pa).

L'huile peut être choisie parmi les huiles hydrocarbonées et/ou siliconées et/ou fluorées volatiles et non volatiles et leurs mélanges. Ces huiles peuvent être d'origine animale, végétale, minérale ou synthétique. Par « huile hydrocarbonée », on entend une huile comportant principalement des atomes de carbone et d'hydrogène et éventuellement une ou plusieurs fonctions choisies parmi les fonctions hydroxyle, ester, éther, carboxylique. A titre d'exemple d'huile utilisable dans l'invention, on peut citer :

- les huiles hydrocarbonées d'origine animale telles que le perhydrosqualène ;
- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;
- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam ;
- les esters et les éthers de synthèse notamment d'acides gras comme les huiles de formule $R_1COOR_2$ dans laquelle $R_1$ représente le reste d'un acide gras supérieur comportant de 1 à 40 atomes de carbone et $R_2$ représente une chaîne hydrocarbonée contenant de 1 à 40 atomes de carbone avec $R_1 + R_2 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétraisostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée,

groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ;

- leurs mélanges.

De préférence, l'huile a une masse moléculaire supérieure ou égale à 250 g/mol notamment entre 250 et 10000 g/mol, de préférence supérieure ou égale à 300g/mol, notamment entre 300 et 8000 g/mol et mieux, supérieure ou égale à 400 g/mol, notamment entre 400 et 5000 g/mol.

Généralement dans la phase grasse, le rapport du ou des huile(s) au(x) composé(s) particulier(s) est de 10/90 à 90/10, de préférence de 20/80 à 80/20 et de préférence encore de 30/70 à 70/30.

Cette huile peut être choisie parmi :

- les polybutylènes tels que l'INDOPOL H-100 (de masse molaire ou MM=965 g/mol), l'INDOPOL H-300 (MM=1340 g/mol), l'INDOPOL H-1500 (MM=2160g/mol) commercialisés ou fabriqués par la société AMOCO ;
- les polyisobutylènes hydrogénés tels que le PANALANE H-300 E commercialisé ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol) ;
- Les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
- les esters tels que
- les esters d'acides gras linéaires ayant un nombre total de carbone allant de 30 à 70 comme le tétrapélargonate de pentaérythrityle (MM=697,05 g/mol),
- les esters hydroxylés tels que le malate de diisostéaryle (MM= 639 g/mol),
- les esters aromatiques tels que le tridecyl trimellitate (MM=757,19 g/mol),
- les esters d'alcool gras ou d'acides gras ramifiés en C24-C28 tels que ceux décrits dans la demande EP-A-0 955 039, et notamment le citrate de triisocétyle (MM= 865 g/mol), le tétraisononanoate de pentaérythrityle (MM=697,05g/mol), le triisostéarate de glycéryle (MM=891,51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
- les huiles d'origine végétale telles que l'huile de sésame (820,6 g/mol),
- et leurs mélanges.

*Polymères cristallins*

a) Polymères semi-cristallins

On précise que, selon l'invention, dans le cas des associations susmentionnées, on entend par "polymère semi-cristallin", des polymères comportant une partie cristallisable, chaîne pendante cristallisable ou séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin selon l'invention a une température de fusion supérieure ou égale à 30 °C (notamment allant de 30 °C à 80 °C), de préférence allant de 30 °C à 60 °C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous

de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. En outre, ils ne comportent pas de squelette polysaccharidique. De façon générale, les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins.

Selon l'invention, le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

Les polymères semi-cristallins utilisables dans l'invention sont en particulier :

- les copolymères séquencés de polyoléfines à cristallisation contrôlée, notamment ceux dont les monomères sont décrits dans EP-A-0 951 897.
- les polycondensats et notamment de type polyester, aliphatique ou aromatique

ou copolyester aliphatique/aromatique,

- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré (s) tels que décrits dans le document WO-A-01/19333,
- et leurs mélanges.

Dans ces deux derniers cas, la ou les chaînes latérales ou séquences cristallisables sont hydrophobes.

A) Polymères semi-cristallins à chaînes latérales cristallisables

On peut citer en particulier ceux définis dans le document US-A-5156911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.

. Ces homo- ou co-polymères sont de toute nature du moment qu'ils présentent les conditions indiquées précédemment.

Ils peuvent résulter :

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
- de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

D'une façon générale, ces polymères sont choisis notamment parmi les homopolymères et copolymères résultant de la polymérisation d'au moins un monomère à chaîne(s) cristallisable(s) qui peut être représenté par la formule X :

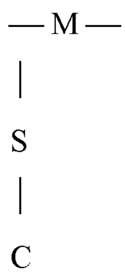

avec M représentant un atome du squelette polymérique, S représentant un espaceur, C représentant un groupe cristallisable.

Les chaînes « -S-C » cristallisables peuvent être aliphatiques ou aromatiques, éventuellement fluorées ou perfluorées. « S » représente notamment un groupe $(CH_2)_n$ ou $(CH_2CH_2O)_n$ ou $(CH_2O)$, linéaire ou ramifié ou cyclique, avec n entier allant de 0 à 22. De préférence « S » est un groupe linéaire. De préférence, « S » et « C » sont différents.

Lorsque les chaînes « -S-C » cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en $C_{14}$-$C_{24}$. Lorsqu'il s'agit de chaînes alkyle fluorées ou perfluorées, elles comportent au moins 6 atomes de carbone fluorés et notamment au moins 11 atomes de carbone dont au moins 6 atomes de carbone sont fluorés.

Comme exemple de polymères ou copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en $C_{14}$-$C_{24}$, les (méth)acrylates de perfluoroalkyle avec un groupe alkyle perfluoro en $C_{11}$-$C_{15}$, les N-alkyl (méth) acrylamides avec le groupe alkyle en $C_{14}$ à $C_{24}$ avec ou sans atome de fluor, les esters vinyliques à chaînes alkyle ou perfluoro (alkyle) avec le groupe alkyle en $C_{14}$ à $C_{24}$ (avec au moins 6 atomes de fluor pour une chaîne perfluoro alkyle), les éthers vinyliques à chaînes alkyle ou perfluoro (alkyle) avec le groupe alkyle en $C_{14}$ à $C_{24}$ et au moins 6 atomes de fluor pour une chaîne perfluoro alkyle, les alpha-oléfines en $C_{14}$ à $C_{24}$ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50% de groupes Y ou Z résultant de la copolymérisation :

α) de Y qui est un monomère polaire ou non polaire ou un mélange des deux :

- Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth) acrylamide, un N-alkyl(méth)acrylamide, un N,N-dialkyl(méth)acrylamide comme par exemple le N,N-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
- Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou styrène substitué par un groupe alkyle en $C_1$ à $C_{10}$, comme l'α-méthylstyrène, un macromonomère du type polyorganosiloxane à insaturation vinylique.

Par "alkyle», on entend au sens au sens de l'invention un groupement saturé notamment en $C_8$ à $C_{24}$, sauf mention exprès, et mieux en $C_{14}$ à $C_{24}$.

β) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en $C_{14}$-$C_{24}$, des copolymères de ces monomères avec un monomère hydrophile de préférence de nature différente de l'acide (méth) acrylique comme la N-vinylpyrrolidone ou l'hydroxyéthyl (méth)acrylate et leurs mélanges.

B) Les polymères portant dans le squelette au moins une séquence cristallisable

Ces polymères sont notamment des copolymères séquencés constitués d'au moins 2 séquences de nature chimique différente dont l'une est cristallisable.

- On peut utiliser les polymères séquencés définis dans le brevet US-A-5 156 911 ;
- Les copolymères séquencés d'oléfine ou de cyclooléfine à chaîne cristallisable comme ceux issus de la polymérisation séquencée de :
- cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
- avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
- et en particulier les copoly(éthylène/norbornène) blocs et les terpolymères (éthylène/propylène/éthylidène-norbornène) blocs. On peut aussi utiliser ceux résultants de la copolymérisation séquencée d'au moins 2 $\alpha$-oléfines en $C_2$-$C_{16}$ et mieux en $C_2$-$C_{12}$ et encore mieux en $C_4$-$C_{12}$ tels que ceux cités précédemment et en particulier les bipolymères séquencés d'éthylène et d'l-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
- Séquence cristallisable par nature : a) polyester comme les poly(alkylène téréphtalate), b) polyoléfine comme les polyéthylènes ou polypropylènes.
- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer :

$\alpha$) les copolymères séquencés poly($\varepsilon$-caprolactone)-b-poly(butadiène), utilisés de préférence hydrogénés, tels que ceux décrits dans l'article "Melting behavior of poly($\varepsilon$-caprolactone)-block-polybutadiène copolymers" de S. Nojima, Macromolécules, 32, 3727-3734 (1999).

$\beta$) les copolymères séquencés poly(butylènetéréphtalate)-b-poly(isoprène) hydrogénés séquencés ou multiséquencés, cités dans l'article "Study ofmorphological and mechanical properties of PP/PBT" de B. Boutevin et al., Polymer Bulletin, 34, 117-123 (1995).

$\gamma$) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et " Polymer agregates with crystalline cores : the system poly(ethylene)-poly(ethylene-propylene)" de P. Richter et al., Macromolécules, 30, 1053-1068 (1997).

$\delta$) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins de la composition de l'invention peuvent être ou non réticulés en partie du moment que le taux de réticulation ne gène pas leur dissolution ou dispersion dans la phase grasse liquide par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes,

portées par le polymère.

De préférence, les polymères semi-cristallins de la composition selon l'invention sont non réticulés.

Selon un mode particulier de réalisation de l'invention, le polymère est choisi parmi les copolymères résultant de la polymérisation d'au moins un monomère à chaîne cristallisable choisi parmi les (méth)acrylates d'alkyle saturés en $C_{14}$ à $C_{24}$, les (méth)acrylates de perfluoroalkyle en $C_{11}$ à $C_{15}$, les N alkyl (méth)acrylamides en $C_{14}$ à $C_{24}$ avec ou sans atome de fluor, les esters vinyliques à chaînes alkyle ou perfluoroalkyle en $C_{14}$ à $C_{24}$, les éthers vinyliques à chaînes alkyle ou perfluoroalkyle en $C_{14}$ à $C_{24}$, les alphaoléfines en $C_{14}$ à $C_{24}$, les para-alkyl styrènes avec un groupe alkyle comportant de 12 à 24 atomes de carbone, avec au moins un ester ou amide d'acide monocarboxylique en $C_1$ à $C_{10}$ éventuellement fluoré, qui peut être représenté par la formule suivante :

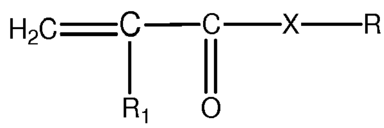

dans laquelle $R_1$ est H ou $CH_3$, R représente un groupe alkyle en $C_1$-$C_{10}$ éventuellement fluoré et X représente O, NH ou $NR_2$, où $R_2$ représente un groupe alkyle en $C_1$-$C_{10}$ éventuellement fluoré.

Selon un mode plus particulier de réalisation de l'invention, le polymère est issu d'un monomère à chaîne cristallisable choisi parmi les (méth)acrylates d'alkyle saturés en $C_{14}$ à $C_{22}$.

A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

Les polymères semi-cristallins peuvent être notamment :

ceux décrits dans les exemples 3, 4, 5, 7, 9, 13 du brevet US-A-5 156 911 à groupement -COOH, résultant de la copolymérisation d'acide acrylique et d'alkyl(méth)acrylate en $C_5$ à $C_{16}$ et plus particulièrement de la copolymérisation :

- d'acide acrylique, d'hexadécylacrylate et d'isodécylacrylate dans un rapport pondéral 1/16/3,
- d'acide acrylique et de pentadécylacrylate dans un rapport pondéral 1/19,
- d'acide acrylique, d'hexadécylacrylate, éthylacrylate dans un rapport pondéral 2,5/76,5/20,
- d'acide acrylique, d'hexadécylacrylate et de méthylacrylate dans un rapport pondéral 5/85/10,
- d'acide acrylique et de octadécylméthacrylate dans un rapport pondéral 2,5/97,5,
- d'hexadécylacrylate, de monométhyl éther de méthacrylate polyéthylèneglycol à 8 motifs d'éthylèneglycol, et d'acide acrylique dans un rapport pondéral 8,5/1/0,5.

On peut aussi utiliser le polymère de structure « O » de National Starch tel que celui décrit dans le document US-A-5 736 125 de point de fusion 44°C ainsi que les polymères semi-cristallins à chaînes pendantes cristalisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP tels que décrits dans le document US-A-5 519 063 ou EP-A-550745, de température de fusion respectivement de 40°C et 38°C.

On peut aussi utiliser les polymères semi-cristallins obtenus par copolymérisation de l'acrylate de béhényle et de l'acide acrylique ou de NVP tels que décrits dans les documents US-A-5519063 et EP-A-550745, de température de fusion respectivement de 60°C et 58°C.

De préférence, les polymères semi-cristallins ne comportent pas de groupement carboxylique.

b) Esters de dextrine et d'acide gras

Les esters de dextrine et d'acides gras peuvent être notamment choisis parmi les mono-ou poly-ester de dextrine et d'au moins un acide gras, et les composés répondant à la formule (C) :

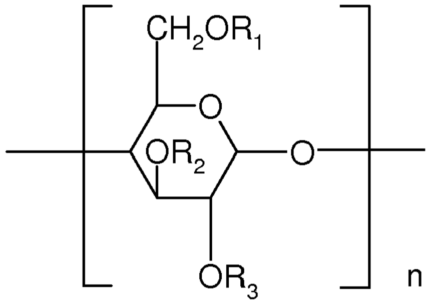

(C)

dans laquelle :

- n est un entier allant de 3 à 200, notamment allant de 20 à 150, et en particulier allant de 25 à 50,
- les radicaux $R_1$, $R_2$ et $R_3$, identiques ou différents, sont choisis parmi l'hydrogène ou un groupement acyle (R-CO-) dans lequel le radical R est un groupement hydrocarboné, linéaire ou ramifié, saturé ou insaturé, possédant de 7 à 29, en particulier de 7 à 21, notamment de 11 à 19, plus particulièrement de 13 à 17, voire 15, atomes de carbone, sous réserve qu'au moins un desdits radicaux $R_1$, $R_2$ ou $R_3$ est différent de l'hydrogène.

En particulier, $R_1$, $R_2$ et $R_3$ peuvent représenter l'hydrogène ou un groupement acyle (R-CO-) dans lequel R est un radical hydrocarboné tel que défini précédemment, sous réserve qu'au moins deux desdits radicaux $R_1$, $R_2$ ou $R_3$ sont identiques et différents de l'hydrogène.

L'ensemble des radicaux $R_1$, $R_2$ et $R_3$ peuvent figurer un groupement acyle (R-CO) identique ou différent et notamment identique.

En particulier, n est avantageusement varie de 25 à 50, notamment est égal à 38 dans la formule générale (C) de l'ester selon l'invention.

Notamment lorsque les radicaux $R_1$, $R_2$ et/ou $R_3$, identiques ou différents figurent un groupement acyle (R-CO), ceux-ci peuvent être choisis parmi les radicaux caprylique, caprique, laurique, myristique, palmitique, stéarique, arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, et leurs mélanges.

De préférence, on utilise à titre d'ester de dextrine et d'acide(s) gras au moins un palmitate de dextrine. Celui-ci peut être utilisé seul ou en mélange avec d'autres esters.

Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000.

Des esters de dextrine, en particulier des palmitates de dextrine, sont disponibles commercialement sous la dénomination RHEOPEARL TL ou RHEOPEARL KL de la société Chiba Flour.

c) Polysaccharides modifiés hydrophobes

Le polysaccharide utilisé dans la présente invention est de préférence choisi parmi les fructanes.

Les fructanes ou fructosanes sont des oligosaccharides ou des polysaccharides comprenant un enchaînement d'unités anhydrofructose éventuellement associé à un plusieurs résidus saccharidiques différents du fructose. Les fructanes peuvent être linéaires

ou ramifiés. Les fructanes peuvent être des produits obtenus directement à partir d'une source végétale ou microbienne ou bien des produits dont la longueur de chaîne a été modifiée (augmentée ou réduite) par fractionnement, synthèse ou hydrolyse en particulier enzymatique. Les fructanes ont généralement un degré de polymérisation de 2 à environ 1000 et de préférence de 2 à environ 60.

On distingue 3 groupes de fructanes. Le premier groupe correspond à des produits dont les unités fructose sont pour la plupart liées par des liaisons β-2-1.Ce sont des fructanes essentiellement linéaires tes que les inulines. Le second groupe correspond également à des fructoses linéaires mais les unités fructose sont essentiellement liées par des liaisons β-2-6. Ces produits sont des levanes. Le troisième groupe correspond à des frucanes mixtes, c'est à dire

ayant des enchainements β-2-6 et β-2-1. Ce sont des fructanes essentiellement ramifiés tes que les graminanes.

Les fructanes utilisés dans les compositions selon l'invention sont les inulines. L'inuline peut être obtenue par exemple à partir de chicorée, de dahlia ou de topinambours. De préférence, l'inuline utilisée dans la composition selon l'invention est obtenue par exemple à partir de chicorée.

Les polysaccharides, en particulier les inulines, utilisés dans les compositions selon l'invention sont modifiés hydrophobes. En particulier, elles sont obtenues par greffage de chaînes hydrophobes sur le squelette hydrophyle du fructane.

Les chaînes hydrophobes susceptibles d'être greffées sur la chaîne principale du fructane peuvent notamment être des chaînes hydrocarbonées linéaires ou ramifiées, saturées ou insaturées, ayant de 1 à 50 atomes de carbone, telles que les groupements alkyle, arylalkyle, alkylaryle, alcoylène ; des groupements divalents cycloaliphatiques ou des chaînes organopolysiloxanes. Ces chaînes hydrocarbonées ou organopolysiloxanes peuvent notamment comprendre une ou plusieurs fonctions ester, amide, uréthane, carbamate, thiocarbamate, urée, thio-urée, et/ou sulfonamide tels que notamment méthylènedicyclohexyl et isophorone ; ou des groupements divalents aromatiques tels que phénylène.

En particulier, le polysaccharide, notamment de l'inuline, présente un degré de polymérisation de 2 à environ 1000 et de préférence de 2 à environ 60, et un degré de substitution inférieur à 2 sur la base d'une unité fructose.

Selon un mode préféré de réalisation, les chaînes hydrophobes présentent au moins un groupement alkyle carbamate de formule R-NH-CO- dans laquelle R est groupement alkyle ayant de 1 à 22 atomes de carbone.

Selon un mode plus préféré de réalisation, les chaines hydrophobes sont des groupements lauryle carbamate.

En particulier, à titre illustratif et non limitatif des inulines modifiées hydrophobes pouvant être utilisées dans les compositions selon l'invention, on peut citer la stéaroyl inuline telle que celles vendues sous les dénominations Lifidrem INST par la société Engelhard et Rheopearl INS par la société Ciba ; la palmitoyl inuline ; l'undécylénoyl inuline telle que celles vendues sous les dénominations Lifidrem INUK et Lifidrem INUM par la société Engelhard ; et l'inuline lauryl carbamate tel que celui vendu sous la dénomination INUTEC SP1 par la société ORAFTI

En particulier, le polysaccharide modifié hydrophobe est une inuline greffée lauryl carbamate, notamment issue de la réaction d'isocyanate de lauryle sur une inuline, en particulier issue de la chicorée. A titre d'exemple de ces composés, on peut notamment citer le produit vendu sous la dénomination INUTEC SP1 par la société ORAFTI.

d) Copolymères d'oléfines cristallins

Le copolymère d'oléfines cristallin utilisé dans les compositions de la présente demande peut être tout copolymère d'oléfines, c'est-à-dire un copolymère comportant uniquement des motifs oléfiniques, ayant un caractère cristallin contrôlé et modéré, c'est-à-dire un taux de cristallinité au plus égal à 50%, de préférence compris entre 5 et 40%, et mieux compris entre 10 et 35%.

Ces copolymères sont généralement des élastomères ou des plastomères et peuvent être synthétisés par tout procédé connu, en particulier par voie radicalaire, par catalyse Ziegler-Natta ou par catalyse métallocène, de préférence par catalyse métallocène.

Une première classe de copolymères d'oléfines cristallins, utilisables dans les compositions selon l'invention, sont les copolymères d'α-oléfine, en particulier d' α -oléfine en $C_2$-$C_{16}$ et mieux en $C_2$-$C_{12}$. De préférence, ces copolymères sont des bi- ou terpolymères et tout particulièrement des bipolymères.

Parmi les bipolymères recommandés pour les compositions de l'invention, on peut citer les bipolymères d'éthylène et d' α -oléfine en $C_4$-$C_{16}$, de préférence en $C_4$-$C_{12}$ et les bipolymères de propylène et d' α -oléfine en $C_4$-$C_{16}$, de préférence en $C_4$-$C_{12}$. De préférence encore, l'α-oléfine est choisie parmi le butène-1, le pentène-1, l'hexène-1, l'octène-1, le nonène-1, le décène-1, l'undécène-1, le dodécène-1, le 3,5,5-triméthylhexène-1, le 3-méthylpentène-1, et le 4-méthylpentène-1.

Parmi ces monomères, le butène-1 et l'octène-1 sont particulièrement préférés.

Le taux d' α-oléfine dans le bipolymère est généralement compris entre 2 et 40% en mole, de préférence 3 à 30% en mole, et mieux 4 à 20% en mole.

Les bipolymères éthylène-octène recommandés sont les plastomères ayant une teneur en octène comprise entre 5,2% et 6,2% en mole, un taux de cristallinité compris entre 28 et 38% et les élastomères ayant une teneur en octène entre 8 et 14% en mole et un taux de cristallinité compris entre 10 et 28%.

Ces bipolymères sont synthétisés par catalyse métallocène.

De tels bipolymères sont commercialisés par la Société DOW CHEMICAL sous les dénominations commerciales AFFINITY□ (plastomères) et ENGAGE□ (élastomères).

Des bipolymères éthylène-butène sont commercialisés par la Société EXXON sous l'appellation commerciale EXACT RESINS□.

Parmi les terpolymères, on peut citer les terpolymères d'éthylène, de propylène et d'□-oléfine en $C_4$-$C_{16}$, de préférence $C_4$-$C_{12}$.

Dans ces terpolymères, les teneurs en □-oléfine en $C_4$-$C_{16}$ sont comme indiquées précédemment et les α -oléfines préférées sont le butène, l'hexène et l'octène.

Une seconde classe de copolymères d'oléfines convenant pour les compositions selon l'invention, sont les copolymères d'éthylène ou de propylène et d'une cyclooléfine, en particulier les bipolymères.

Généralement, la teneur en cyclooléfine des copolymères est inférieure à 20% en mole.

Parmi les cyclooléfines utilisables, on peut citer le cyclobutène, le cyclohexène, le cyclooctadiène, le norbornène, le diméthano-octahydronaphtalène (DMON), l'éthylidène norbornène, le vinyl norbornène et le 4-vinylcyclohexène.

Les copolymères recommandés de cette classe sont les copolymères d'éthylène et de norbornène. La teneur en norbornène de ces copolymères est généralement inférieure à 18% en mole pour présenter le caractère cristallin requis et ces copolymères sont synthétisés par catalyse métallocène.

Des copolymères éthylène/norbornène appropriés sont commercialisés par les Sociétés MITSUI PETROCHEMICAL ou MITSUI-SEKKA sous la dénomination commerciale APEL□ et par la Société HOECHST-CELANESE sous la dénomination commerciale TOPAS□.

D'autres copolymères d'éthylène/cyclooléfine recommandés sont les bipolymères éthylène/cyclobutène et éthylène/cyclohexène à faible teneur en cyclooléfine, généralement inférieure à 20% en mole.

Une troisième classe de copolymères d'oléfines appropriés est constituée par les copolymères d'oléfines de tacticité contrôlée, c'est-à-dire des copolymères comportant des motifs de tacticité différente.

Parmi ces copolymères de tacticité contrôlée, on peut citer les copolymères propylène isotactique/propylène atactique et propylène syndiotactique/propylène atactique.

Les motifs ou séquences iso- ou syndiotactiques confèrent au copolymère le caractère cristallin, cependant que les motifs ou séquences atactiques amorphes empêchent une trop forte cristallinité du copolymère et règlent le taux de cristallinité ainsi que la morphologie et la taille des cristallites.

La teneur en motifs iso- ou syndiotactiques, motifs conférant le caractère cristallin au copolymère, est donc déterminée pour obtenir le pourcentage de cristallinité voulu (≤ 50%) dans le copolymère.

La teneur en motifs tactiques est généralement comprise entre 10 et 80% en mole. Toutefois, de préférence, la teneur en motifs atactiques est inférieure à 30% en mole.

Ces copolymères sont synthétisés par catalyse métallocène.

Une quatrième classe de copolymères d'oléfines convenant pour la présente invention, est constituée par les copolymères de monooléfine et de diène, par exemple les bipolymères éthylène/butadiène, propylène/butadiène, éthylène/isoprène et propylène/isoprène, et les terpolymères éthylène/propylène/diène, obtenus également par synthèse métallocène.

La proportion de motifs diène dans le copolymère à cristallisation contrôlée est généralement comprise entre 3 et 20% en mole.

Pour améliorer le réglage de la cristallinité du copolymère, on peut éventuellement ajouter à la composition selon l'invention des additifs gênant la cristallisation et favorisant la formation de petits cristaux. Ces additifs, bien qu'utilisés en faible proportion, constituent des "sites" de germination nombreux et petits répartis uniformément dans la masse. Ces additifs sont typiquement des cristaux d'une substance organique ou minérale.

Dans le cas d'un additif organique devant cristalliser, celui-ci doit avoir un point de fusion supérieur à la zone de fusion du copolymère et former, de préférence, de petits cristaux.

A une température supérieure à son point de fusion, cette substance est de préférence soluble dans le mélange de la phase grasse liquide et de polymère fondu. Ainsi, lors du refroidissement, l'additif initialement dissous, recristallise sous forme de petits cristaux nombreux et bien diffusés dans le mélange, puis le polymère recristallise en donnant des domaines cristallins petits du fait de la présence des cristaux d'additifs. Cette technique de recristallisation des polymères est classique.

On peut également ajuster le taux de cristallisation, la taille et la morphologie des copolymères d'oléfines selon l'invention en mélangeant un premier copolymère d'oléfines selon l'invention avec un second polymère ou copolymère cristallin, compatible en partie avec le premier copolymère d'oléfines. Le second polymère ou copolymère peut être un copolymère d'oléfines selon l'invention, mais de taux de cristallinité différent de celui du premier copolymère, y compris un taux de cristallinité plus élevé que le taux de cristallinité des copolymères d'oléfines selon l'invention.

Le deuxième polymère cristallisable peut aussi être un polymère de nature différente, par exemple un copolyéthylène/acétate de vinyle obtenu par copolymérisation radicalaire ou même un polyéthylène cristallisable tel que ceux habituellement utilisés dans le domaine cosmétique.

Pour plus de détails quant à cette méthode d'ajustement du taux de cristallinité, on se réfèrera aux articles intitulés "Elastomeric blends of homogeneous ethylene-octene copolymers (Mélanges élastomériques de copolymères homogènes éthylène-octène)" S. Bensason et al., Polymer, Volume 38, N° 15, 1997, pages 3913-19, et "Blends of homogeneous ethylene-octene copolymers (Mélanges de copolymères homogènes éthylène-octène)" S; Bensason et al., Polymer, Volume 38, N° 14, 1997, pages 3513-20.

d) Polycondensats cristallins

Le polycondensat utilisable peut être susceptible d'être obtenu par réaction :

- de 10 à 30% en poids, par rapport au poids total du polycondensat, d'au moins un polyol comprenant 3 à 6 groupes hydroxyles;
- de 30 à 80% en poids, par rapport au poids total du polycondensat, d'au moins un acide monocarboxylique non aromatique, saturé ou insaturé, linéaire, ramifié et/ou cyclique, comprenant 6 à 32 atomes de carbone;
- de 0,1 à 10 % en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 5 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique.

De préférence, le polycondensat est susceptible d'être obtenu par réaction:

- de 10% en poids d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone; et
- de 15 à 30% en poids, par rapport au poids total du polycondensat, d'au moins un polyol comprenant 3 à 6 groupes hydroxyles; et
- de 30 à 40% en poids, par rapport au poids total du polycondensat, d'au moins un acide monocarboxylique non aromatique, saturé ou insaturé, linéaire, ramifié et/ou cyclique, comprenant 6 à 32 atomes de carbone; et
- de 10 à 25% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique;
  ces conditions étant cumulatives,
  alors le rapport entre le nombre de mole d'acide monocarboxylique aromatique et le nombre de mole d'acide monocarboxylique non aromatique est compris entre 0,08 et 0,70.

Le polycondensat peut également être susceptible d'être obtenu par réaction :

- de 10 à 30% en poids, par rapport au poids total du polycondensat, d'au moins un polyol comprenant 3 à 6 groupes hydroxyles;
- de 45 à 80% en poids, par rapport au poids total du polycondensat, d'au moins un acide monocarboxylique non aromatique saturé, linéaire, ramifié et/ou cyclique, comprenant 6 à 32 atomes de carbone;
- de 0,1 à 10% en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 5 à 40% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique.

L'un des constituants nécessaires pour la préparation des polycondensats selon l'invention est un composé comprenant 3 à 6 groupes hydroxyles (polyol), notamment 3 à 4 groupes hydroxyles. On peut bien évidemment utiliser un mélange de tels polyols. Ledit polyol peut notamment être un composé carboné, notamment hydrocarboné, linéaire, ramifié et/ou cyclique, saturé ou insaturé, comprenant 3 à 18 atomes de carbone, notamment 3 à 12, voire 4 à 10 atomes de carbone, et 3 à 6 groupes hydroxy (OH), et pouvant comprendre en outre un ou plusieurs atomes d'oxygène intercalés dans la chaîne (fonction éther). Ledit polyol est de préférence un composé hydrocarboné saturé, linéaire ou ramifié, comprenant 3 à 18 atomes de carbone, notamment 3 à 12, voire 4 à 10 atomes de carbone, et 3 à 6 groupes hydroxy (OH). Il peut être choisi parmi, seul ou en mélange :

- les triols, tels que le 1,2,4-butanetriol, le 1,2,6-hexanetriol, le triméthyloléthane, le triméthylolpropane, le glycérol;
- les tétraols, tels que le pentaérythritol (tétraméthylolméthane), l'érythritol, le diglycérol

ou le ditriméthylolpropane;

- les pentols tels que le xylitol,
- les hexols tels que le sorbitol et le mannitol; ou encore le dipentaérythritol ou le triglycérol.

De préférence, le polyol est choisi parmi le glycérol, le pentaérythritol, le diglycérol, le sorbitol et leurs mélanges; et encore mieux est du pentaérythritol. Le polyol,
ou le mélange de polyol, représente de préférence 10 à 30% en poids, notamment 12 à 25% en poids, et mieux 14 à 22% en poids, du poids total du polycondensat final.
Un autre constituant nécessaire pour la préparation des polycondensats selon l'invention est un acide monocarboxylique non aromatique, saturé ou insaturé, linéaire, ramifié et/ou cyclique, comprenant 6 à 32 atomes de carbone, notamment 8 à 28 atomes de carbone et encore mieux 10 à 24, voire 12 à 20, atomes de carbone. On peut bien évidemment utiliser un mélange de tels acides monocarboxyliques non aromatiques. Par acide monocarboxylique non aromatique, on entend un composé de formule RCOOH, dans laquelle R est un radical hydrocarboné saturé ou insaturé, linéaire, ramifié et/ou cyclique, comprenant 5 à 31 atomes de carbone, notamment 7 à 27 atomes de carbone, et encore mieux 9 à 23 atomes de carbone, voire 11 à 19 atomes de carbone. De préférence, le radical R est saturé. Encore mieux, ledit radical R est linéaire ou ramifié, et préférentiellement en C5-C31, voire en C11-C21.
Dans un mode de réalisation particulier de l'invention, l'acide monocarboxylique non aromatique présente une température de fusion supérieure ou égale à 25°C, notamment supérieure ou égale à 28°C, voire à 30°C; on a en effet constaté que lorsque l'on emploie un tel acide, en particulier en quantité importante, il est possible, d'une part d'obtenir une bonne brillance et la tenue de ladite brillance, et d'autre part de réduire la quantité de cires usuellement présente dans la composition envisagée.
Parmi les acides monocarboxyliques non aromatiques susceptibles d'être employés, on peut citer, seul ou en mélange :

- les acides monocarboxyliques saturés tels que l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentyl-propionique, l'acide cyclohexanecarboxylique, l'acide cyclohexylacétique, l'acide 4-cyclohexylbutyrique;
- les acides monocarboxyliques insaturés mais non aromatiques, tels que l'acide caproléique, l'acide obtusilique, l'acide undécylénique, l'acide dodécylénique, l'acide lindérique, l'acide myristoléique, l'acide physétérique, l'acide tsuzuique, l'acide palmitoléique, l'acide oléique, l'acide pétrosélinique, l'acide vaccénique, l'acide élaidique, l'acide gondoïque, l'acide gadoléique, l'acide érucique, l'acide cétoléique, l'acide nervonique, l'acide linoléique, l'acide linolénique, l'acide arachidonique.

Parmi les acides monocarboxyliques non aromatiques ayant une température de fusion supérieure ou égale à 25°C, on peut citer, seul ou en mélange :

- parmi les acides monocarboxyliques saturés : l'acide décanoïque (caprique), l'acide laurique, l'acide tridécanoïque, l'acide myristique, l'acide palmitique, l'acide stéarique, l'acide arachidique, l'acide béhénique, l'acide cérotique (hexacosanoïque);
- parmi les acides monocarboxyliques insaturés mais non aromatiques : l'acide pétrosélinique, l'acide vaccénique, l'acide élaidique, l'acide gondoïque, l'acide gadoléique, l'acide érucique, l'acide nervonique.

De préférence, on peut utiliser l'acide 2-éthylhexanoïque, l'acide isooctanoïque, l'acide laurique, l'acide myristique, l'acide isoheptanoïque, l'acide isononanoïque, l'acide nonanoïque, l'acide palmitique, l'acide isostéarique, l'acide stéarique, l'acide béhénique et leurs mélanges, et encore mieux l'acide isostéarique seul ou l'acide stéarique seul. Ledit acide monocarboxylique non aromatique, ou le mélange desdits acides, représente de préférence 30 à 80% en poids, notamment 40 à 75% en poids, voire 45 à 70% en poids, et mieux 50 à 65% en poids, du poids total du polycondensat final.
Un autre constituant nécessaire pour la préparation des polycondensats selon l'invention est un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone, notamment 2 à 12, voire 3 à 8 atomes de carbone._On peut bien évidemment utiliser un mélange de tels acides monocarboxyliques aromatiques.
Par acide monocarboxylique aromatique, on entend un composé de formule R'COOH, dans laquelle R' est un radical hydrocarboné aromatique, comprenant 6 à 10 atomes de carbone, et en particulier les radicaux benzoïque et

naphtoïque. Ledit radical R' peut en outre être substitué par 1 à 3 radicaux alkyles, saturés ou insaturés, linéaires, ramifiés et/ou cycliques, comprenant 1 à 32 atomes de carbone, notamment 2 à 12, voire 3 à 8 atomes de carbone; et notamment choisis parmi méthyle, éthyle, propyle, isopropyle, butyle, isobutyle, terbutyle, pentyle, isopentyle, néopentyle, cyclopentyle, hexyle, cyclohexyle, heptyle, isoheptyle, octyle ou isooctyle. Parmi les acides monocarboxyliques aromatiques susceptibles d'être employés, on peut citer, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl-benzoïque, l'acide 1-méthyl-2-naphtoïque, l'acide 2-isopropyl-1-naphtoïque. De préférence, on peut utiliser l'acide benzoïque, l'acide 4-tert-butyl-benzoïque, l'acide o-toluique, l'acide m-toluique, l'acide 1-naphtoïque, seuls ou en mélanges; et encore mieux l'acide benzoïque seul. Ledit acide monocarboxylique aromatique, ou le mélange desdits acides, représente de préférence 0,1 à 10% en poids, notamment 0,5 à 9,95% en poids, mieux encore de 1 à 9,5% en poids, voire 1,5 à 8% en poids, du poids total du polycondensat final.

Un autre constituant nécessaire pour la préparation des polycondensats selon l'invention est un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique. On peut bien évidemment utiliser un mélange de tels acides polycarboxyliques et/ou d'anhydrides. Ledit acide polycarboxylique peut notamment être choisi parmi les acides polycarboxyliques linéaires, ramifiés et/ou cycliques, saturés ou insaturés, voire aromatiques, comprenant 2 à 50, notamment 2 à 40, atomes de carbone, en particulier 3 à 36, voire 3 à 18, et encore mieux 4 à 12 atomes de carbone, voire 4 à 10 atomes de carbone; ledit acide comprend au moins deux groupes carboxyliques COOH, de préférence de 2 à 4 groupes COOH.

De préférence, ledit acide polycarboxylique est aliphatique saturé, linéaire et comprend 2 à 36 atomes de carbone, notamment 3 à 18 atomes de carbone, voire 4 à 12 atomes de carbone; ou bien est aromatique et comprend 8 à 12 atomes de carbone. Il comprend de préférence 2 à 4 groupes COOH. Ledit anhydride cyclique d'un tel acide polycarboxylique peut notamment répondre à l'une des formules suivantes :

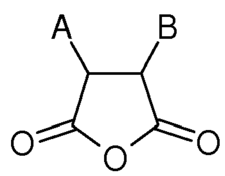

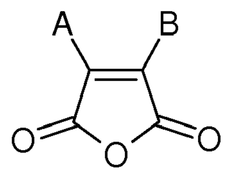

dans lesquelles les groupements A et B sont, indépendamment l'un de l'autre, :

- un atome d'hydrogène,
- un radical carboné, aliphatique, saturé ou insaturé, linéaire, ramifié et/ou cyclique, ou bien aromatique; comprenant 1 à 16 atomes de carbone, notamment 2 à 10 atomes de carbone, voire 4 à 8 atomes de carbone, notamment méthyle ou éthyle;
- ou bien A et B pris ensemble forment un cycle comprenant au total 5 à 7, notamment 6 atomes de carbone, saturé ou insaturé, voire aromatique.
  De préférence, A et B représentent un atome d'hydrogène ou forment ensemble un cycle aromatique comprenant au total 6 atomes de carbone.

Parmi les acides polycarboxyliques ou leurs anhydrides, susceptibles d'être employés, on peut citer, seul ou en mélange :

- les acides dicarboxyliques tels que l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide tétrahydrophtalique, l'acide hexahydrophtalique, l'acide pimélique, l'acide sébacique, l'acide azélaïque, l'acide glutarique, l'acide adipique, l'acide fumarique, l'acide maléïque, l'acide itaconique, les dimères d'acides gras (notamment en C36) tels que les produits commercialisés sous les dénominations Pripol 1006, 1009, 1013 et 1017, par Uniqema;
- les acides tricarboxyliques tels que l'acide cyclohexanetricarboxylique, l'acide trimellitique, l'acide 1,2,3-benzènetricarboxylique, l'acide 1,3,5-benzènetricarboxy-lique;
- les acides tétracarboxyliques tels que l'acide butanetétracarboxylique et l'acide pyroméllitique,
- les anhydrides cycliques de ces acides et notamment l'anhydride phtalique, l'anhydride trimellitique, l'anhydride maléïque et l'anhydride succinique.

De préférence, on peut utiliser l'acide adipique, l'anhydride phtalique et/ou l'acide isophtalique, et encore mieux l'acide isophtalique seul.
Ledit acide polycarboxylique et/ou son anhydride cyclique, représente de préférence 5 à 40% en poids, notamment 10 à 30% en poids, et mieux 14 à 25% en poids, du poids total du polycondensat final.

Le polycondensat selon l'invention peut en outre comprendre une silicone à fonction hydroxyle (OH) et/ou carboxylique (COOH).
Elle peut comprendre 1 à 3 fonctions hydroxyle et/ou carboxylique, et comprend de préférence deux fonctions hydroxyle ou bien deux fonctions carboxyliques.
Ces fonctions peuvent être situées en bout de chaîne ou dans la chaîne, mais avantageusement en bout de chaîne.
On emploie de préférence des silicones ayant une masse moléculaire moyenne en poids (Mw) comprise entre 300 et 20000, notamment 400 et 10 000, voire 800 et 4000.
Cette silicone peut être de formule :

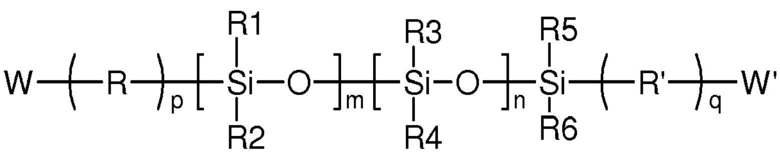

dans laquelle :

- W et W' sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carboné, notamment hydrocarboné, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (éther);
  notamment R et/ou R' peuvent être de formule $-(CH_2)_a-$ avec a=1-12, et notamment méthylène, éthylène, propylène, phénylène;

ou bien de formule $-[(CH_2)_xO]_z-$ avec x = 1, 2 ou 3 et z = 1-10; en particulier x=2 ou 3 et z=1-4; et mieux x=3 et z=1.

- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.
  On peut notamment citer les polyalkylsiloxanes α,ω-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryl/alkylsiloxanes à fonctions silanol tels que le polyphényl/méthylsiloxane ou encore le polyphényl/propylsiloxane.
  Tout particulièrement, on utilisera les polydiméthysiloxanes α,ω-diol de masse moléculaire moyenne en poids (Mw) comprise entre 400 et 10 000, voire entre 500 et 5000, et notamment entre 800 et 4000.
  Lorsqu'elle est présente, ladite silicone peut de préférence représenter 0,1 à 15% en poids, notamment 1 à 10% en poids, voire 2 à 8% en poids, du poids du polycondensat.

Dans un mode de réalisation préféré de l'invention, l'acide monocarboxylique aromatique est présent en quantité molaire inférieure ou égale à celle de l'acide monocarboxylique non aromatique; notamment le rapport entre le nombre de mole d'acide monocarboxylique aromatique et le nombre de mole d'acide monocarboxylique non aromatique est de préférence compris entre 0,08 et 0,70, notamment entre 0,10 et 0,60, en particulier entre 0,12 et 0,40.

De préférence, le polycondensat selon l'invention est susceptible d'être obtenu par réaction :

- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol;
  présent de préférence en une quantité de 10 à 30% en poids, notamment 12 à 25% en poids, et mieux 14 à 22% en poids, par rapport au poids total du polycondensat final;
- d'au moins un acide monocarboxylique non aromatique choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
  présent de préférence en une quantité de 30 à 80% en poids, notamment 40 à 75% en poids, et mieux 45 à 70% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
  présent de préférence en une quantité de 0,1 à 10% en poids, notamment 1 à 9,5% en poids, voire 1,5 à 8% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique;
  présent de préférence en une quantité de 5 à 40% en poids, notamment 10 à 30% en poids, et mieux 14 à 25% en poids, par rapport au poids total du polycondensat final.

Préférentiellement, le polycondensat selon l'invention est susceptible d'être obtenu par réaction :

- d'au moins un polyol choisi parmi, seul ou en mélange, le glycérol, le pentaérythritol, le sorbitol et leurs mélanges, et encore mieux le pentaérythritol seul; présent en une quantité de 10 à 30% en poids, notamment 12 à 25% en poids, et mieux 14 à 22% en poids, par rapport au poids total du polycondensat final;
- d'au moins un acide monocarboxylique non aromatique choisi parmi, seul ou en mélange, l'acide 2-éthylhexanoïque, l'acide isooctanoïque, l'acide laurique, l'acide palmitique, l'acide isostéarique, l'acide isononanoïque, l'acide stéarique, l'acide béhénique et leurs mélanges, et encore mieux l'acide isostéarique seul ou l'acide stéarique seul;
  présent en une quantité de 30 à 80% en poids, notamment 40 à 75% en poids, et mieux 45 à 70% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide 1-naphtoïque, et encore mieux l'acide benzoïque seul; présent en une quantité de 0,1 à 10% en poids, notamment 1 à 9,5% en poids, voire 1,5 à 8% en poids par rapport au poids total du polycondensat final; et
- d'au moins un acide polycarboxylique ou un de ses anhydrides, choisi parmi, seul ou en mélange, l'anhydride phtalique et l'acide isophtalique, et encore mieux l'acide isophtalique seul; présent en une quantité de 5 à 40% en poids, notamment 10 à 30% en poids, et mieux 14 à 25% en poids, par rapport au poids total du polycondensat final.

Le polycondensat selon l'invention peut être préparé par les procédés d'estérification/polycondensation usuellement employés par l'homme du métier. A titre d'illustration, un procédé général de préparation consiste :

- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques (atteint lorsque l'indice d'acide est inférieur ou égal à 1), de préférence en distillant au fur et à mesure l'eau formée, puis

- à éventuellement refroidir le mélange à une température comprise entre 90 et 150°C,
- à ajouter l'acide polycarboxylique et/ou l'anhydride cyclique, et optionnellement la silicone à fonctions hydroxyles ou carboxyliques, en une seule fois ou de façon séquencée, puis
- à chauffer à nouveau à une température inférieure ou égale à 220°C, notamment comprise entre 170 et 220°C, de préférence en continuant à éliminer l'eau formée, jusqu'à l'obtention des caractéristiques requises en terme d'indice d'acide, de viscosité, d'indice d'hydroxyle et de solubilité.
  Il est possible d'ajouter des catalyseurs d'estérification conventionnels, par exemple de type acide sulfonique (notamment à une concentration pondérale comprise entre 1 et 10%)

ou type titanate (notamment à une concentration pondérale comprise entre 5 et 100 ppm). Il est également possible de réaliser la réaction, en tout ou en partie, dans un solvant inerte tel que le xylène et/ou sous une pression réduite, pour faciliter l'élimination de l'eau. Avantageusement, on n'utilise ni catalyseur ni solvant.

Ledit procédé de préparation peut comprendre en outre une étape d'addition d'au moins un agent antioxydant dans le milieu réactionnel, notamment à une concentration pondérale comprise entre 0,01 et 1%, par rapport au poids total de monomères, de façon à limiter les éventuelles dégradations liées à un chauffage prolongé.

L'agent antioxydant peut être de type primaire ou de type secondaire, et peut être choisi parmi les phénols encombrés, les amines secondaires aromatiques, les composés organophosphorés, les composés soufrés, les lactones, les bisphénols acrylés; et leurs mélanges.

*Agents structurants lipophiles minéraux*

L'agent rhéologique de phase grasse peut être un agent structurant lipophile minéral.

On peut notamment citer les argiles lipophiles, comme les argiles éventuellement modifiées telles que les hectorites modifiées par un chlorure d'ammonium d'acide gras en $C_{10}$ à $C_{22}$, comme l'hectorite modifiée par du chlorure de di-stéaryl di-méthyl ammonium.

On peut également citer les silices hydrophobes, comme la silice pyrogénée éventuellement traitée hydrophobe en surface dont la taille des particules est inférieure à 1 μm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée hydrophobe présente de préférence une taille de particules pouvant être nanométrique à micrométrique, par exemple allant d'environ de 5 à 200 nm.

*Polymères polyamides lipophiles*

Par polymère, on entend au sens de l'invention un composé ayant au moins 2 motifs de répétition, de préférence au moins 3 motifs de répétition et mieux encore 10 motifs de répétition.

Comme polymères polyamides structurants lipophiles préférés utilisables dans l'invention, on peut citer les polyamides ramifiés par des chaînes grasses pendantes et/ou des chaînes grasses terminales ayant de 12 à 120 atomes de carbone et notamment de 12 à 68 atomes de carbone, les chaînes grasses terminales étant liées au squelette polyamide par des groupes ester. Ces polymères sont plus spécialement ceux décrits dans le document US-A-5783657 de la société Union Camp. Chacun de ces polymères satisfait notamment à la formule (I) suivante :

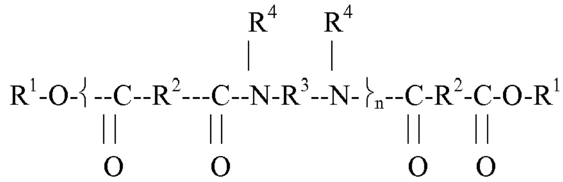

dans laquelle n désigne un nombre entier de motifs amide tel que le nombre de groupes ester représente de 10 % à 50 % du nombre total des groupes ester et amide ; $R^1$ est à chaque occurrence indépendamment un groupe alkyle ou alcényle ayant au moins 4 atomes de carbone ; $R^2$ représente à chaque occurrence indépendamment un groupe hydrocarboné en $C_4$ à $C_{42}$ à condition que 50 % des groupes $R^2$ représentent un groupe hydrocarboné en $C_{30}$ à $C_{42}$ ; $R^3$ représente à chaque occurrence indépendamment un groupe organique pourvu d'au moins 2 atomes de carbone, d'atomes d'hydrogène et optionnellement d'un ou plusieurs atomes d'oxygène ou d'azote ; et $R^4$ représente à chaque occurrence indépendamment un atome d'hydrogène, un groupe alkyle en $C_1$ à $C_{10}$ ou une liaison directe à $R^3$ ou à un autre $R^4$ de sorte que l'atome d'azote auquel sont liés à la fois $R^3$ et $R^4$ fasse partie d'une structure hétérocyclique définie par $R^4$-N-$R^3$, avec au moins 50 % des $R^4$ représentant un atome d'hydrogène.

En particulier, les groupes ester de la formule (I), qui font partie des chaînes grasses terminales et/ou pendantes au sens de l'invention, représentent de 15 à 40 % du nombre total des groupes ester et amide et mieux de 20 à 35 %. De plus, n représente avantageusement un nombre entier allant de 1 à 5. De préférence, $R^1$ est un groupe alkyle en $C_{12}$ à $C_{22}$ et de préférence en $C_{16}$ à $C_{22}$. Avantageusement, $R^2$ peut être un groupe hydrocarboné (alkyle ou alcényle notamment) en $C_{10}$ à $C_{42}$ ayant une structure d'acide gras polymérisé ou de dimère dont les groupements acide carboxylique ont été enlevés (ces groupements servant à la formation de l'amide). De préférence, 50 % au moins et mieux 75 % des $R^2$ sont des groupes ayant de 30 à 42 atomes de carbone. Les autres $R^2$ sont des groupes hydrogénés en $C_4$ à $C_{19}$ et même en $C_4$ à $C_{12}$. De préférence, $R^3$ représente un groupe hydrocarboné en $C_2$ à $C_{36}$ ou un groupe polyoxyalkyléné et $R^4$ représente un atome d'hydrogène. De préférence, $R^3$ représente un groupe hydrocarboné en $C_2$ à $C_{12}$. Les groupes hydrocarbonés peuvent être des groupes linéaires, cycliques ou ramifiés, saturés ou insaturés. Par ailleurs, les groupes alkyle et alcényle peuvent être des groupes linéaires ou ramifiés.

De façon avantageuse, le polymère de la composition de l'invention comprend une masse moléculaire moyenne en poids allant de 2 000 à 20 000 et mieux de 2 000 à 10 000.

Selon l'invention, la structuration de l'huile est obtenue à l'aide d'un ou plusieurs polymères de formule (I). En général, les polymères de formule (I) se présentent sous forme de mélanges de polymères, ces mélanges pouvant en outre contenir un produit de synthèse tel que n vaut 0, c'est-à-dire un diester.

A titre d'exemple de polymères structurants utilisables dans la composition selon l'invention, on peut citer les produits commerciaux vendu par la société Bush Boake Allen sous les noms Uniclear 80 et Uniclear 100. Ils sont vendus respectivement sous forme de gel à 80 % (en matière active) dans une huile minérale et à 100 % (en matière active). Ils ont un point de ramollissement de 88 à 94°C. Ces produits commerciaux sont un mélange de copolymère d'un diacide en $C_{36}$ condensé sur l'éthylène diamine, de masse moléculaire moyenne d'environ 6000. Les terminaisons d'acide restantes sont, en outre, estérifiées par l'alcool cétylstéarylique.

Comme polymère structurant utilisable dans l'invention, on peut encore citer les résines polyamides résultant de la condensation d'un acide di-carboxylique aliphatique et d'un diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US-A-3645705 et US-A-3148125. Plus spécialement, on utilise les Versamid® 930 ou 744.

On peut aussi utiles les polyamides vendus par la société Union Camp Corp. sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US-A-5500209.

Les polymères structurant de la composition de l'invention ont avantageusement une température de ramollissement supérieur à 70°C et pouvant aller jusqu'à 190°C. De préférence, il présente une température de ramollissement allant de 80 à 130°C. Ces polymères sont en particulier des polymères non cireux.

*Polymères polyurées ou polyuréthanes lipophiles*

Comme agent rhéologique de phase grasse, on peut également citer les polyuréthanes et les polyurées solubles

ou dispersibles dans la (ou les) huile(s) hydrocarbonée(s), et comportant:

- au moins deux groupes uréthanes, ou au moins deux groupes urées ou au moins un groupe uréthane et un groupe urée dans la chaîne,
- au moins une séquence ou un greffon hydrocarboné ou polyester aliphatique à longue chaîne hydrocarbonée, de préférence ramifiée.

Par longue chaîne hydrocarbonée, on entend une chaîne hydrocarbonée, linéaire ou ramifiée, comportant au moins 8 atomes de carbone et de préférence 10 à 500 atomes de carbone.
Les polymères préférés selon l'invention sont définis par l'une des trois formules suivantes:

I) 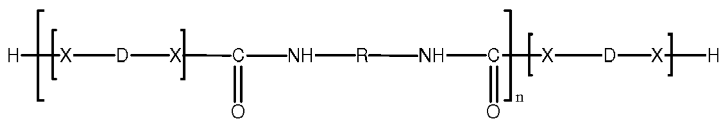

II) 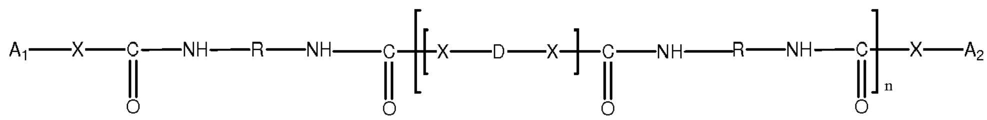

III) 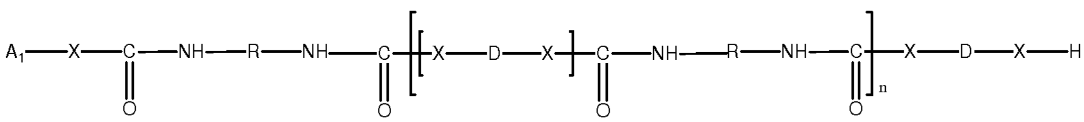

dans lesquelles:

n désigne un nombre entier de 1 à 10.000, et de préférence de 1 à 1000,
x représente, séparément ou conjointement, -O- ou -NH-,
R est un radical divalent choisi parmi les radicaux alkylènes, cycloalkylènes, aromatiques, et leurs mélanges, éventuellement fonctionnalisés,
$A_1$ et $A_2$, identiques ou différents, désignent des radicaux hydrocarbonés monovalents linéaires, ramifiés ou cycliques, saturés ou pouvant présenter des insaturations, comportant de 1 à 80 atomes de carbone,
D est

1) une séquence divalente hydrocarbonée aliphatique, et/ou cycloaliphatique saturée ou non, et/ou polyester aliphatique à longue chaîne hydrocarbonée, ou
2) un greffon

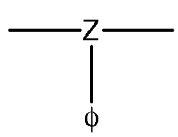

où Z est un radical trivalent hydrocarboné pouvant contenir un ou plusieurs hétéroatomes, et φ est une chaîne aliphatique linéaire, ramifiée ou cyclique,
3) des mélanges des séquences 1) et des greffons 2).

Les radicaux hydrocarbonés monovalents $A_1$ et $A_2$ sont de préférence choisis parmi les radicaux aliphatiques, cycloaliphatiques et aromatiques, saturés ou insaturés. Les radicaux $A_1$ et $A_2$ proviennent des monoalcools et/ou monoamines éventuellement utilisés pour consommer les groupes isocyanates résiduels en fin de polymérisation.
Dans le cas où D est une séquence hydrocarbonée aliphatique et/ou cycloaliphatique, saturée ou non, celle-ci provient:

- d'une huile naturelle ou synthétique, ou
- du produit d'addition (dimère, trimère ou polymère) d'au moins deux chaînes aliphatiques insaturées, tels que les radicaux aliphatiques dérivés des acides gras " dimères ", comme par exemple les produits d'addition entre chaînes oléiques, ou
- de polyènes, de préférence hydrogénés, tels que le polybutadiène, le polyisoprène hydrogéné, ou les polyoléfines ou copolyoléfines.

Dans le cas où D est une séquence polyester aliphatique à longue chaîne hydrocarbonée, celle-ci provient de préférence de polyesters ramifiés à longues chaînes hydrocarbonées, comme par exemple le poly(12-hydroxystéarate).

Dans le cas où D est un greffon, φ est une chaîne aliphatique, linéaire, ramifiée ou cyclique, saturée ou non, comportant de 8 à 40 atomes de carbone. Les hétéroatomes éventuels du radical trivalent Z sont de préférence -O-, -N-, et -S-.

Les polyuréthanes et/ou polyurées structurants selon l'invention résultent de la réaction de polymérisation entre:

1) au moins un diisocyanate aliphatique, cycloaliphatique et/ou aromatique de formule générale O=C=N-R-N=C=O, où R est tel que défini précédemment,
2) au moins un dérivé difonctionnel HX-D-XH, présentant deux hydrogènes actifs pouvant réagir chacun avec un groupe isocyanate, où

   - X désigne -O- ou -NH-, et
   - D est tel que défini précédemment, et

3) éventuellement, un dérivé monofonctionnel $A_1$-XH, ou deux dérivés monofonctionnels $A_1$-XH et $A_2$-XH, présentant un seul hydrogène actif pouvant réagir avec un groupe isocyanate, pour consommer les groupes isocyanates résiduels qui n'auraient pas entièrement réagi avec les réactifs difonctionnels H-X-D-X-H, les dérivés monofonctionnels $A_1$-XH et $A_2$-XH pouvant être identiques ou différents, et $A_1$ et $A_2$ étant tel que définis précédemment.

Les isocyanates utilisés dans la réaction de polymérisation peuvent être aliphatiques, cycloaliphatiques ou aromatiques. On utilisera de manière avantageuse l'hexaméthylène diisocyanate, l'isophorone diisocyanate, le toluène diisocyanate, le 4,4'-dicyclohexylméthane diisocyanates.

Les dérivés difonctionnels H-X-D-X-H peuvent être choisis parmi les dimères diols et leurs dérivés, les alcane diols, les polydiènes à extrémités hydroxyles, de préférence hydrogénés, les polyoléfines à extrémités hydroxyles, les polyesters ramifiés à longue chaîne alkyle portant au moins deux groupes réactifs, les huiles naturelles ou synthétiques portant de deux à trois groupes hydroxyles, et enfin les dimères-diamines et les diamines à chaîne aliphatique longue.

Les dimères-diols sont des diols ramifiés en $C_{36}$, aliphatiques et/ou alicycliques, et/ou un mélange desdits dimères. Ces diols sont préparés à partir des " acides gras dimères correspondants ".

On entend par " acides gras dimères correspondants " les acides gras dimères qui ont la même structure que ces diols, mais qui possèdent deux extrémités acides carboxyliques à la place des extrémités diols. La transformation des acides gras dimères en dimères-diols peut être réalisée soit par hydrogénation d'esters méthyliques des acides gras dimères, soit par dimérisation directe de l'alcool oléique. On citera, en particulier, les dimères-diols vendus par la société COGNIS sous les noms commerciaux de SOVERMOL 908 (à 97% de pureté), et SOVERMOL 650 NS (à 68% de pureté).

On peut aussi utiliser les oligomères polyéthers-diols et les polycarbonates-diols, préparés par éthérification ou estérification ultérieure de ces mêmes dimères-diols ramifiés en $C_{36}$. Ces oligomères ont généralement une masse moléculaire moyenne en nombre de l'ordre de 500 à 2000, et possèdent deux fonctions hydroxyles.

Les polydiènes à extrémités hydroxyles sont par exemple ceux définis dans le brevet français FR-2782723. Ils sont choisis dans le groupe comprenant les homo et copolymères de polybutadiène, de polyisoprène et de poly (1,3-pentadiène). Ces oligomères ont une masse moléculaire moyenne en nombre inférieure à 7000, et de préférence de 1000 à 5000. Ils présentent une fonctionnalité en bout de chaîne de 1,8 à 3, et de préférence voisine de 2. Ces polydiènes à extrémités hydroxyles sont par exemple les polybutadiènes hydroxylés commercialisés par la société ELF ATOCHEM sous les marques POLY BD-45H® et POLY BD R-20 LM®. Ces produits sont utilisés de préférence hydrogénés.

On peut également utiliser des polyoléfines, homopolymères ou copolymères, à extrémités α, ω hydroxyles, comme par exemple:

- les oligomères de polyisobutylène à extrémités α, ω hydroxyles, ou
- les copolymères commercialisés par la société MITSUBISHI sous la marque POLYTAIL®, notamment ceux de

structure:

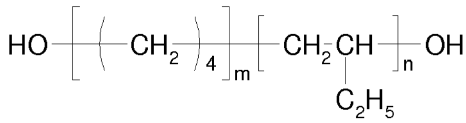

ayant un point de fusion de 60 à 70˚C.

Il est possible d'utiliser comme dérivé difonctionnel H-X-D-X-H, un polyester ramifié à longue chaîne alkyle et comportant au moins deux groupes réactifs, comme par exemple le poly(12-hydroxystéarate) à extrémités hydroxyles. Ce polyester est obtenu par auto-condensation de l'acide 1,2-hydroxystéarique sur lui-même, puis réaction avec un polyol pour consommer les groupes acides résiduels. Cet oligomère de structure

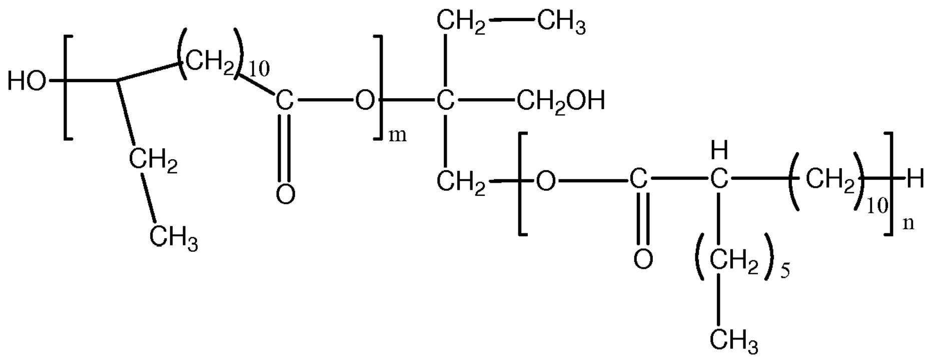

où la somme m+n est telle que l'oligomère a une masse moléculaire moyenne en nombre de l'ordre de 2000 et une fonctionnalité hydroxyle de l'ordre de 1,8.

On peut également utiliser comme dérivé difonctionnel H-X-D-X-H, des huiles naturelles ou synthétiques, portant de deux à trois groupes hydroxyles.

Dans un mode de réalisation particulier de l'invention, on utilisera les huiles portant deux groupes hydroxyles par chaîne, et de préférence les monoglycérides de structure:

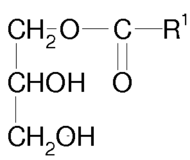

ou

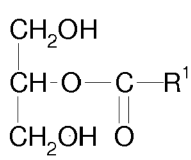

$R^1$ étant une chaîne alkyle linéaire ou ramifiée de $C_8$ à $C_{30}$ comme par exemple le monostéarate de glycérol.

De tels monoesters de glycérol correspondent par exemple aux dérivés difonctionnels H-X-D-X-H, où:

- D représente

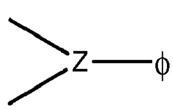

- X représente - O -, et

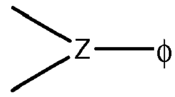

représente

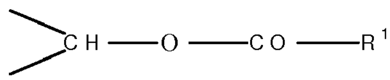

où $R^1$ est défini comme précédemment.

Lorsque l'on fait réagir ces monoesters de glycérol avec un diisocyanate, on introduit dans la chaîne polymère un greffon solubilisant et non pas une séquence, comme c'était le cas avec les dérivés difonctionnels cités précédemment.

Dans une variante, on utilisera un dérivé difonctionnel H-X-D-X-H choisi parmi les huiles portant trois groupes hydroxyles par chaîne, comme par exemple l'huile de ricin, hydrogénés ou non.

Dans ce cas, la réaction de polymérisation est réalisée avec un défaut de diisocyanate par rapport à la stoechiométrie de la réaction, pour éviter la réticulation du polymère et en conserver une bonne solubilité.

On peut aussi utiliser des diols à chaîne aliphatique longue. De manière avantageuse, on utilisera les diols de structure HO-D-OH où D est une chaîne alkyle linéaire ou ramifiée, comportant de 8 à 40 atomes de carbone. Ces diols sont commercialisés par la société ATOCHEM sous la dénomination VIKINOL®. On citera également le 1,12 dodécane diol et le 1,10 décane diol, ce dernier étant commercialisé par la société COGNIS sous le nom commercial de SOVERMOL 110® .

On peut également utiliser les diols de structure

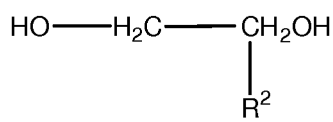

où $R^2$ est une chaîne alkyle, comportant de 8 à 40 atomes de carbone.

Ces diols à chaîne aliphatique longue sont, de manière préférée, utilisés avec l'un ou l'autre des dérivés H-X-D-X-H précédemment cités, pour servir de coupleurs de chaîne lors de la synthèse des polyuréthanes et/ou des polyurées.

Enfin, on peut utiliser comme dérivé difonctionnel H-X-D-X-H des dimères-diamines ou des diamines à chaîne aliphatique longue.

L'utilisation de tels réactifs dans la réaction de polymérisation permet d'introduire dans le polymère, des groupes urées au lieu des groupes uréthanes.

Selon un mode de réalisation particulier de l'invention, on utilisera des dimères-diamines ayant la même structure que les dimères-diols décrits précédemment, c'est-à-dire des dimères-diamines comportant deux fonctions amines primaires à la place des groupes hydroxyles.

Ces dimères-diamines peuvent être obtenues à partir de la transformation des acides gras dimères, comme les dimères-diols.

Dans une variante, on peut utiliser des diamines de structure $H_2N\text{-}D\text{-}NH_2$ où D est une chaîne alkyle linéaire ou ramifiée, comportant de 8 à 40 atomes de carbone. Ces diamines sont de préférence utilisées en mélange avec un dérivé difonctionnel H-X-D-X-H choisi parmi les dimères-diols et leurs dérivés, les polydiènes et polyoléfines à extrémités hydroxyles, les polyesters ramifiés à longue chaîne alkyle, et les huiles portant de 2 à 3 groupes hydroxyles, citées précédemment.

Parmi ces diamines, on peut citer:

- le 1,10-diamino décane et le 1,12 diamino dodécane, et

- les huiles diaminées suivantes commercialisées par la société AKZO NOBEL: cocopropylène diamine (distillée ou non) DUOMEEN® C ou CD, Tallowpropylène diamine hydrogénée DUOMEEN® HT, $C_{16-22}$ alkylpropylène diamine DUOMEEN® M, oléylpropylène diamine DUOMEEN® O, Tallowpropylène diamine DUOMEEN® T.

En ce qui concerne les dérivés monofonctionnels $A_1$-XH et $A_2$-XH, ils sont avantageusement choisis parmi les monoalcools ou monoamines à chaînes alkyles linéaires ou ramifiées comportant de 1 à 80 atomes de carbone, les huiles naturelles ou synthétiques portant un seul groupe hydroxyle par chaîne, comme par exemple les diesters de glycérols ou les triesters d'acide citrique et d'alcool gras.

Les réactions de polycondensations envisagées sont classiquement réalisées dans un solvant organique, apte à dissoudre les réactifs et le polymère formé. Ce solvant est de préférence facilement éliminable en fin de réaction, notamment par distillation et ne réagit pas avec les groupes isocyanates.

Généralement, chacun des réactifs est dissous dans une partie du solvant organique avant la réaction de polymérisation.

Il est parfois souhaité d'utiliser un catalyseur pour activer la polymérisation. Celui-ci sera généralement choisi parmi les catalyseurs couramment utilisés dans la chimie des polyuréthanes et des polyurées, comme par exemple le 2-éthyl hexanoate d'étain.

La proportion molaire entre les réactifs principaux de la réaction de polymérisation dépend de la structure chimique et du poids moléculaire des polymères (polyuréthanes et/ou polyurées) que l'on souhaite obtenir, comme cela est classiquement le cas dans la chimie des polyuréthanes et des polyurées. De même, l'ordre d'introduction des réactifs sera adapté à cette chimie.

Ainsi, la réaction de deux moles de dérivé fonctionnel H-X-D-X-H avec une mole de diisocyanate donne, après consommation totale des réactifs un polymère défini par la formule I:

I) 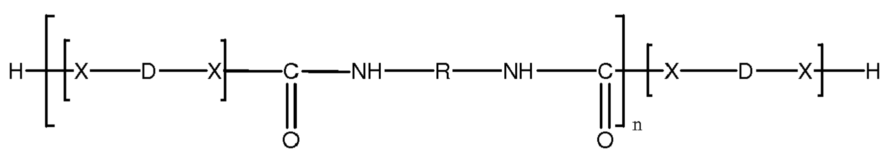

Pour cette réaction, on procédera avantageusement de la manière suivante:

- le milieu initial est une solution comprenant deux moles de dérivé H-X-D-X-H, par exemple deux moles de dimère-diol, dans un solvant, par exemple le tétrahydrofuranne,
- on ajoute goutte à goutte, dans cette solution initiale, une solution comprenant une mole de diisocyanate dissous dans le même solvant, comme par exemple du toluène diisocyanate dissous dans le tétrahydrofuranne.

Par ailleurs, la réaction équimolaire d'un dérivé difonctionnel H-X-D-X-H avec un diisocyanate, avec consommation des isocyanates résiduels par un composé monofonctionnel $A_1$-XH, donne un polymère défini par la formule III:

III) 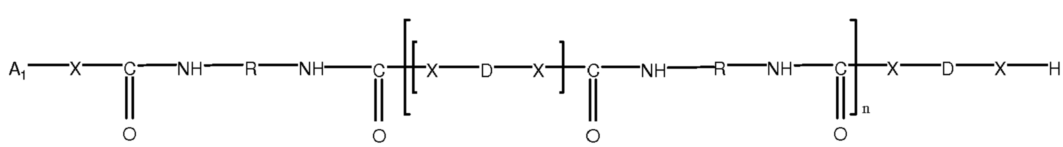

Cette réaction se fera alors, de préférence, par un ajout simultané, dans un réacteur, d'une solution organique d'une mole de H-X-D-X-H, comme par exemple un POLYTAIL® décrit précédemment, et d'une solution organique d'une mole de diisocyanate, comme par exemple le 4,4'-dicyclohexylméthane diisocyanate. L'ajout simultané de ces deux solutions organiques est également appelé " double coulée ". En fin de double coulée, le mélange réactionnel est chauffé à 60°C pendant 5 heures. Puis, on prélève un échantillon du milieu réactionnel pour doser les isocyanates résiduels en utilisant une méthode connue par l'homme de l'art. Enfin, on ajoute au milieu réactionnel, une solution d'un composé monofonctionnel choisi $A_1$-X-H, en quantité suffisante pour consommer les isocyanates résiduels, cette quantité ayant été estimée à partir du dosage des isocyanates résiduels. On utilisera avantageusement comme dérivé monofonctionnel $A_1$-X-H le 1-décanol.

Enfin, la réaction entre

- une mole de composé H-X-D-X-H, comme par exemple un dimère-diol,

- trois moles de diisocyanate comme par exemple le 4,4'-dicyclohexyl méthane diisocyanate, et
- deux moles de coupleur de structure

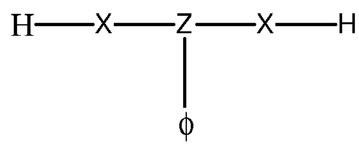

où ϕ est une chaîne aliphatique, linéaire, ramifiée ou cyclique comportant de 8 à 20 atomes de carbone, conduit à la formation d'un polymère à la fois séquencé et greffé de structure:

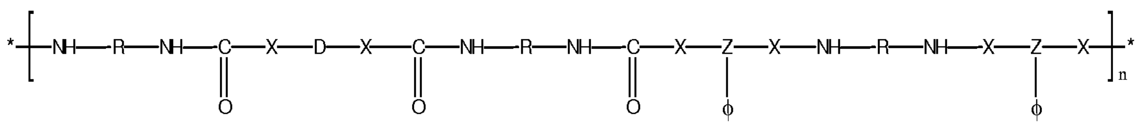

Les isocyanates éventuellement résiduels pouvant être consommés par ajout d'une quantité appropriée de réactif monofonctionnel $A_1$-X-H.

Pour obtenir un tel polymère, on procède de la manière suivante:

- le milieu réactionnel initial est constitué par une solution comprenant une mole d'un dérivé difonctionnel H-X-D-X-H,
- on ajoute à ce milieu, goutte à goutte, une solution de trois moles de diisocyanate,
- on laisse ensuite réagir pendant 3 heures à 60˚C:

- puis, on coule dans ce milieu, une solution organique comprenant deux moles d'un coupleur, défini par la formule

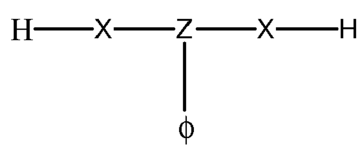

les isocyanates éventuellement résiduels pourront être consommés par ajout d'une quantité appropriée de réactif monofonctionnel $A_1$-XH.

*Polymères siliconés lipophiles*

Les agents structurants lipophiles polymériques siliconés sont par exemple des polymères du type polyorganosiloxane comme ceux décrits dans les documents US-A-5 874 069, US-A-5,919,441, US-A-6,051,216 et US-A-5,981,680. Selon l'invention, les polymères utilisés comme agent structurant peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.

Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.

Selon une première variante, les polymères siliconés sont des polyorganosiloxanes tels que définis ci-dessus et dont les motifs capables d'établir des interactions hydrogènes sont disposés dans la chaîne du polymère.

Les polymères siliconés peuvent plus particulièrement être des polymères comprenant au moins un motif répondant à la formule générale I :

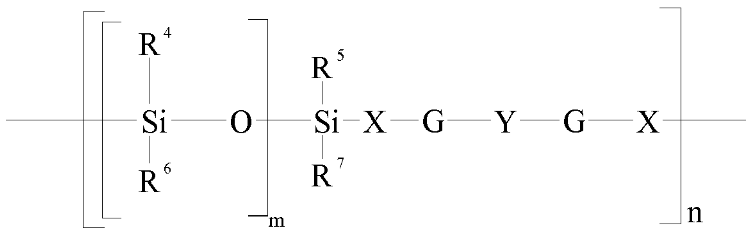

(I)

dans laquelle :

1) $R^4$, $R^5$, $R^6$ et $R^7$, identiques ou différents, représentent un groupe choisi parmi :

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote,

2) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou
4) Y représente un groupe répondant à la formule :

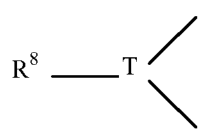

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S,

ou T représente un atome trivalent choisi parmi N, P et Al, et

- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5) les G, identiques ou différents, représentent les groupes divalents choisis parmi :

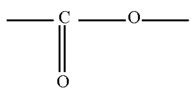 ; 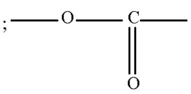 ; 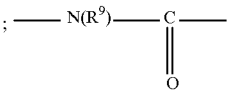 ;

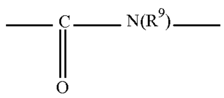 ; 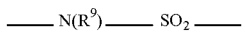 ; 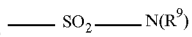 ;

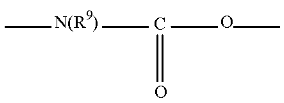 ; 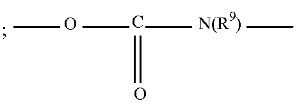 ; 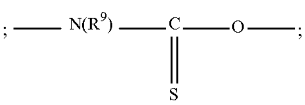 ;

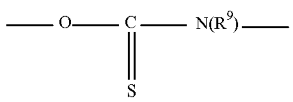 ; 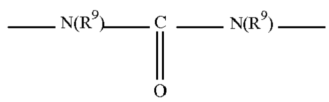 ,

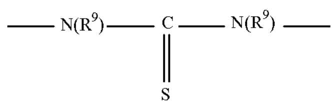 ,

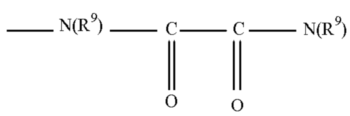 ; 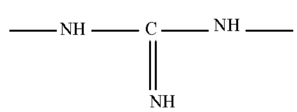 ;

et

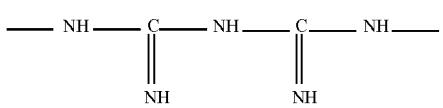

où $R^9$ représente un atome d'hydrogène ou un groupe alkyle, linéaire ou ramifié, en $C_1$ à $C_{20}$, à condition qu'au moins 50 % des $R^9$ du polymère représente un atome d'hydrogène et qu'au moins deux des groupes G du polymère soient un autre groupe que :

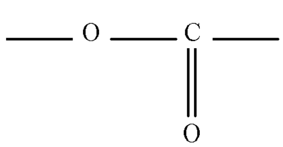 et 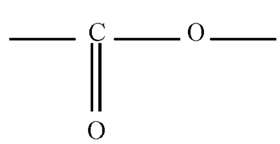 ;

6) n est un nombre entier allant de 2 à 500, de préférence de 2 à 200, et m est un nombre entier allant de 1 à 1000,

de préférence de 1 à 700 et mieux encore de 6 à 200. Selon l'invention, 80 % des $R^4$, $R^5$, $R^6$ et $R^7$, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :

a) les groupes alkylène linéaires en $C_1$ à $C_{20}$, de préférence en $C_1$ à $C_{10}$,
b) les groupes alkylène ramifiés pouvant comporter des cycles et des insaturations non conjuguées, en $C_{30}$ à $C_{56}$,
c) les groupes cycloalkylène en $C_5$-$C_6$,
d) les groupes phénylène éventuellement substitués par un ou plusieurs groupes alkyle en $C_1$ à $C_{40}$,
e) les groupes alkylène en $C_1$ à $C_{20}$, comportant de 1 à 5 groupes amides,
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$,
g) les chaînes polyorganosiloxane de formule :

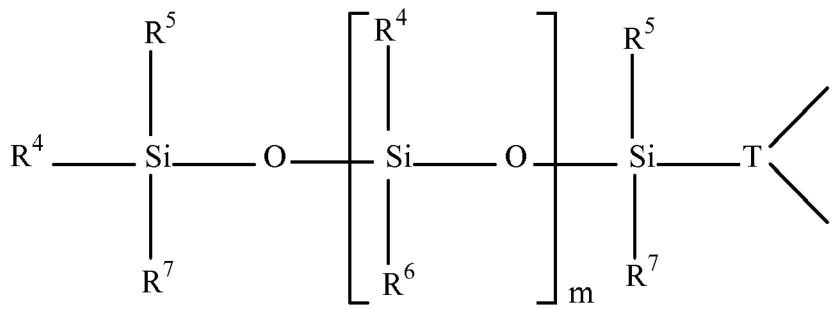

dans laquelle $R^4$, $R^5$, $R^6$, $R^7$, T et m sont tels que définis ci-dessus, et
h) les chaînes polyorganosiloxanes de formule :

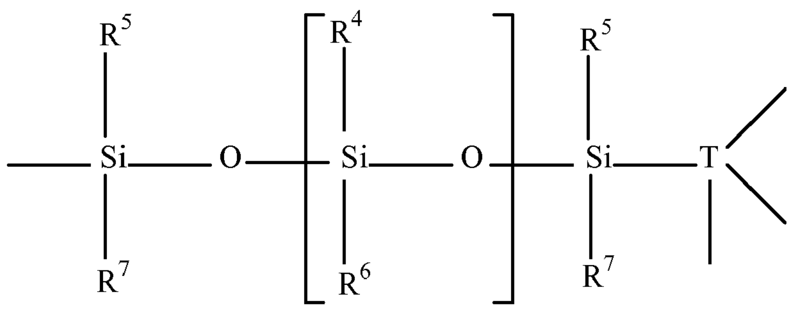

Selon la seconde variante, les polyorganosiloxanes peuvent être des polymères comprenant au moins un motif répondant à la formule (II) :

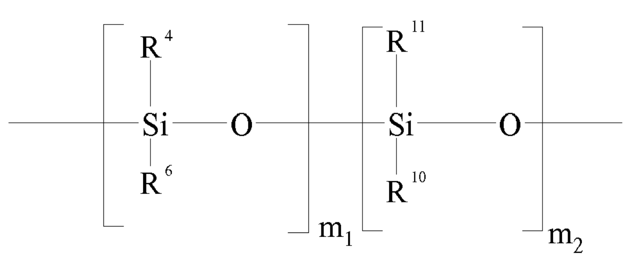

(II)

dans laquelle

- $R^4$ et $R^6$, identiques ou différents, sont tels que définis ci-dessus pour la formule (I),
- $R^{10}$ représente un groupe tel que défini ci-dessus pour $R^4$ et $R^6$, ou représente le groupe de formule -X-G-$R^{12}$ dans laquelle X et G sont tels que définis ci-dessus pour la formule (I) et $R^{12}$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$,
- $R^{11}$ représente le groupe de formule -X-G-$R^{12}$ dans laquelle X, G et $R^{12}$ sont tels que définis ci-dessus,
- $m_1$ est un nombre entier allant de 1 à 998, et
- $m_2$ est un nombre entier allant de 2 à 500.

Selon l'invention, le polymère siliconé utilisé comme agent structurant, peut être un homopolymère, c'est-à-dire un polymère comportant plusieurs motifs identiques, en particulier des motifs de formule (I) ou de formule (II).

Selon l'invention, on peut aussi utiliser un polymère siliconé constitué par un copolymère comportant plusieurs motifs de formule (I) différents, c'est-à-dire un polymère

dans lequel l'un au moins des $R^4$, $R^5$, $R^6$, $R^7$, X, G, Y, m et n est différent dans l'un des motifs. Le copolymère peut être aussi formé de plusieurs motifs de formule (II), dans lequel l'un au moins des $R^4$, $R^6$, $R^{10}$, $R^{11}$, m1 et m2 est différent dans l'un au moins des motifs.

On peut encore utiliser un polymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un polymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des polymères blocs, des polymères séquencés ou des polymères greffés.

Selon un mode de réalisation avantageux de l'invention, les groupes capables d'établir des interactions hydrogènes sont des groupes amides de formule -C(O)NH- et - HN-C(O)-. Dans ce cas, l'agent structurant peut être un polymère comprenant au moins un motif de formule (III) ou (IV) :

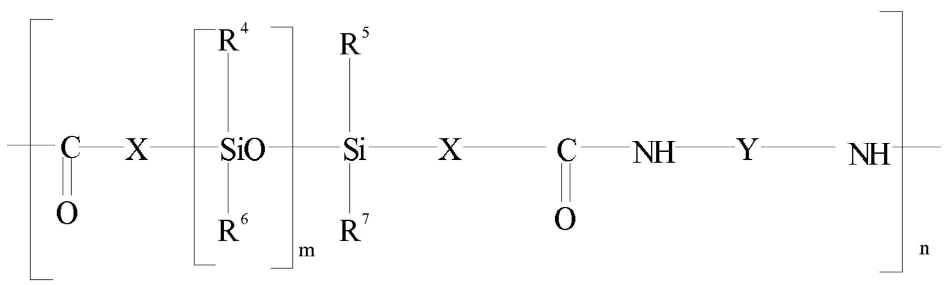

(III)

ou

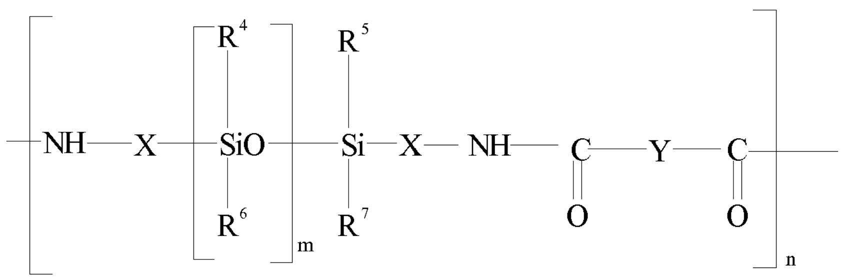

(IV)

dans lesquelles $R^4$, $R^5$, $R^6$, $R^7$, X, Y, m et n sont tels que définis ci-dessus.

Un tel motif peut être obtenu :

- soit par une réaction de condensation entre un silicone à extrémités α, ω-acides carboxyliques et une ou plusieurs diamines, selon le schéma réactionnel suivant :

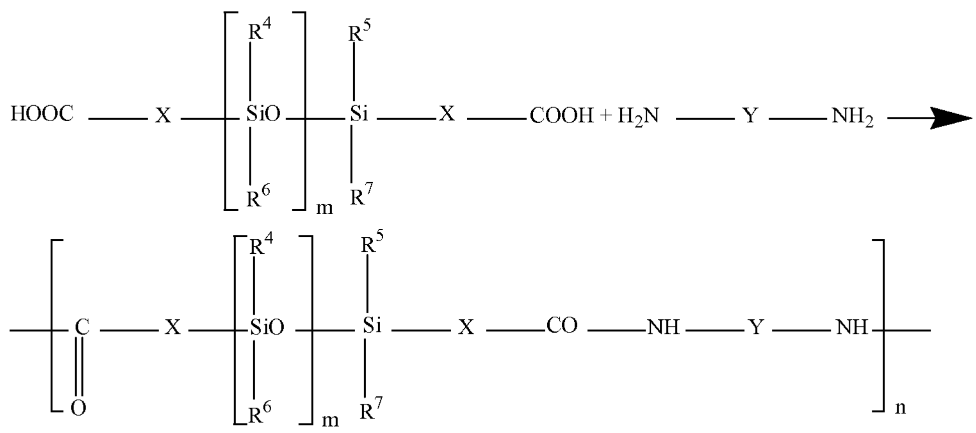

- soit par réaction de deux molécules d'acide carboxylique α-insaturé avec une diamine selon le schéma réactionnel suivant :

$CH_2=CH-X^1-COOH+H_2N-Y-NH_2 \rightarrow$

$CH_2=CH-X^1-CO-NH-Y-NH-CO-X^1-CH=CH_2$

suivie de l'addition d'un siloxane sur les insaturations éthyléniques, selon le schéma suivant :

$CH_2=CH-X^1-CO-NH-Y-NH-CO-X^1-CH=CH_2$

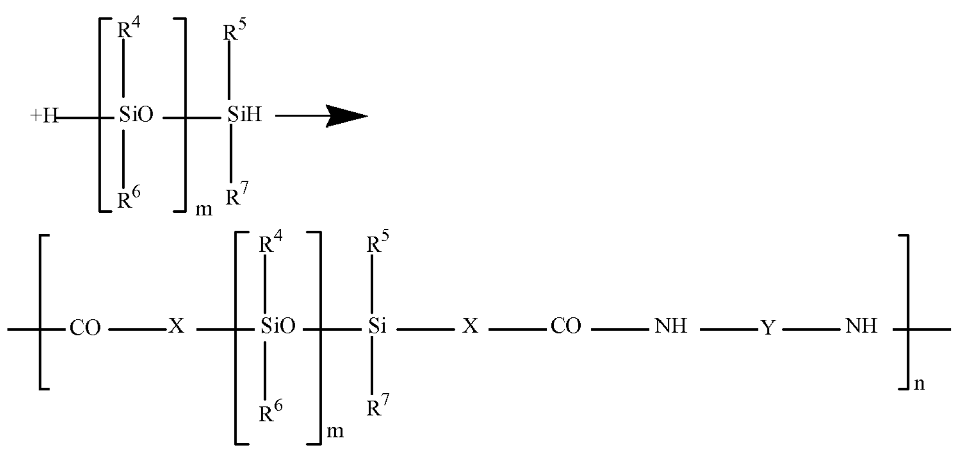

dans lesquels $X^1-(CH_2)_2-$ correspond au X défini ci-dessus et Y, $R^4$, $R^5$, $R^6$, $R^7$ et m sont tels que définis ci-dessus,

- soit par réaction d'un silicone à extrémités α, ω-$NH_2$ et d'un diacide de formule HOOC-Y-COOH selon le schéma réactionnel suivant :

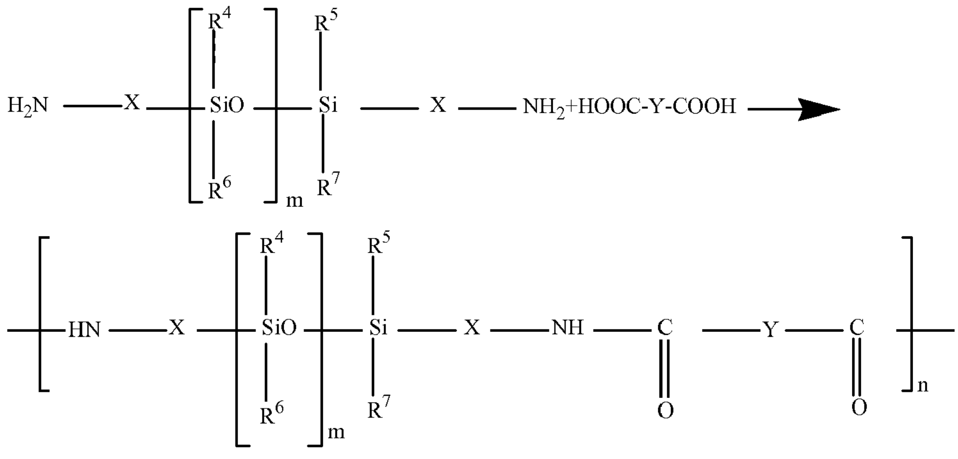

Dans ces polyamides de formule (III) ou (IV), m va de 1 à 700, en particulier de 15 à 500 et notamment de 50 à 200 et n va particulier de 1 à 500, de préférence de 1 à 100 et mieux encore de 4 à 25,

- X est de préférence une chaîne alkylène linéaire ou ramifiée ayant de 1 à 30 atomes de carbone, en particulier 1 à 20 atomes de carbone, notamment de 5 à 15 atomes de carbone et plus particulièrement de 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.

Dans les formules (III) et (IV), le groupe alkylène représentant X ou Y peut éventuellement contenir dans sa partie alkylène au moins l'un des éléments suivants :

1) 1 à 5 groupes amides, urée, uréthane, ou carbamate,
2) un groupe cycloalkyle en $C_5$ ou $C_6$, et
3) un groupe phénylène éventuellement substitué par 1 à 3 groupes alkyles identiques ou différents en $C_1$ à $C_3$.

Dans les formules (III) et (IV), les groupes alkylènes peuvent aussi être substitués par au moins un élément choisi dans le groupe constitué de :

- un groupe hydroxy,
- un groupe cycloalkyle en $C_3$ à $C_8$,
- un à trois groupes alkyles en $C_1$ à $C_{40}$,
- un groupe phényle éventuellement substitué par un à trois groupes alkyles en $C_1$ à $C_3$,
- un groupe hydroxyalkyle en $C_1$ à $C_3$, et
- un groupe aminoalkyle en $C_1$ à $C_6$.

Dans ces formules (III) et (IV), Y peut aussi représenter :

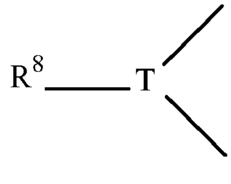

où $R^8$ représente une chaîne polyorganosiloxane, et T représente un groupe de formule :

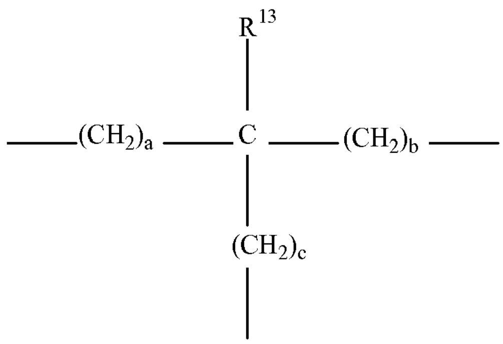

ou

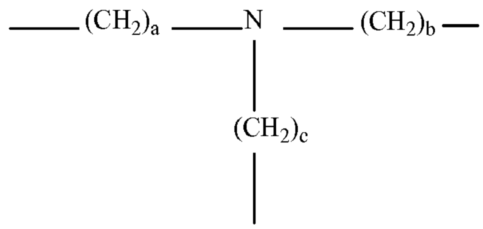

dans lesquelles a, b et c sont indépendamment des nombres entiers allant de 1 à 10, et $R^{13}$ est un atome d'hydrogène ou un groupe tel que ceux définis pour $R^4$, $R^5$, $R^6$ et $R^7$.

Dans les formules (III) et (IV), $R^4$, $R^5$, $R^6$ et $R^7$ représentent de préférence, indépendamment, un groupe alkyle en $C_1$ à $C_{40}$, linéaire ou ramifié, de préférence un groupe $CH_3$, $C_2H_5$, n-$C_3H_7$ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (III) ou (IV) identiques ou différents.

Ainsi, le polymère peut être un polyamide contenant plusieurs motifs de formule (III) ou (IV) de longueurs différentes, soit un polyamide répondant à la formule (V) :

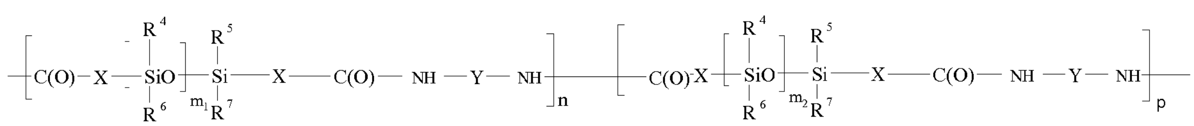

(V)

dans laquelle X, Y, n, $R^4$ à $R^7$ ont les significations données ci-dessus, $m_1$ et $m_2$ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le polymère peut répondre à la formule VI:

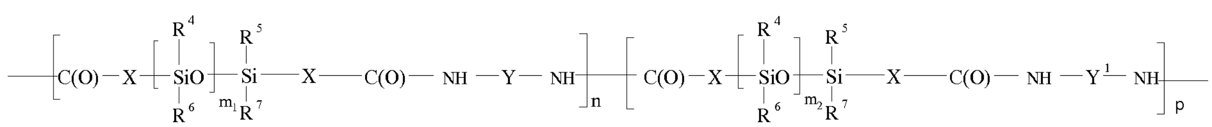

(VI)

dans laquelle $R^4$ à $R^7$, X, Y, $m_1$, $m_2$, n et p ont les significations données ci-dessus et $Y^1$ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, l'agent structurant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le polymère peut comprendre au moins un motif de formule (VII)

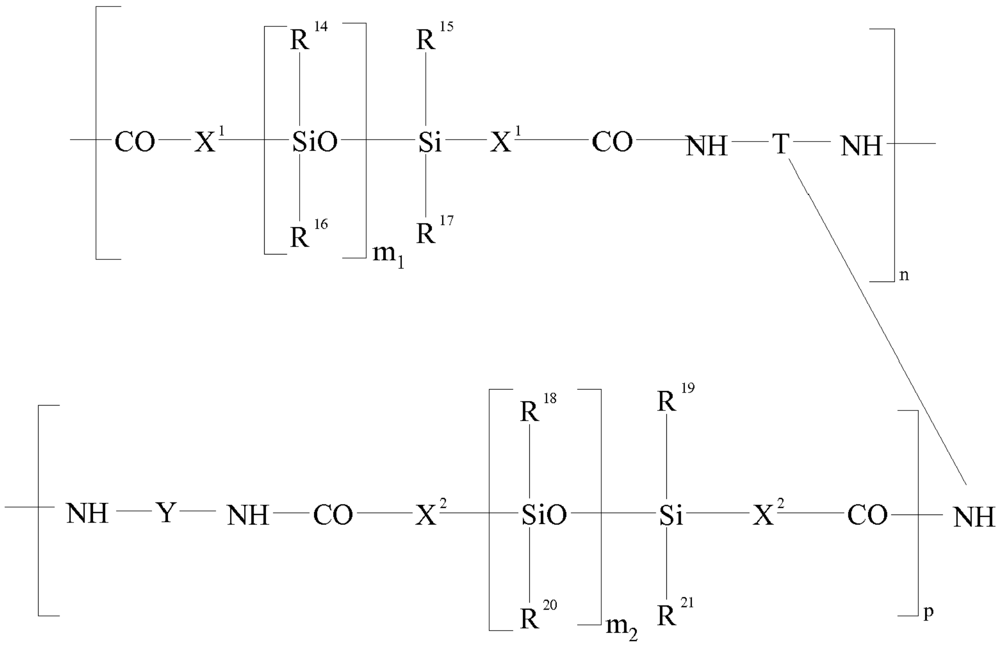

(VII)

dans laquelle $X^1$ et $X^2$ qui sont identiques ou différents, ont la signification donnée pour X dans la formule (I), n est tel que défini dans la formule (I), Y et T sont tels que définis dans la formule (I), $R^{14}$ à $R^{21}$ sont des groupes choisis dans le même groupe que les $R^4$ à $R^7$, $m_1$ et $m_2$ sont des nombres situés dans la gamme allant de 1 à 1 000, et p est un nombre entier allant de 2 à 500.

Dans la formule (VII), on préfère que :

- p soit va de 1 à 25, mieux encore de 1 à 7,
- $R^{14}$ à $R^{21}$ soient des groupes méthyle,
- T réponde à l'une des formules suivantes :

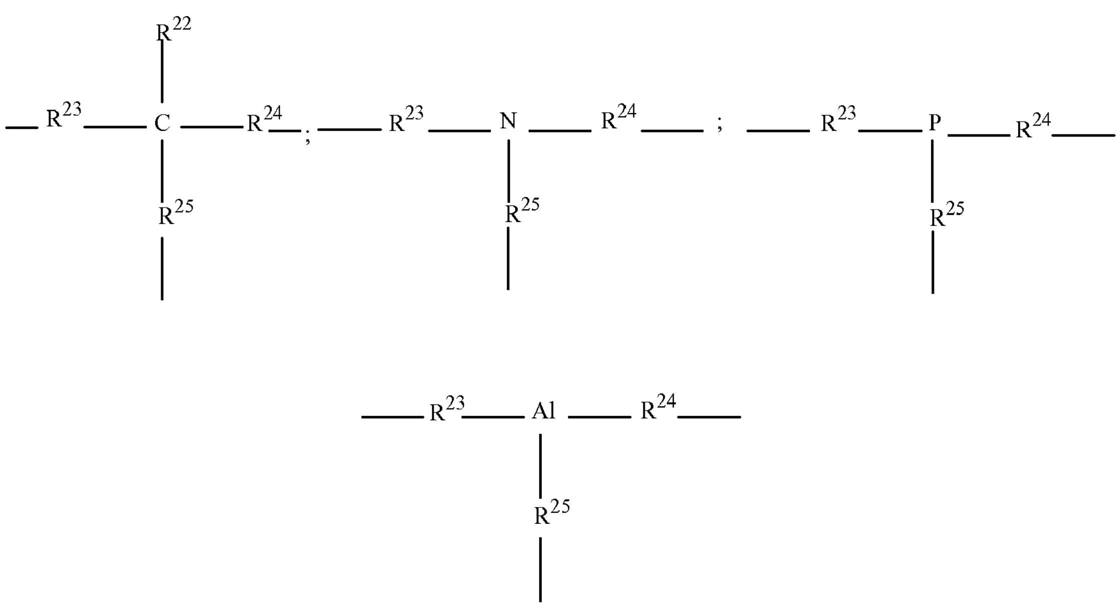

dans lesquelles $R^{22}$ est un atome d'hydrogène ou un groupe choisi parmi les groupes définis pour $R^4$ à $R^7$, et $R^{23}$, $R^{24}$ et $R^{25}$ sont indépendamment des groupes alkylène, linéaires ou ramifiés, de préférence encore, à la formule :

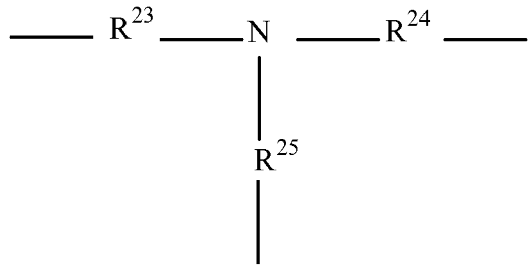

en particulier avec $R^{23}$, $R^{24}$ et $R^{25}$ représentant $-CH_2-CH_2-$,

- $m_1$ et $m_2$ vont de 15 à 500, et mieux encore de 15 à 45,
- $X_1$ et $X_2$ représentent $-(CH_2)_{10}-$, et
- Y représente $-CH_2-$.

Ces polyamides à motif silicone greffé de formule (VII) peuvent être copolymérisés avec des polyamides-silicones de formule (II) pour former des copolymères blocs, des copolymères alternés ou des copolymères aléatoires. Le pourcentage en poids de motifs silicone greffé (VII) dans le copolymère peut aller de 0,5 à 30 % en poids.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon une variante de réalisation de l'invention, on peut utiliser un copolymère de polyamide silicone et de polyamide hydrocarboné, soit un copolymère comportant des motifs de formule (III) ou (IV) et des motifs polyamide hydrocarboné. Dans ce cas, les motifs polyamide-silicone peuvent être disposés aux extrémités du polyamide hydrocarboné.

Avantageusement, la composition selon l'invention comprend au moins un polymère bloc polydiméthylsiloxane de formule générale (I) possédant un indice m de valeur environ 15.

De préférence encore, la composition selon l'invention comprend au moins un polymère comprenant au moins un motif de formule (III) où m va de 5 à 100, en particulier de 10 à 75 et plus particulièrement est de l'ordre de 15 ; de préférence encore $R^4$, $R^5$, $R^6$ et $R^7$ représentent indépendamment, un groupe alkyle en $C_1$ à $C_{40}$, linéaire ou ramifié, de préférence un groupe $CH_3$, $C_2H_5$, $n-C_3H_7$ ou isopropyle dans la formule (III).

A titre d'exemple de polymère siliconé utilisable, on peut citer un des polyamides siliconés, obtenus conformément aux exemples 1 à 3 du document US-A-5 981 680.

Selon une variante de réalisation de l'invention, le polymère est constitué par un homopolymère ou copolymère comportant des groupes uréthane ou urée. Ces polymères sont décrits en détail dans la demande WO 2003/106614 publiée le 24/12/2003 dont le contenu est incorporé dans la présente demande par référence

Comme précédemment, un tel polymère peut comporter des motifs polyorganosiloxanes contenant deux ou plusieurs groupes uréthanes et/ou urées, soit dans le squelette du polymère, soit sur des chaînes latérales ou comme groupes pendants. Les polymères comportant au moins deux groupes uréthanes et/ou urées dans le squelette peuvent être des polymères comprenant au moins un motif répondant à la formule suivante (VIII) :

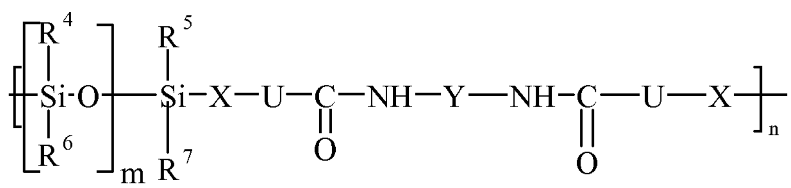

(VIII)

dans laquelle les $R^4$, $R^5$, $R^6$, $R^7$, X, Y, m et n ont les significations données ci-dessus pour la formule (I), et U représente -O- ou -NH-, afin que :

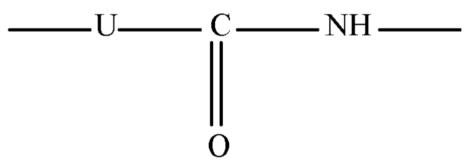

corresponde à un groupe uréthane ou urée.

Dans cette formule (VIII), Y peut être un groupe alkylène, en $C_1$ à $C_{40}$, linéaire ou ramifié, substitué éventuellement par un groupe alkyle en $C_1$ à $C_{15}$ ou un groupe aryle en $C_5$ à $C_{10}$. De préférence, on utilise un groupe $-(CH_2)_6-$.

Y peut aussi représenter un groupe cycloaliphatique ou aromatique en $C_5$ à $C_{12}$ pouvant être substitué par un groupe alkyle en $C_1$ à $C_{15}$ ou un groupe aryle en $C_5$ à $C_{10}$, par exemple un radical choisi parmi le radical méthylène-4-4-biscyclohexyle, le radical dérivé de l'isophorone diisocyanate, les 2,4 et 2,6-tolylènes, le 1,5-naphtylène, le p-phénylène et le 4,4'-biphénylène méthane. Généralement, on préfère que Y représente un radical alkylène en $C_1$ à $C_{40}$, linéaire ou ramifié, ou un radical cycloalkylène en $C_4$ à $C_{12.}$

Y peut aussi représenter une séquence polyuréthane ou polyurée correspondant à la condensation de plusieurs molécules de diisocyanate avec une ou plusieurs molécules de coupleurs du type diol ou diamine. Dans ce cas, Y comprend plusieurs groupes uréthane ou urée dans la chaîne alkylène.

Il peut répondre à la formule (IX) :

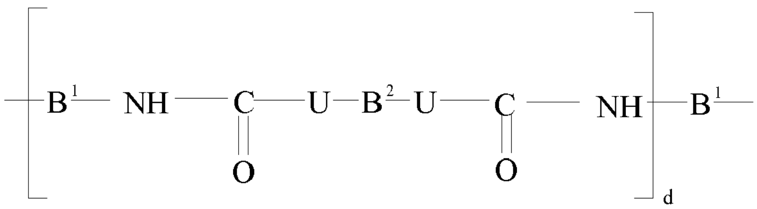

(IX)

dans laquelle $B^1$ est un groupe choisi parmi les groupes donnés ci-dessus pour Y, U est -O-ou -NH-, et $B^2$ est choisi parmi :

- les groupes alkylène en $C_1$ à $C_{40}$, linéaires ou ramifiés,
- les groupes cycloalkylène en $C_5$ à $C_{12}$, éventuellement porteurs de substituants alkyle, par exemple un à trois groupes méthyle ou éthyle, ou alkylène, par exemple le radical du diol : cyclohexane diméthanol,
- les groupes phénylène pouvant éventuellement être porteurs de substituants alkyles en $C_1$ à $C_3$, et
- les groupes de formule :

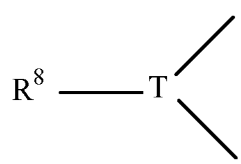

dans laquelle T est un radical trivalent hydrocarboné pouvant contenir un ou plusieurs hétéroatomes tels que l'oxygène, le soufre et l'azote et $R^8$ est une chaîne polyorganosiloxane ou une chaîne alkyle en $C_1$ à $C_{50}$, linéaire ou ramifiée.

T peut représenter par exemple :

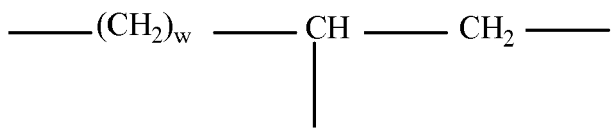

ou

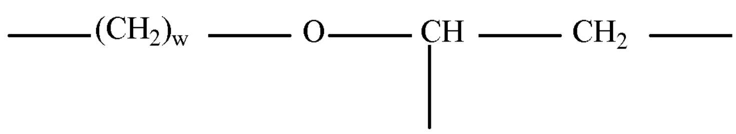

avec w étant un nombre entier allant de 1 à 10 et $R^8$ étant une chaîne polyorganosiloxane. Lorsque Y est un groupe alkylène, en $C_1$ en $C_{40}$ linéaire ou ramifié, on préfère les groupes $-(CH_2)_2-$ et $-(CH_2)_6-$.
Dans la formule donnée ci-dessus pour Y, d peut être un entier allant de 0 à 5, de préférence de 0 à 3, de préférence encore égal à 1 ou 2.
De préférence $B^2$ est un groupe alkylène en $C_1$ à $C_{40}$, linéaire ou ramifié, en particulier $-(CH_2)_2-$ ou $-(CH_2)_6-$, ou le groupe :

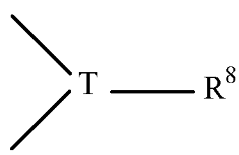

avec $R^8$ étant une chaîne polyorganosiloxane.
Comme précédemment, le polymère constituant le copolymère texturant peut être formé de motifs silicone uréthane et/ou silicone-urée de longueur et/ou de constitution différentes, et se présenter sous la forme de copolymères blocs, séquencés ou statistiques (aléatoires).
Les polymères de formule (VIII) comportant des groupes urées ou uréthanes dans la chaîne du polymère siliconé peuvent être obtenus par réaction entre un silicone à groupes terminaux $\alpha,\omega-NH_2$ ou -OH, de formule :

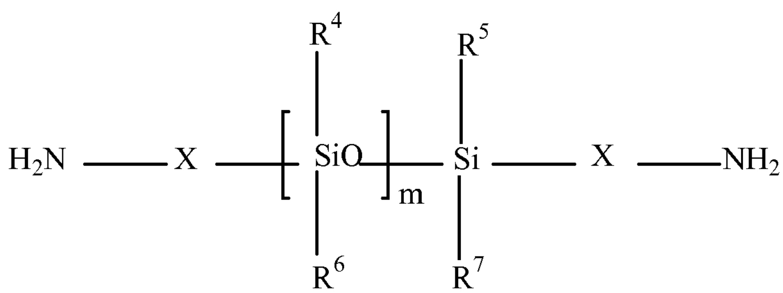

dans laquelle m, $R^4$, $R^5$, $R^6$, $R^7$ et X sont tels que définis pour la formule (I), et un diisocyanate OCN-Y-NCO où Y a la signification donnée dans la formule (I) ; et éventuellement un coupleur diol ou diamine de formule $H_2N-B^2-NH_2$ ou $HO-B^2-OH$, où $B^2$ est tel que défini dans la formule (IX).
Suivant les proportions stoechiométriques entre les deux réactifs, diisocyanate et coupleur, on pourra avoir pour Y la formule (IX) avec d égale 0 où d égale 1 à 5.
Comme dans le cas des polyamides silicones de formule (IV), (II) ou (III), on peut utiliser dans l'invention des polyuréthanes ou des polyurées silicones ayant des motifs de longueur et de structure différentes, en particulier des motifs de longueurs différentes par le nombre d'unités silicones. Dans ce cas, le copolymère peut répondre par exemple à la formule :

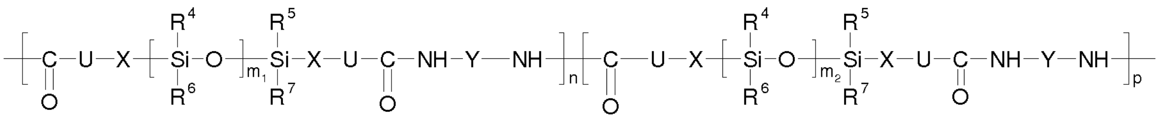

(XII)

dans laquelle $R^4$, $R^5$, $R^6$, $R^7$, X, Y et U sont tels que définis pour la formule (VIII) et $m_1$, $m_2$, n et p sont tels que définis pour la formule (V).

Selon l'invention, le silicone peut aussi comporter les groupes uréthane et/ou urée non plus dans le squelette mais en ramifications latérales. Dans ce cas, le polymère peut comprendre au moins un motif de formule :

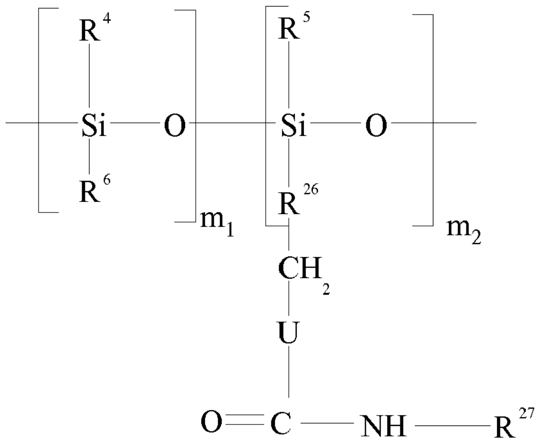

(X)

dans laquelle $R^4$, $R^6$, $R^5$, $m_1$ et $m_2$ ont les significations données ci-dessus pour la formule (II), et $R^5$ pour la formule (I),

- U représente O ou NH,
- $R^{26}$ représente un groupe alkylène en $C_1$ à $C_{40}$, comportant éventuellement un ou plusieurs hétéroatomes choisis parmi O et N, ou un groupe phénylène, et
- $R^{27}$ est choisi parmi les groupes alkyle en $C_1$ à $C_{50}$, linéaires, ramifiés ou cycliques, saturés ou insaturés, et les groupes phényle éventuellement substitués par un à trois groupes alkyles en $C_1$ à $C_3$.

Les polymères comportant au moins un motif de formule (X) contiennent des unités siloxanes et des groupes urées ou uréthanes, et ils peuvent être utilisés comme copolymère texturant dans les compositions de l'invention.

Les polymères siloxanes peuvent avoir un seul groupe urée ou uréthane par ramification ou peuvent avoir des ramifications à deux groupes urée ou uréthane, ou encore contenir un mélange de ramifications à un groupe urée ou uréthane et de ramifications à deux groupes urée ou uréthane.

Ils peuvent être obtenus à partir de polysiloxanes ramifiés, comportant un ou deux groupes amino par ramification, en faisant réagir ces polysiloxanes avec des monoisocyanates.

A titre d'exemples de polymères de départ de ce type ayant des ramifications amino et diamino, on peut citer les polymères répondant aux formules suivantes :

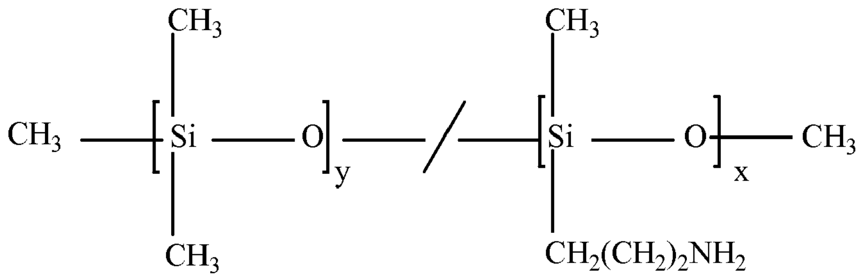

y=57 ; x = 3

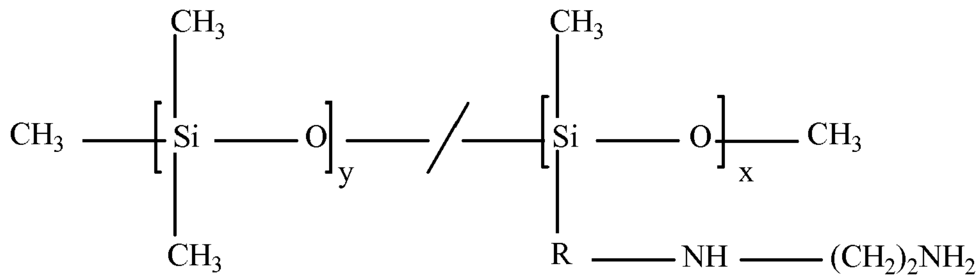

y=56 ; x = 4

Dans ces formules, le symbole "/" indique que les segments peuvent être de longueurs différentes et dans un ordre aléatoire, et R représente un groupe aliphatique linéaire ayant de préférence 1 à 6 atomes de carbone et mieux encore 1 à 3 atomes de carbone.

De tels polymères à ramification peuvent être formés en faisant réagir un polymère siloxane, ayant au moins trois groupes amino par molécule de polymère, avec un composé ayant un seul groupe monofonctionnel (par exemple un acide, un isocyanate ou isothiocyanate) pour faire réagir ce groupe monofonctionnel avec l'un des groupes amino et former les groupes capables d'établir des interactions hydrogène. Les groupes amino peuvent être sur des chaînes latérales s'étendant de la chaîne principale du polymère siloxane de sorte que les groupes capables d'établir des interactions hydrogène sont formés sur ces chaînes latérales, ou bien les groupes amino peuvent être aux extrémités de la chaîne principale de sorte que les groupes capables d'interaction hydrogène seront des groupes terminaux du polymère.

Comme mode opératoire pour former un polymère contenant des unités siloxanes et des groupes capables d'établir des interactions hydrogène, on peut citer la réaction d'une siloxane diamine et d'un diisocyanate dans un solvant siliconé de façon à fournir directement un gel. La réaction peut être exécutée dans un fluide siliconé, le produit résultant étant dissous dans le fluide siliconé, à température élevée, la température du système étant ensuite diminuée pour former le gel.

Les polymères préférés pour l'incorporation dans les compositions selon la présente invention, sont des copolymères siloxanes-urées qui sont linéaires et qui contiennent des groupes urées comme groupes capables d'établir des interactions hydrogène dans le squelette du polymère.

A titre d'illustration d'un polysiloxane terminé par quatre groupes urées, on peut citer le polymère de formule :

(Ph = Phényle)

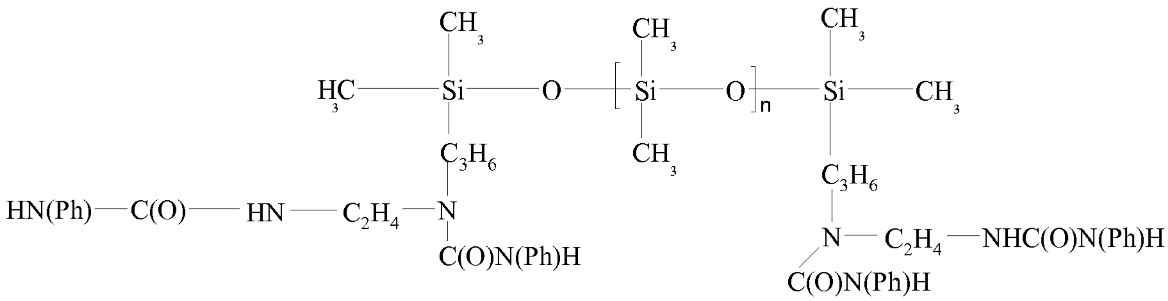

(XI)

où Ph est un groupe phényle et n est un nombre de 0 à 300, en particulier de 0 à 100, par exemple de 50. Ce polymère est obtenu par réaction du polysiloxane à groupes amino suivant :

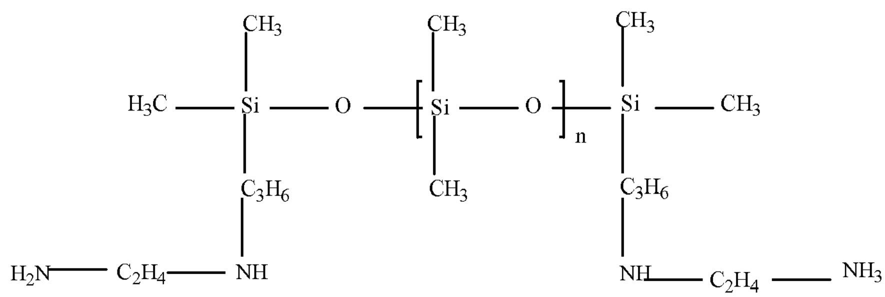

(n-50)

avec l'isocyanate de phényle.

On peut obtenir également des polyuréthanes ou polyurées silicones ramifiés en utilisant à la place du diisocyanate OCN-Y-NCO, un triisocyanate de formule :

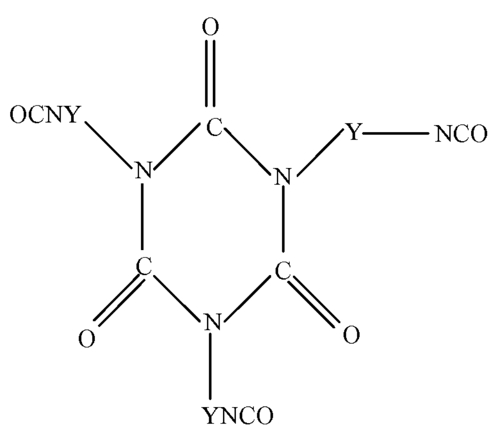

On obtient ainsi une polyuréthane ou polyurée silicone ayant des ramifications comportant une chaîne organosiloxane avec des groupes capables d'établir des interactions hydrogène. Un tel polymère comprend par exemple un motif répondant à la formule :

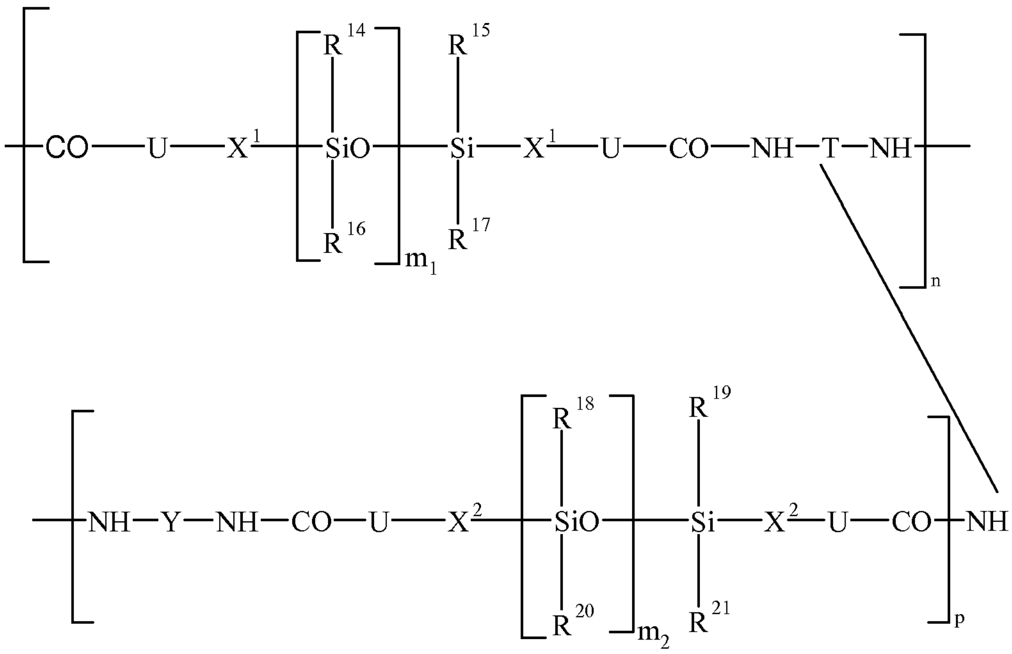

(XIII)

dans laquelle $X^1$ et $X^2$ qui sont identiques ou différents, ont la signification donnée pour X dans la formule (I), n est tel que défini dans la formule (I), Y et T sont tels que définis dans la formule (I), $R^{14}$ à $R^{21}$ sont des groupes choisis dans le même groupe que les $R^4$ à $R^7$ $m_1$ et $m_2$ sont des nombres situés dans la gamme allant de 1 à 1 000, et p est un nombre entier allant de 2 à 500.

Comme dans le cas des polyamides, on peut utiliser dans l'invention des copolymères de polyuréthane -ou de polyurée-silicone et de polyuréthane ou polyurée hydrocarboné en réalisant la réaction de synthèse du polymère en présence d'une séquence α, ω-difonctionnelle de nature non silicone, par exemple un polyester, un polyéther ou une polyoléfine.

Comme on l'a vu précédemment, les copolymères de l'invention peuvent avoir des motifs siloxanes dans la chaîne principale du polymère et des groupes capables d'établir des interactions hydrogène, soit dans la chaîne principale du polymère ou aux extrémités de celle-ci, soit sur des chaînes latérales ou ramifications de la chaîne principale. Ceci peut correspondre aux cinq dispositions suivantes :

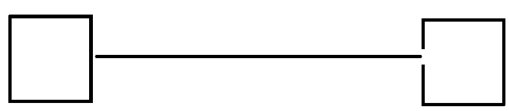

(1)

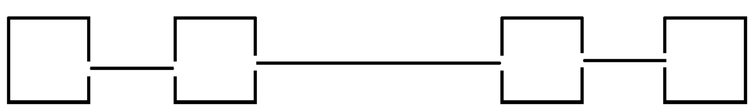

(2)

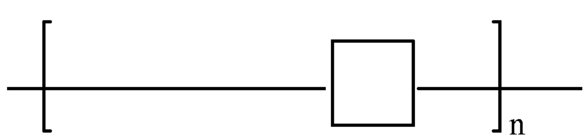

(3)

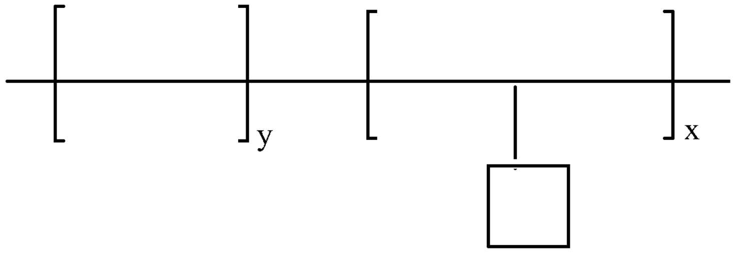

(4)

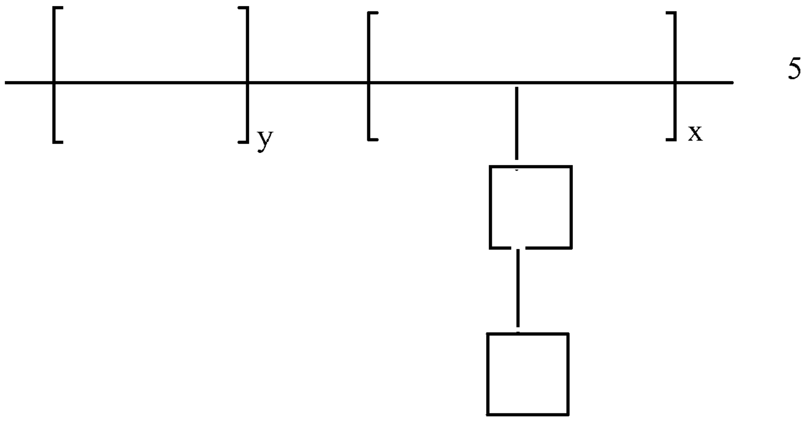

(5)

dans lesquelles, la ligne continue est la chaîne principale du polymère siloxane et les carrés représentent les groupes capables d'établir des interactions hydrogène. Dans le cas (1), les groupes capables d'établir des interactions hydrogène sont disposés aux extrémités de la chaîne principale. Dans le cas (2), deux groupes capables d'établir des interactions hydrogène, sont disposés à chacune des extrémités de la chaîne principale.

Dans le cas (3), les groupes capables d'établir des interactions hydrogène sont disposés à l'intérieur de la chaîne principale dans des motifs répétitifs.

Dans les cas (4) et (5), il s'agit de copolymères dans lesquels les groupes capables d'établir des interactions hydrogène sont disposés sur des ramifications de la chaîne principale d'une première série de motifs qui sont copolymérisés avec des motifs ne comportant pas de groupes capables d'établir des interactions hydrogène.

Les polymères et copolymères utilisés dans la composition de l'invention ont avantageusement une température de transition solide-liquide de 45˚C à 190˚C. De préférence, ils présentent une température de transition solide-liquide allant de 70 à 130˚C et mieux de 80˚C à 105˚C.

<u>*Organogélateurs*</u>

Le structurant huileux peut également être choisi parmi les gélifiants organiques moléculaires non polymériques, également appelés organogélateurs, qui sont des composés dont les molécules sont capables d'établir entre elles des interactions physiques conduisant à une auto-agrégation des molécules avec formation d'un réseau supra-moléculaire 3D qui est responsable de la gélification de la (des) huile(s) (appelée également phase grasse liquide).

Le réseau supra-moléculaire peut résulter de la formation d'un réseau de fibrilles (dues aux empilements ou agrégations de molécules d'organogélateur), immobilisant les molécules de la phase grasse liquide.

L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.

Les interactions physiques sont diverses mais excluent la co-cristallisation. Ces interactions physiques sont en particulier des interactions du type interactions hydrogènes auto-complémentaires, interactions $\pi$ entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs selon l'invention comportent au moins un groupement capable d'établir des liaisons hydrogènes et mieux au moins deux groupements capables d'établir des liaisons hydrogène, au moins un cycle aromatique et mieux aux moins deux cycles aromatiques, au moins une ou plusieurs

liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs selon l'invention sont solubles dans la phase grasse liquide après chauffage jusqu'à obtention d'une phase liquide homogène transparente. Ils peuvent être solides ou liquides à température ambiante et pression atmosphérique.

Le ou les organogélateurs moléculaires utilisables dans la composition selon l'invention sont notamment ceux décrits dans le document "Specialist Surfactants", édité par D. Robb de 1997, p.209-263, chapitre 8 de P. Terech, les demandes européennes EP-A-1068854 et EP-A-1086945 ou encore dans la demande WO-A-02/47031.

On peut notamment citer parmi ces organogélateurs, les amides d'acides carboxyliques en particulier les acides tri-carboxyliques comme les cyclohexanetricarboxamides (voir la demande de brevet européen EP-A-1068854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1086945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO-93/23008 et notamment les amides de l'acide N-acylglutamique où le groupe acyle représente une chaîne alkyle en $C_8$ à $C_{22}$ tels que le dibutylamide de l'acide N-Lauroyl-L-glutamique, fabriqué ou commercialisé par la société Ajinomoto sous la dénomination GP-1 et leurs mélanges.

On peut également utiliser comme organogélateurs des composés de type bis-urée de formule générale suivante :

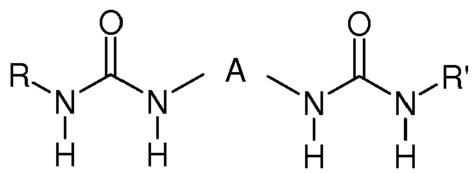

(I)

dans laquelle :

- A est un groupement de formule (II) :

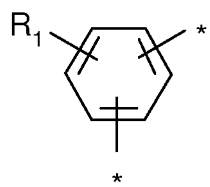

avec R1 étant un radical alkyle $C_1$ à $C_4$ linéaire ou ramifié, et les * symbolisant les points de rattachement du groupement A à chacun des deux atomes d'azote du reste du composé de formule générale (I), et

- R et R', identiques ou différents, sont choisis parmi :

  - i) les radicaux de formule (III) :

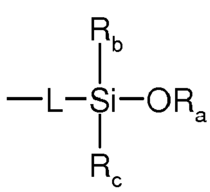

(III)

dans laquelle :

- L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S;
- Ra est :

  a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
  b) un radical siliconé de formule :

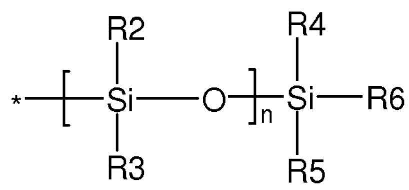

  avec n étant compris entre 0 et 100, notamment entre 1 et 80, voire 2 à 20;
  et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;
- Rb et Rc sont, indépendamment l'un de l'autre, choisis parmi:

  a) les radicaux carbonés, notamment hydrocarbonés (alkyles), linéaires, ramifiés et/ou cycliques, saturés ou insaturés, comprenant 1 à 18 atomes de carbone, et pouvant comprendre 1 à 4 hétéroatomes choisis parmi N, O, Si et S;
  b) les radicaux de formule :

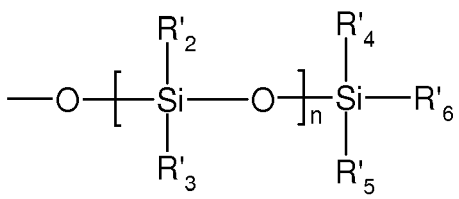

avec n étant compris entre 0 et 100, notamment entre 1 et 80, voire 2 à 20;
et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
et

- ii) les radicaux alkyle en $C_1$ à $C_{30}$, linéaire, ramifié et/ou cyclique, saturé ou insaturé, et comprenant éventuellement 1 à 3 hétéroatomes choisis parmi O, S, F et N; étant entendu que l'un au moins des radicaux R et/ou R' est de formule (III).

Notamment le groupement A peut être de formule :

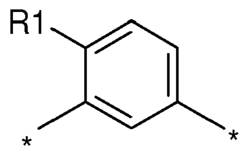 ou 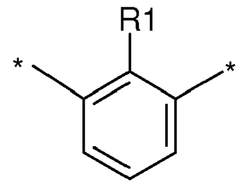

avec R1 et les * étant tels que définis précédemment.

En particulier, R1 peut être un groupement méthyle, ce qui conduit à un groupement A de formule :

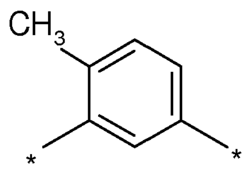 ou 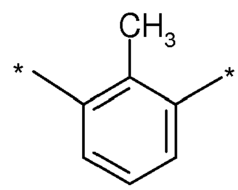

dans laquelle les * sont tels que définis précédemment.

En particulier, les composés selon l'invention peuvent être sous forme de mélange lié au fait que A peut être un mélange de 2,4-tolylène et 2,6-tolylène, notamment dans des proportions (isomère 2,4)/(isomère 2,6) variant de 95/5 à 80/20.

Selon l'invention, l'un au moins des radicaux R et/ou R' doit être de formule (III):

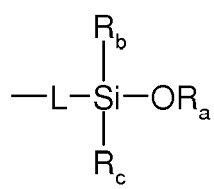 (III)

Dans cette formule, L est de préférence un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S. Dans le radical L, la chaîne carbonée peut être interrompue par le/les hétéroatomes et/ou peut comprendre un substituant comprenant ledit/lesdits hétéroatomes.

En particulier, L peut être de structure $-(CH_2)n-$ avec n= 1 à 18, notamment 2 à 12, voire 3 à 8. De préférence, L est choisi parmi les radicaux méthylène, éthylène, propylène, butylène et notamment n-butylène ou octylène.

Le radical L peut également être ramifié, par exemple du type $-CH_2-CH(CH_3)-$, ce qui conduit au radical de formule (III) suivant :

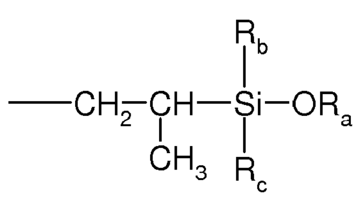

Le radical Ra peut être un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S. La chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou -OSi)-.

Ainsi, le radical Ra peut être de structure $-(CH_2)n'-CH_3$ avec n'= 0 à 17, notamment 1 à 12, voire de 1 à 6. En

particulier, Ra peut être méthyle, éthyle, propyle ou butyle.
Il peut également être de structure $-(CH_2)x-O-(CH_2)z-CH_3$ ou bien $-(CH_2)x-O-(CH_2)y-O-(CH_2)z-CH_3$, avec x = 1 à 10, de préférence 2; y=1 à 10 de préférence 2, et z= 0 à 10, de préférence 0 ou 1.
Le radical Ra peut encore être de structure $-SiR_4R_5R_6$ (cas où n=0), dans laquelle R4, R5 et R6 sont, indépendamment les uns des autres, de préférence, des radicaux alkyle, ayant 1 à 12 atomes de carbone, notamment 1 à 6 atomes de carbone; en particulier R4, R5 et/ou R6 peuvent être choisis parmi méthyle, éthyle, propyle, butyle.
Le radical Ra peut également être un radical siliconé de formule :

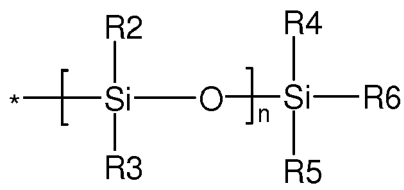

dans laquelle R2 à R6 sont, indépendamment les uns des autres, de préférence, des radicaux alkyle, ayant 1 à 12 atomes de carbone, notamment 1 à 6 atomes de carbone; en particulier R2 à R6 peuvent être choisis parmi méthyle, éthyle, propyle, butyle;
et en particulier un radical :

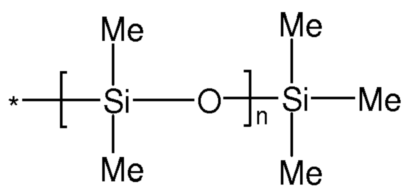

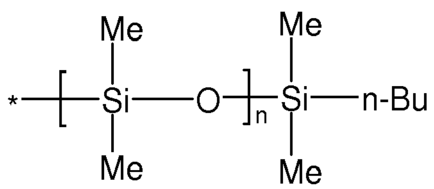

avec n=1 à 100; et encore plus particulièrement un radical :

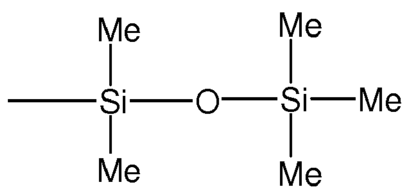

Les radicaux Rb et Rc, identiques ou différents, peuvent être des radicaux carbonés, notamment hydrocarbonés (alkyles), linéaires, ramifiés et/ou cycliques, saturés ou insaturés, comprenant 1 à 18 atomes de carbone, et pouvant comprendre 1 à 8 hétéroatomes choisis parmi N, O, Si et S. Dans ces radicaux, la chaîne carbonée peut être interrompue par le/les hétéroatomes et/ou peut comprendre un substituant comprenant ledit/lesdits hétéroatomes; les hétéroatomes peuvent notamment former un ou plusieurs groupements -SiO- (ou -OSi)-.
Ainsi, ils peuvent être de structure $-(CH_2)m-CH_3$ avec m= 0 à 17, notamment 1 à 12, voire 2 à 5; en particulier, Rb et/ou Rc peuvent être méthyl, éthyle, propyle ou butyle;
Ils peuvent également être de structure $-O-(CH_2)m'-CH_3$ avec m'= 0 à 5, notamment 1 à 4, et en particulier méthoxy ou éthoxy. Ils peuvent aussi être de structure $-O-(CH_2)x-O-(CH_2)z-CH_3$ ou $-O-(CH_2)x-O-(CH_2)y-O-(CH_2)z-CH_3$, avec x = 1 à 10, de préférence 2; y=1 à 10 de préférence 2, et z= 0 à 10, de préférence 0 ou 1.
Ils peuvent encore être de structure :

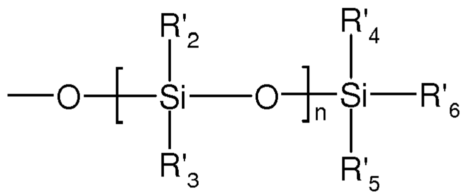

avec n étant compris entre 0 et 100, notamment entre 1 et 80, voire 2 à 20;
et R'2 à R'6 étant, indépendamment les uns des autres, de préférence, des radicaux alkyle, ayant 1 à 12 atomes de carbone, notamment 1 à 6 atomes de carbone; en particulier R'2 à R'6 peuvent être choisis parmi méthyle, éthyle, propyle, butyle.

Lorsqu'ils sont de formule (III), les radicaux R et/ou R' sont de préférence choisis parmi les radicaux suivants :

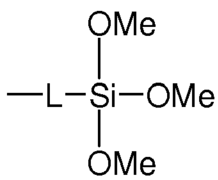 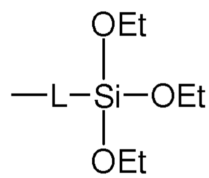

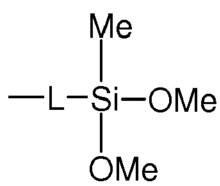 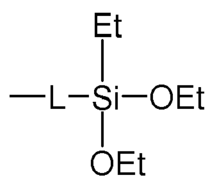 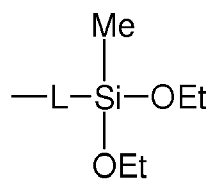

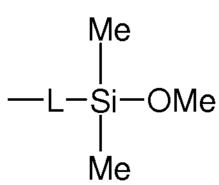 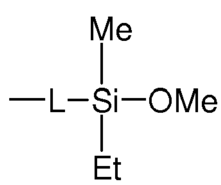 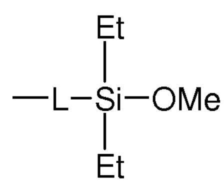

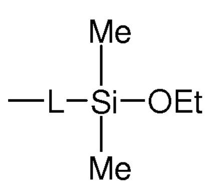 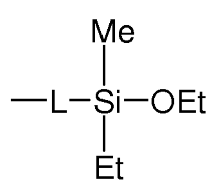 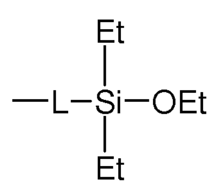

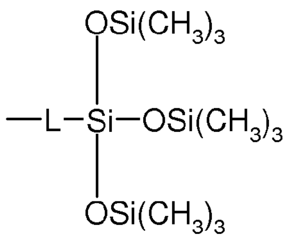

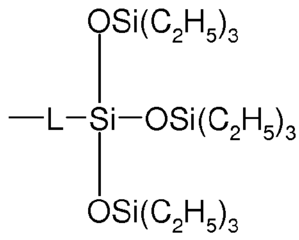

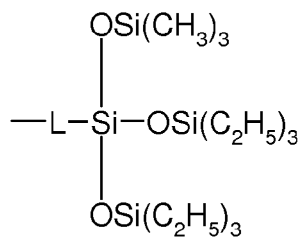

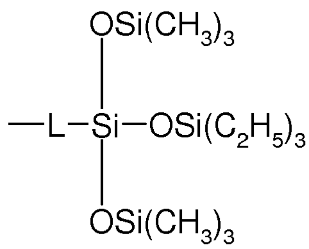

et également ceux de formule :

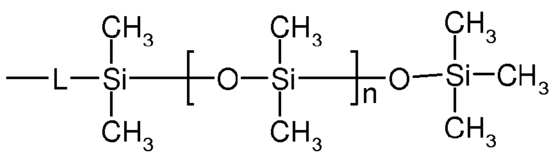

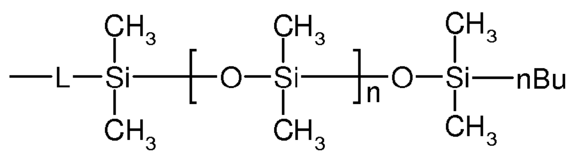

avec n variant de 0 à 100 et en particulier

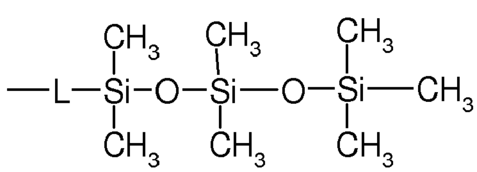

et

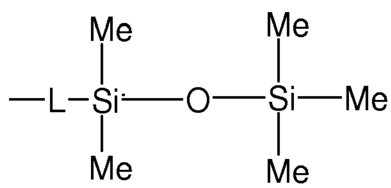

et

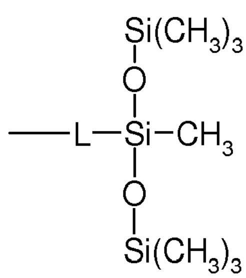

ou encore

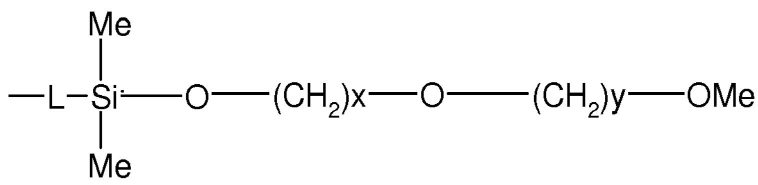

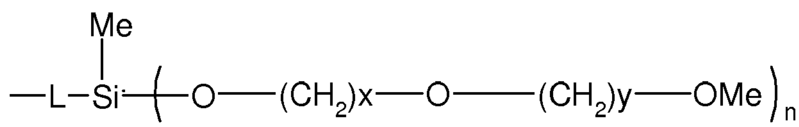

avec n=2 $-L-S_i\{O-(CH_2)x-O-(CH_2)y-OMe)_m$ avec m=3
dans lesquelles x = 1 à 10, de préférence 2; et y = 1 à 10, de préférence 2; et L étant tel que défini ci-dessus.
De préférence, dans ces formules, L est un radical alkylène en C1-C8, linéaire ou ramifié, notamment méthylène, éthylène, propylène, butylène et notamment n-butylène, octylène ou de formule $-CH_2-CH(CH_3)-$.
Dans un mode de réalisation particulier, R et R', identiques ou différents, sont tous les deux de formule (III).

Dans un autre mode de réalisation, l'un des radicaux R ou R' représente un radical alkyle en $C_1$ à $C_{30}$, linéaire, ramifié et/ou cyclique, saturé ou insaturé, et comprenant éventuellement 1 à 3 hétéroatomes choisis parmi O, S, F et N. Ceci s'avère particulièrement avantageux pour conférer un caractère universel aux composés de formule (I), c'est-à-dire leur permettre de texturer à la fois des milieux carbonés polaires ou apolaires, des milieux siliconés linéaires ou cycliques, des huiles mixtes c'est-à-dire carbonées partiellement siliconées, ainsi que leurs mélanges.
La chaîne carbonée peut être interrompue par le/les hétéroatomes et/ou peut comprendre un substituant comprenant ledit/lesdits hétéroatomes, notamment sous forme de groupement carbonyle (-CO-), d'un ou plusieurs radicaux hydroxy (-OH), et/ou d'un radical ester - COOR" avec R" = radical alkyle, linéaire ou ramifié, ayant 1 à 8 atomes de carbone.

Ainsi, ledit radical R ou R' peut être un groupement choisi parmi :

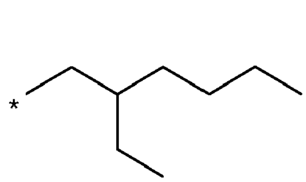

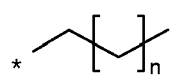

n = 16

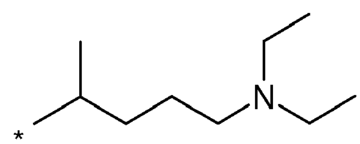

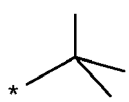

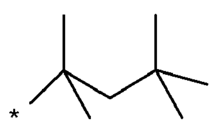

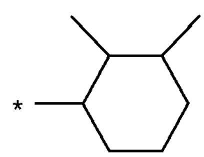

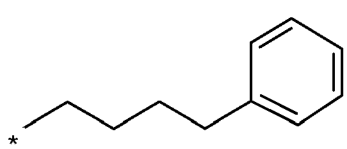

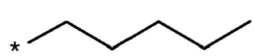

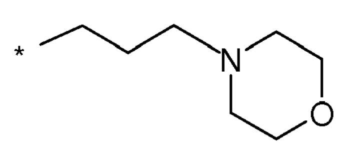

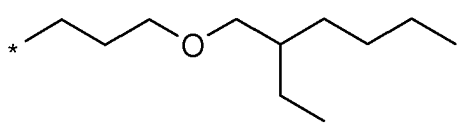
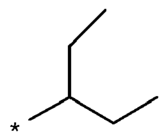
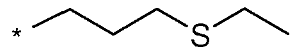

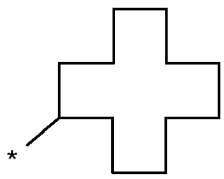
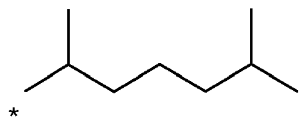
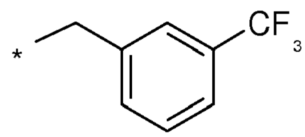

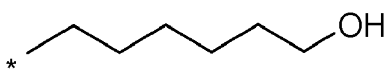
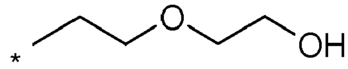

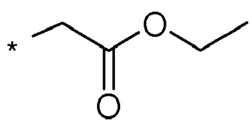
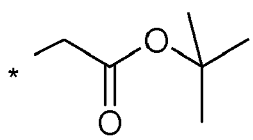
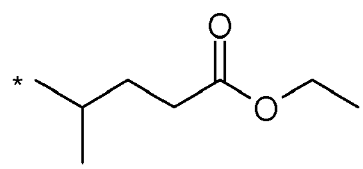

avec * ayant la définition donnée ci-dessus.

Dans un mode de réalisation préféré, R ou R’ représente un radical alkyle ramifié, notamment monoramifié, de préférence non cyclique, saturé ou insaturé, comprenant 3 à 16 atomes de carbone, notamment 4 à 12, voire 4 à 8 atomes de carbone, et comprenant éventuellement 1 à 3 hétéroatomes choisis parmi O, S, F et/ou N, de préférence O et/ou N. En particulier, R ou R’ peuvent être des radicaux tertio-butyle ou 2-éthylhexyle ou de formule :

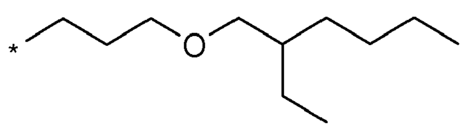

Lorsque le composé de formule (I) comprend un radical R qui est un radical alkyle, et donc un radical R’ qui est de formule (III), le rapport entre $n_R$ et $n_{R'}$ est de préférence compris entre 5/95 et 95/5, par exemple entre 10/90 et 90/10, en particulier entre 40/60 et 85/15, notamment entre 50/50 et 80/20, voire entre 60/40 et 75/25;
avec $n_R$ étant le nombre de moles d’amine $NH_2$-R et $n_{R'}$ étant le nombre de moles d’amine $NH_2$-R’ utilisées pour préparer le composé de formule (I).

Les composés selon l’invention peuvent se présenter sous forme de sels et/ou d’isomères de composés de formule (I).

D’une manière générale, les composés de formule générale (I) selon l’invention peuvent être préparés comme décrit dans la demande FR2910809.

Les composés de type bis-urée siliconés décrits précédemment peuvent être en mélange avec d’autres composés bis-urées non siliconés. Les composés de bis-urée non siliconés peuvent, selon un premier aspect, répondre à la formule générale (II) suivante :

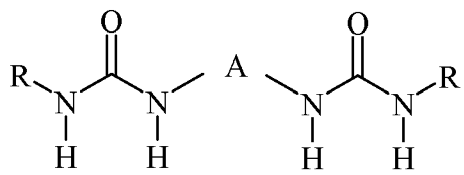

(II)

dans laquelle :

- A est un groupement de formule :

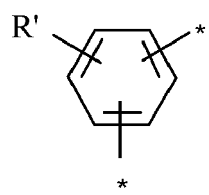

avec R' étant un radical alkyle $C_1$ à $C_4$ linéaire ou ramifié et les * symbolisant les points de rattachement du groupement A à chacun des deux atomes d'azote du reste du composé de formule générale (II), et

- R est un radical alkyle en $C_6$ à $C_{15}$, mono-ramifié, non cyclique, saturé ou insaturé et dont la chaîne hydrocarbonée est éventuellement interrompue par 1 à 3 hétéroatomes choisis parmi O, S et N, ou

l'un de ses sels ou isomères.

Selon un mode de réalisation préféré de l'invention, le groupement figuré par A est un groupement de formule :

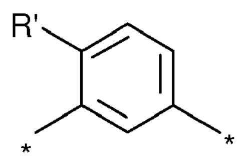

ou

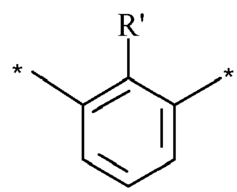

avec R' et les * étant tels que définis précédemment.

En particulier, R' peut être un groupement méthyle, et le groupement A est alors plus particulièrement un groupement de formule :

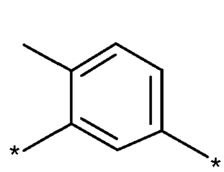

ou

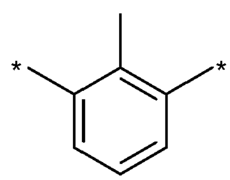

avec les * étant tel que défini précédemment.

Selon un premier mode de réalisation de l'invention, R peut être choisi parmi les radicaux mono-ramifiés de formule générale $C_nH_{2n+1}$, n étant un entier variant de 6 à 15, en particulier de 7 à 9 voire égal à 8.

Ainsi, les deux groupements R du composé de formule (II) peuvent figurer respectivement un groupement :

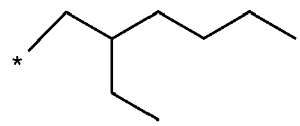

avec * symbolisant le point de rattachement de chacun des groupements R à chacun des atomes d'azote du reste du composé de formule générale (II).

Selon un deuxième mode de réalisation de l'invention, R peut être choisi parmi les radicaux mono-ramifiés de formule générale $C_{m-p}H_{2m+1-2p}X_p$, p étant égal à 1, 2 ou 3, de préférence égal à 1, m étant un entier variant de 6 à 15, de préférence de 10 à 14, en particulier de 10 à 12, voire égal à 11 et X représentant les atomes de soufre et/ou d'oxygène, en particulier les atomes d'oxygène.

Plus particulièrement, R peut être un radical de formule $C_{m'}H_{2m'}X\text{-}(C_{p'}H_{2p'}X')_r\text{-}C_xH_{2x+1}$,

dans laquelle X et X' sont indépendamment l'un de l'autre un atome d'oxygène ou de soufre, de préférence d'oxygène, r vaut 0 ou 1, m', p' et x sont des entiers tels que leur somme varie de 6 à 15, en particulier de 10 à 12, voire est égale à 11 et étant entendu qu'au moins une des chaînes carbonées $C_{m'}H_{2m'}$, $C_{p'}H_{2p'}$, ou $C_xH_{2x+1}$ est ramifiée. De préférence c'est la chaîne $C_xH_{2x+1}$ qui est ramifiée, de préférence r égal 0, de préférence m' est un entier variant de 1 à 10, notamment de 2 à 6, en particulier égal à 3, et/ou de préférence x est un entier variant de 4 à 16, notamment de 6 à 12, en particulier égal à 8.

Ainsi, les deux groupements R du composé de formule (I) peuvent figurer respectivement un groupement :

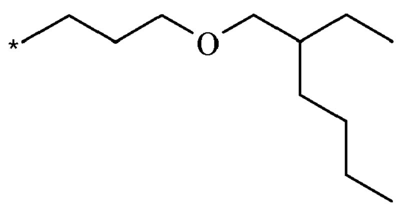

avec * symbolisant le point de rattachement de chacun des groupements R à chacun des atomes d'azote du reste du composé de formule générale (I).

De tels composés peuvent être présents dans les compositions selon l'invention en mélanges avec des isomères, notamment des isomères de position sur le groupement A, notamment dans des proportions 95/5 ou 80/20.

Comme il ressort des exemples ci-après, la présence de l'un ou l'autre de ses radicaux dans la molécule de formule générale (II) s'avère particulièrement avantageuse pour conférer un caractère universel, au sens de l'invention, aux dérivés bis-urée non siliconés correspondants.

A titre représentatif et non limitatif des composés convenant tout particulièrement à l'invention, on peut plus particulièrement citer les composés suivants, utilisés purs ou en mélange :

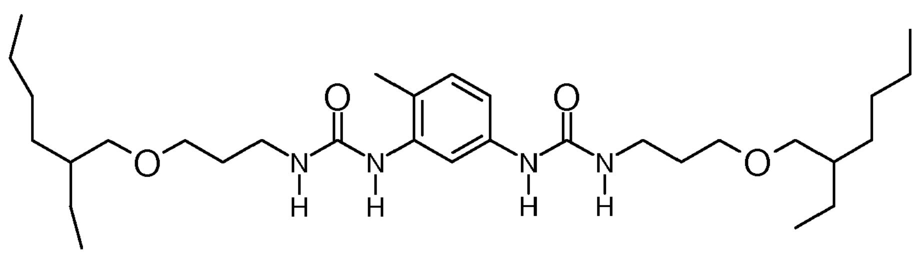

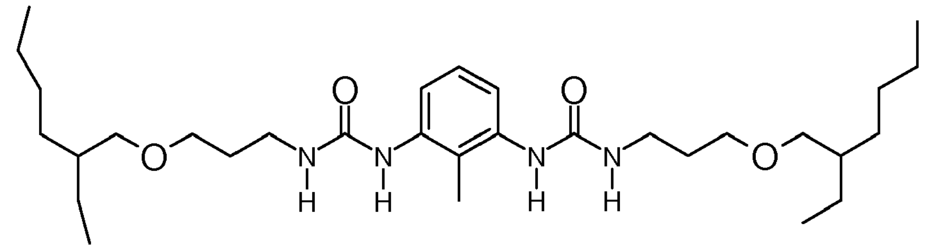

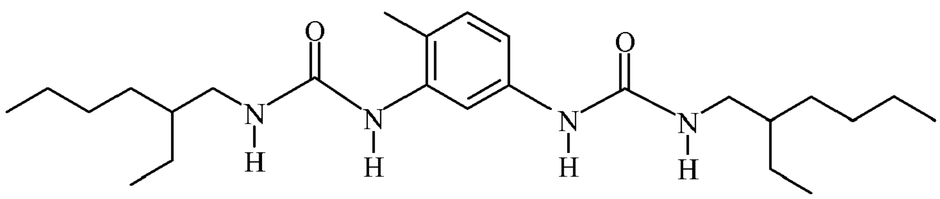

.

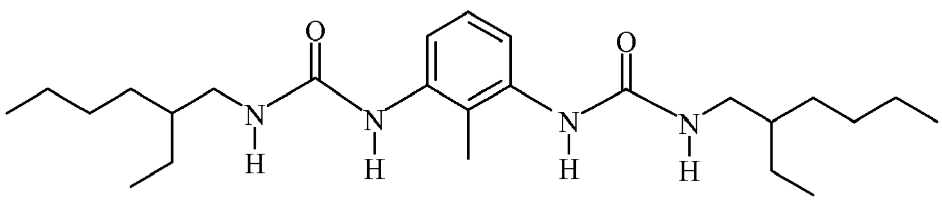

.

et leurs sels.

Selon un autre aspect de l'invention, les dérivés bis-urée non siliconés formule (III) suivante :

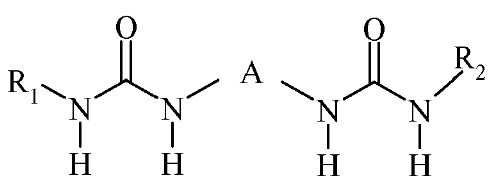

(III)

dans laquelle :

- A est un groupement de formule :

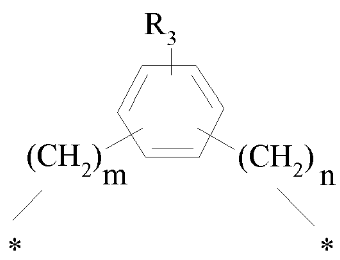

avec

- $R_3$ étant un atome d'hydrogène ou un radical alkyle en $C_1$ à $C_4$ linéaire ou ramifié,
- n et m étant, indépendamment l'un de l'autre, égaux à 0 ou 1, et
- * symbolisant le point de rattachement du groupement A aux deux atomes d'azote du reste du composé de formule générale (III),
- $R_1$ est un radical carboné en $C_3$ à $C_{15}$, ramifié, non cyclique, saturé ou insaturé et contenant éventuellement de 1 à 3 hétéroatomes choisis parmi O, S, F et N et/ou un carbonyle, et leurs combinaisons,
- $R_2$ est différent de $R_1$ et est choisi parmi les radicaux alkyles en $C_1$-$C_{24}$, saturé ou insaturé, linéaire, ramifié ou cyclique, contenant éventuellement de 1 à 3 hétéroatomes choisis parmi O, S, F et N, et éventuellement substitué par :

- 1, 2 ou 3 radicaux hydroxy,
- un radical ester ($-COOR_4$), avec $R_4$ étant un radical alkyle, linéaire

ou ramifié, ayant de 1 à 8, notamment 1 à 6, voire 2 à 4 atomes de carbone;

- un radical cyclique saturé, insaturé ou aromatique ayant de 5 à 12 atomes de carbone, en particulier un radical phényle éventuellement substitué par un ou plusieurs radicaux identiques ou différents choisis parmi les radicaux alkyle en $C_1$-$C_4$, trifluorométhyle, ou un dérivé morpholine, et/ou
- un ou plusieurs radicaux alkyles linéaires ou ramifiés en $C_1$-$C_4$,

ou l'un de ses sels ou isomères.

En particulier n et m sont égaux, et plus particulièrement égaux à zéro et $R_3$ est un radical $R'_3$, tel que défini ci-après. Ainsi, de manière préférée, A représente un groupement

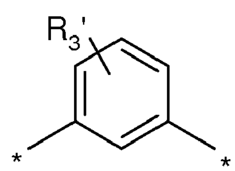

Avec $R_3$' étant un radical alkyle en $C_1$ à $C_4$ linéaire ou ramifié et * symbolisant les point de rattachement du groupement A aux deux atomes d'azote du reste du composé de formule générale (III).

Selon une variante de l'invention, le composé de formule générale (III) comprend à titre de A, au moins un groupement choisi parmi :

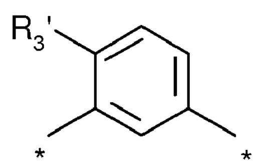

ou

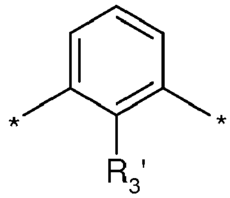

avec $R_3$' et * étant tels que défini précédemment.

En particulier, $R_3$' peut être un groupement méthyle, et dans ce cas le groupement A représente un groupement

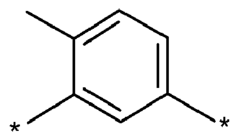

ou

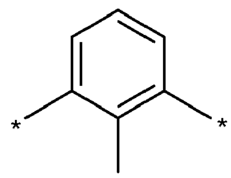

* étant tel que défini précédemment.

En particulier, les composés sont tels que A est un mélange de 2,4-tolylène et 2,6-tolylène notamment dans des proportions (isomère 2,4)/(isomère 2,6) variant de 95/5 à 80/20.

Selon un mode de réalisation de l'invention, le composé de formule générale (III) comprend à titre de $R_1$, un radical en $C_6$-$C_{15}$ ramifié.

Selon un mode de réalisation de l'invention, le composé de formule générale (III) comprend à titre de $R_1$, un groupement choisi parmi :

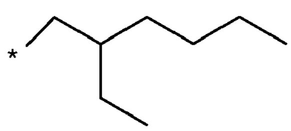

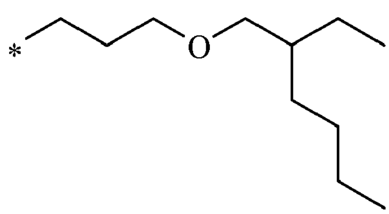

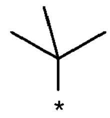

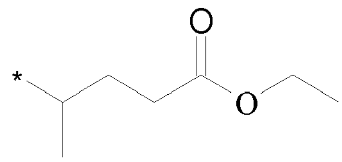

avec * symbolisant le point de rattachement du groupement $R_1$ à l'azote du reste du composé de formule générale (III). Comme il ressort des exemples ci-après, la présence de l'un et/ou l'autre de ses deux radicaux dans la molécule de formule générale (III) s'avère particulièrement avantageuse pour conférer un caractère universel au sens de l'invention, aux dérivés bis-urée asymétriques correspondants.
En ce qui concerne $R_2$ qui est différent de $R_1$, il peut être avantageusement choisi parmi les groupements suivants :

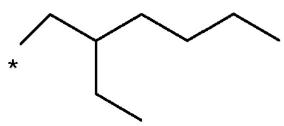

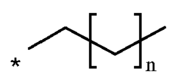

n = 16

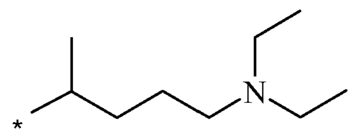

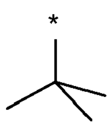

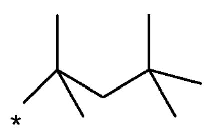

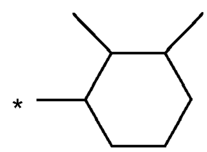

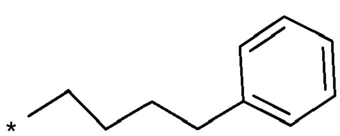

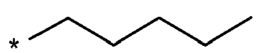

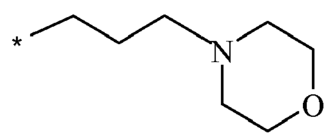

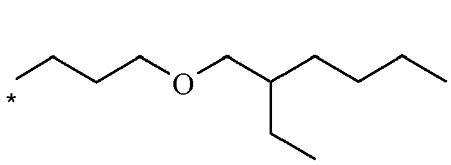

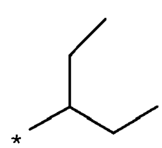

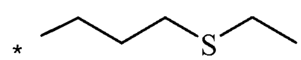

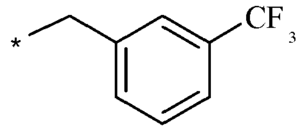

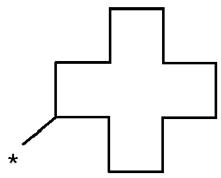

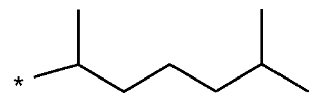

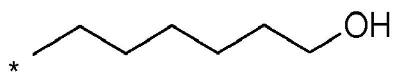

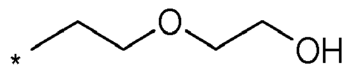

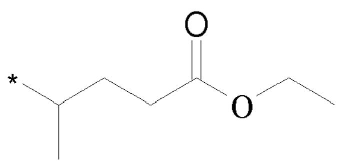

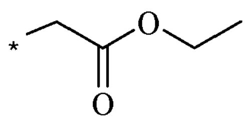

et

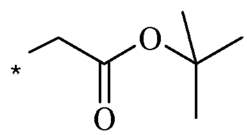

avec * symbolisant le point de rattachement du groupement $R_2$ à l'azote du reste du composé de formule générale (III). D'une manière générale, les composés décrits peuvent être préparés comme décrit dans la demande FR2910809.

*Polymères blocs*

On peut aussi utiliser comme agent rhéologique de phase grasse des polymères blocs greffés ou séquencés.
On peut notamment utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.
On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "Dow Corning 3225C" par la société Dow Corning, les lauryl méthicones tels que ceux vendu sous la dénomination "Dow Corning Q2-5200 par la société "Dow Corning".
Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton

G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile) ; OS129880, OS129881 et OS84383 de chez Lubrizol (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

*Agents cristaux liquides cholestériques*

On entend par agents cristaux liquides des composés générant un état mésomorphe, c'est-à-dire un état pour lequel la fusion des cristaux permet d'obtenir des liquides possédant des propriétés optiques comparables à celles de certains cristaux. Ces composés sont plus précisément définis dans le chapitre Liquid crystals dans l'encyclopédie Ullmann.

Ces agents cristaux liquides sont en particulier décrits dans les brevets ou demandes de brevet EP 545 409, WO 94109086, EP 709 445, GB 2 282 145, GB 2 276 883, WO 95132247, WO 95132248, EP 686 674, EP 711 780.

Ces agents cristaux liquides peuvent réagir en réponse aux vibrations par un changement de viscosité et/ou par un changement de couleur.

Plus particulièrement, les composés générant un état mésomorphe sont des composés à fonction cholestérique, dont la structure est la suivante :

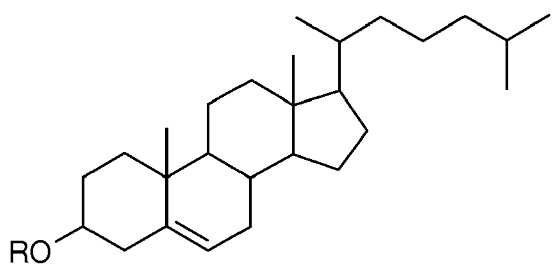

R est un groupement alkyl, alkylcarbonyl comprenant de 1 à 30 atomes de carbone substitué ou non par des groupements cycliques, aromatiques, halogènes, ramifié ou non.

A titre non limitatif, on peut citer comme agent cristaux liquides répondant à cette définition : le cholesterol erucyl carbonate, le choleterol methyl carbonate, le cholesterol oleyl carbonate, le cholesterol para-nonyl phneyl carbonate, le cholesterol phenyl carbonate, la cholesterol acetate, le cholesterol benzoate, le cholesterol butyrate, le cholesterol isobutyrate, le cholesterol chloride, le cholesterol chloroacetate, le cholesterol cinnamate, le cholesterol crotanoate, le cholesterol decanoate, le cholesterol erucate, le cholesterol heptanoate, le cholesterol hexanoate, le cholesterol myristate, le cholesterol nonaoate, le cholesterol octanoate, le cholesterol oleate, le cholesterolpropionate, le cholesterol valerate, le dicholesteryl carbonate.

La composition selon l'invention peut comporter une phase continue aqueuse ou une phase continue huileuse.

Par composition à phase continue aqueuse, on entend que la composition présente une conductivité, mesurée à 25 °C, supérieure ou égale à 23 μS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

Par composition à phase continue huileuse, on entend que la composition présente une conductivité, mesurée à 25 °C, inférieure à 23 μS/cm (microSiemens/cm), la conductivité étant mesurée par exemple à l'aide d'un conductimètre

MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

Les compositions peuvent contenir de 1 à 60% d'agent rhéologique de phase grasse. De préférence, la composition contient de 2% à 50% en poids, mieux de 5% à 40% d'agent rhéologique de phase grasse.

Dans le cas où la composition comprend un agent rhéologique de phase grasse, elle comporte une phase continue huileuse.

## PHASE AQUEUSE

La composition conforme à l'invention peut comprendre une phase aqueuse comprenant de l'eau et/ou au moins un solvant hydrosoluble.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$ et $C_4$ et les aldéhydes en $C_2$-$C_4$.

La phase aqueuse (eau et éventuellement le solvant miscible à l'eau) peut être présente dans la composition en une teneur allant de 1 % à 95 % en poids, par rapport au poids total de la composition, de préférence allant de 5 % à 80 % en poids, et préférentiellement allant de 10 % à 60 % en poids.

La phase aqueuse selon l'invention peut également comprendre au moins un polymère filmogène hydrophile et/ou au moins un épaississant hydrophile et/ou au moins un tensioactif, tel que ceux listés précédemment.

### *Polymère filmogène hydrophile*

Dans la présente invention, on entend par "polymère filmogène", un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

De façon générale, la teneur en matières sèches en polymère filmogène dans la composition peut varier de 0,1 à 40 %, de préférence de 0,5 à 30 % et mieux de 1 à 10 % en poids, par rapport au poids total de la composition, Le polymère filmogène hydrophile peut être un polymère hydrosoluble ou se présenter en dispersion dans un milieu aqueux.

Parmi les polymères filmogènes utilisables dans la composition de la présente invention, on peut citer les polymères synthétiques, de type radicalaire ou de type polycondensat, les polymères d'origine naturelle, et leurs mélanges.

Comme exemples de polymères filmogènes hydrosolubles, on peut citer :

- les protéines comme les protéines d'origine végétale telles que les protéines de blé, de soja; les protéines d'origine animale tels que les kératines, par exemples les hydrolysats de kératine et les kératines sulfoniques;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle; les copolymères de vinylpyrrolidone et de caprolactame ; l'alcool polyvinylique;
- les polymères de chitine ou de chitosane anioniques, cationiques, amphotères ou non-ioniques,
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque;
- les muccopolysaccharides tels les chondroïtines sulfate,
- et leurs mélanges.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer Allianz Opt® par la société Rohm and Haas ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Avalure UR-445® et Sancure 2060® par la société NOVEON, Impranil 85® par la société BAYER, Aquamere H-1511® par la société HYDROMER; les sulfopolyesters vendus sous le nom de marque Eastman AQ® par la société EASTMAN CHEMICAL PRODUCTS, les dispersions vinyliques comme le Mexomère PAM®, les dispersions aqueuses de polyvinyl acétate comme le "Vinybran®" de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur®" par la société AIR PRODUCTS ou "Duromer®" de NATIONAL STARCH, les dispersions type core/shell : par exemple celles commercialisées par la société ATOFINA sous la référence Kynar (core : fluoré-shell : acrylique) ou encore ceux décrits dans le document US 5 188 899 (core ; silice - shell : silicone) et leurs mélanges.

Selon un mode préféré, on utilisera les polymères ou copolymères acryliques, tels que les polyacrylates ou les polyméthacrylates, en particulier en des teneurs allant de 1 à 40%, en particulier de 5 à 30%, de préférence de 8 à 15%, et mieux de 8 à 12% en poids par rapport au poids total de ladite composition.

Selon un mode particulier de réalisation, la composition conforme à l'invention comprend à titre de polymères filmogènes hydrophiles au moins l'association d'un polymère cationique et d'un polymère anionique.

Le polymère cationique peut être choisi parmi les dérivés d'éther de cellulose quaternaires, les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylaminoalkyle quaternisés ou non, les polymères quaternaires de vinylpyrrolidone et de vinylimidazole, les polyamidoamines et leurs mélanges.

De préférence, le polymère cationique est une hydroxyalkyl($C_1$-$C_4$)cellulose comportant des groupements ammonium quaternaires.

Le polymère anionique est avantageusement choisi parmi :

A) les homo- ou copolymères d'acide acrylique ou méthacrylique ou leurs sels, les copolymères d'acide acrylique et d'acrylamide et leurs sels, les sels de sodium d'acides polyhydroxycarboxyliques tels que les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL,

B) les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol; les copolymères de ce type comportant dans leur chaîne un motif acrylamide éventuellement N-alkylé et/ou hydroxyalkylé, les copolymères d'acide acrylique et de méthacrylate d'alkyle en C1-C4 et les terpolymères de vinylpyrrolidone, d'acide acrylique et de méthacrylate d'alkyle en C1-C20;

C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ;

D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters; les copolymères d'anhydrides maléique, citraconique, itaconique et d'un ester allylique ou méthallylique comportant éventuellement un groupement acrylamide, méthacrylamide, une $\alpha$-oléfine, des esters acryliques ou méthacryliques, des acides acrylique ou méthacrylique ou la vinylpyrrolidone dans leur chaîne, les fonctions anhydrides sont monoestérifiées ou monoamidifiées ;

E) les polyacrylamides comportant des groupements carboxylates,

F) l'acide désoxyribonucléique ;

G) les copolymères d'au moins un diacide carboxylique, d'au moins un diol et d'au moins un monomère aromatique bifonctionnel portant un groupement -SO3M avec M représentant un atome d'hydrogène, un ion ammonium NH4+

ou un ion métallique ;

- et leurs mélanges.

Les polymères anioniques les plus particulièrement préférés sont choisis parmi les polymères anioniques non réticulés comme les copolymères méthylvinyléther /anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP et les homopolymères d'acide acrylique ou méthacrylique commercialisés par exemple sous la dénomination VERSICOL E 5 OU le polyméthacrylate de sodium vendu sous la dénomination DARVAN 7 par la société VANDERBILT, et leurs mélanges.

De préférence, le polymère anionique est un polyméthacrylate de sodium.

La composition conforme à l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

*Epaississant hydrophile*

La composition selon l'invention peut comprendre au moins un épaississant hydrophile.

Ces épaississants peuvent être utilisés seuls ou en association. Ces épaississants peuvent être notamment choisis parmi les gommes et les polymères cellulosiques.

Par épaississant hydrophile, on entend un agent épaississant soluble ou dispersible dans l'eau.

Comme épaississants hydrophiles, on peut citer en particulier les polymères épaississants hydrosolubles ou hydrodispersibles. Ceux-ci peuvent notamment être choisis parmi :

- les polymères carboxyvinyliques modifiés ou non, tels que les produits commercialisés sous les dénominations Carbopol (nom CTFA : carbomer) par la société Goodrich ;
- les homo- ou copolymères d'acides acrylique ou méthacrylique ou leurs sels et leurs esters et en particulier les produits vendus sous les dénominations VERSICOL F® ou VERSICOL K® par la société ALLIED COLLOID, ULTRAHOLD 8® par la société CIBA-GEIGY, les polyacrylates et polyméthacrylates tels que les produits vendus sous les dénominations de Lubrajel et Norgel par la société GUARDIAN ou sous la dénomination Hispagel par la société HISPANO CHIMICA, les acides polyacryliques de type SYNTHALEN K ;
- les polyacrylamides ;
- les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL ;
- les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ;
- les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer /Isohexadecane / Polysorbate 80) par la société SEPPIC ;
- les copolymères acide polyacryliques/acrylates d'alkyle de type PEMULEN ;
- les biopolymères polysaccharidiques comme la gomme de xanthane, la gomme de guar, la gomme arabique, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellulose, l'hydroéthylcellulose et l'hydroxypropylcellulose ;
- les silices pyrogénées hydrophiles obtenues par hydrolyse à haute température d'un composé volatil du silicium dans une flamme oxhydrique, produisant une silice finement divisée. Les silices hydrophiles présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", ROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot. Elles présentent de préférence une taille de particules pouvant être nanométrique à micrométrique, par exemple allant d'environ de 5 à 200 nm;

- les polymères associatifs comme le PEG-150/STEARYL ALCOHOL/SMDI COPOLYIER vendu sous le nom Aculyn 46 par Rohm & Haas, ou le STEARETH-100/PEG-136/HDI COPOLYMER vendu sous le nom Rhéolate FX 1100 par Elementis) ;
- et leurs mélanges.

L'épaississant hydrophile peut être choisi parmi les polymères associatifs. Par "polymère associatif" au sens de la présente invention, on entend tout polymère amphiphile comportant dans sa structure au moins une chaîne grasse et au moins une portion hydrophile. Les polymères associatifs conformes à la présente invention peuvent être anioniques, cationiques, non-ioniques ou amphotères.

Parmi les polymères anioniques associatifs, on peut citer ceux comportant au moins un motif hydrophile, et au moins un motif éther d'allyle à chaîne grasse, plus particulièrement parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyle à chaîne grasse correspond au monomère de formule (I) suivante :

$$CH_2 = C(R')CH_2\ O\ B_n\ R \qquad (I)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone.
Des polymères amphiphiles anioniques de ce type sont décrits et préparés, selon un procédé de polymérisation en émulsion, dans le brevet EP-0 216 479.
Comme polymères anioniques associatifs, on peut citer également les polymères anioniques comportant au moins un motif hydrophile de type acide carboxylique insaturé oléfinique, et au moins un motif hydrophobe exclusivement de type ester d'alkyl ($C_{10}$-$C_{30}$) d'acide carboxylique insaturé. On peut citer à titre d'exemple les polymères anioniques décrits et préparés, selon les brevets US-3 915 921 et 4 509 949.

Comme polymères associatifs cationiques, on peut citer les dérivés de cellulose quaternisée et les polyacrylates à groupements latéraux aminés.

Les polymères associatifs non-ioniques peuvent être choisis parmi:

- les celluloses modifiées par des groupements comportant au moins une chaîne grasse comme par exemple les hydroxyéthylcelluloses modifiées par des groupements comportant au moins une chaîne grasse tels que des groupes alkyle, notamment en $C_8$-$C_{22}$, arylalkyle, alkylaryle, telles que le NATROSOL PLUS GRADE 330 CS (alkyles en $C_{16}$) vendu par la société AQUALON,
- les celluloses modifiées par des groupes polyalkylène glycol éther d'alkyl phénol,
- les guars tels que l'hydroxypropyl guar, modifiés par des groupements comportant au moins une chaîne grasse telle qu'une chaîne alkyle,
- les copolymères de vinyl pyrrolidone et de monomères hydrophobes à chaîne grasse ;
- les copolymères de méthacrylates ou d'acrylates d'alkyles en $C_1$-$C_6$ et de monomères amphiphiles comportant au moins une chaîne grasse,
- les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle,
- les polyuréthanes associatifs
- leurs mélanges.

De préférence, le polymère associatif est choisi parmi les polyuréthanes associatifs. Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de $C_6$ à $C_{30}$ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile. Les polyuréthanes associatifs peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé

par exemple). Ces polymères peuvent être également en greffons ou en étoile. De préférence, les polyuréthanes associatifs sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.

A titre d'exemple, des polymères associatifs utilisables dans l'invention, on peut citer le polymère $C_{16}$-$OE_{120}$-$C_{16}$ de la société SERVO DELDEN (sous le nom SER AD FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204 ou encore le Rhéolate FX 1100 par Elementis. Ces polyuréthannes associatifs sont vendus sous forme pure. Le produit DW 1206B de chez RHOM & HAAS à chaîne alkyle en $C_{20}$ et à liaison uréthane, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le SER AD FX1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit Aculyn 46, DW 1206F et le DW 1206J, ainsi que l'Acrysol RM 184 ou l'Acrysol 44 de la société ROHM & HAAS, ou bien encore le Borchigel LW 44 de la société BORCHERS.

## PHASE HUILEUSE

La composition selon l'invention peut également comprendre une phase huileuse.

Cette phase huileuse peut comprendre au moins une huile telle que listée ci-avant, et/ou au moins une cire, et/ou au moins un polymère filmogène lipophile et/ou un agent rhéologique de phase grasse tel que mentionné ci-avant.

### *Cires*

Par cire au sens de la présente invention, on entend un composé lipophile, solide à température ambiante (25 ˚C), à changement d'état solide/liquide réversible, ayant un point de fusion supérieur ou égal à 30 ˚C pouvant aller jusqu'à 120 ˚C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 ˚C et mieux supérieure à 45 ˚C.

La cire est présente en une teneur au moins égale à 15% en poids. De préférence, elle est présente en une teneur allant de 15 à 40 % en poids par rapport au poids total de la composition, mieux de 16 à 35 % et encore mieux de 16 à 30% en poids.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination "HEST 2T-4S" par la société HETERENE, le tétrabéhénate de di-(triméthylol-1,1,1 propane) vendue sous la dénomination HEST 2T-4B par la société HETERENE.

On peut encore citer les cires de silicone comme les alkyl ou alkoxy-diméticone ayant de 16 à 45 atomes de carbone, les cires fluorées.

On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A- 2792190.

Selon un mode de réalisation particulier, les compositions conformes à l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination "TA-TX2i®" par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0.

Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C20-C40 (le groupe alkyle comprenant de 20 à 40 atomes de carbone), seul ou en mélange, en particulier un 12-(12'-hydroxystéaryloxy)stéarate d'alkyle en C20-C40, de formule (II) :

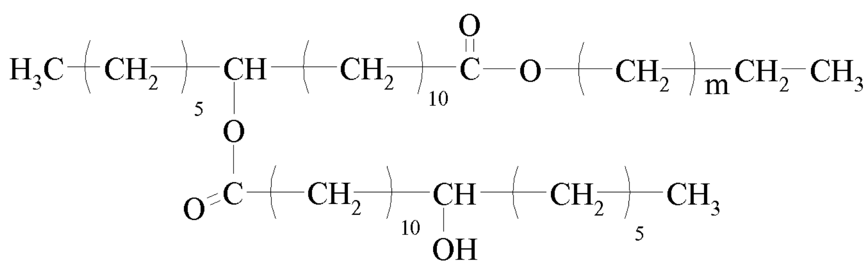

(II)

dans laquelle m est un entier allant de 18 à 38, ou un mélange de composés de formule (II).

Une telle cire est notamment vendue sous les dénominations "Kester Wax K 82 P®" et "Kester Wax K 80 P®" par la société KOSTER KEUNEN.

Les cires citées ci-dessus présentent généralement un point de fusion commençante inférieur à 45 °C.

On peut également utiliser la cire microcristalline commercialisée sous la référence SP18 par la société STRAHL and PITSCH qui présente une dureté d'environ 0,46 MPa et une valeur de collant d'environ 1 N.s.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 μm.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif

et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

*Polymère filmogène lipophile*

La composition selon l'invention peut comprendre au moins un polymère filmogène lipophile qui peut être liposoluble (c'est-à-dire soluble dans une phase grasse liquide comprenant des huiles ou solvants organiques tels que ceux décrits précédemment)

ou être présent dans la composition sous la forme de particules en dispersion dans une phase solvant non aqueuse avec laquelle il est compatible, qui peut être la phase huileuse de la composition selon l'invention.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisées par la société Wacker sous la référence Resin MK tels que la Belsil PMS MK, ou par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

Le polymère filmogène lipophile ou liposoluble peut être également présent dans la composition sous la forme de particules en dispersion dans une phase solvant non aqueuse qui peut être celle de la composition selon l'invention. Les techniques de préparation de ces dispersions sont bien connues de l'homme du métier.

Comme exemples de dispersions non aqueuses de polymère filmogène, on peut citer les dispersion décrite par exemple dans le document EP 749 746 et notamment les particules de polymères acryliques, stabilisées en surface par un stabilisant, en dispersion dans une phase grasse (par exemple l'isododécane) comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

## ADDITIFS

### *Matière colorante*

Les compositions conformes à l'invention peuvent également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi celles listées ci-avant.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

Selon un mode particulier, les oxydes de fer seront présents en une teneur allant de 0,01 à 15% en poids, de préférence de 0,01 à 10% en poids par rapport au poids total de ladite comspoition.

### *Fibres*

Les compositions conformes à l'invention peuvent en outre comprendre des fibres qui permettent une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, en particulier de 0,1 mm à 5 mm et plus particulièrement de 0,3 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.
A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénominations KERMEL®, KERMEL TECH® par la société RHODIA ou de poly-(p-phénylène-téréphtalamide) (ou d'aramide) notamment vendues sous la dénomination Kevlar ® par la société DUPONT DE NEMOURS.
Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

*Actifs cosmétiques*

Comme actifs cosmétiques pouvant être utilisés dans les compositions conformes à l'invention, on peut citer notamment des antioxydants, les conservateurs, les parfums, les neutralisants, émollients, des hydratants, des vitamines et des filtres en particulier solaires.
Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

## ENSEMBLE

L'ensemble selon la présente invention comprend au moins un applicateur de la composition cosmétique conforme à l'invention.
La composition est appliquée à l'aide de l'applicateur, appelé également moyen d'application, décrit plus particulièrement ci-après. Dans les modes de réalisation qui vont être décrits ci-après, c'est l'applicateur qui est couplé à un organe vibrant pour mettre en vibration la composition, simultanément à son application sur les cils, ou postérieurement à son application.
L'ensemble selon l'invention se présente de préférence sous forme d'un seul et même conditionnement. Il peut ainsi se présenter comme un récipient délimitant au moins un compartiment ou réservoir qui comprend la composition selon l'invention, ledit compartiment étant éventuellement fermé par un élément de fermeture, et au moins un moyen d'application vibrant pour la composition.
L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.
Le récipient peut être au moins pour partie réalisé en matériau thermoplastique. A titre d'exemples de matériaux thermoplastiques, on peut citer le polypropylène ou le polyéthylène.
Alternativement, le récipient est réalisé en matériau non thermoplastique, notamment en verre ou en métal (ou alliage).
Le récipient est de préférence équipé d'un essoreur disposé au voisinage d'au moins une ouverture du récipient. Un tel essoreur permet d'essuyer l'applicateur et éventuellement, la tige dont il peut être solidaire. Un tel essoreur est décrit par exemple dans le brevet FR 2 792 618. L'essoreur peut par exemple comporter un bloc d'un matériau alvéolaire tel qu'une mousse à cellules ouvertes ou fermées, avec ou sans flocage. En variante, l'essoreur peut comporter un matériau non alvéolaire, éventuellement floqué, par exemple un élastomère ou une polyoléfine. Dans ce cas notamment, l'essoreur peut par exemple comporter au moins une fente et/ou comporter une lèvre agencée pour essorer la tige.
Selon un mode de réalisation particulièrement préféré, l'ensemble de maquillage comprend un réservoir comprenant la composition selon l'invention, le réservoir étant muni du moyen d'application (ou applicateur) de la composition décrit ci-après.
Selon une alternative, le réservoir a une capacité inférieure ou égale à 20 ml.
Selon une autre alternative, le réservoir a une capacité strictement supérieure à 20 ml.

## MOYEN D'APPLICATION

Le moyen d'application, ou applicateur, peut être utilisé pour appliquer la composition sur une région à maquiller.
L'applicateur peut encore être utilisé pour la finition du maquillage, sur une région maquillée ou chargée de produit au moyen d'un autre applicateur.
Avantageusement il est également utilisé pour faire vibrer la composition au moment de son application ou

après.

La composition selon l'invention peut être prélevée dans un récipient en immergeant l'élément d'application dans celui-ci. Lors du prélèvement, l'élément d'application peut être soumis aux vibrations de la source vibrante, ce qui peut permettre d'obtenir le cas échéant une charge plus homogène de produit sur l'élément d'application.

Lorsque le récipient comporte un organe d'essorage au travers duquel l'élément d'application est retiré, l'élément d'application peut également être soumis à des vibrations au moment du passage à travers l'organe d'essorage, ce qui peut permettre d'obtenir un essorage de l'élément d'application différent de celui qui existe en l'absence de vibration de l'élément d'application. L'utilisateur peut ainsi par exemple choisir entre au moins deux degrés d'essorage de l'élément d'application, selon que l'élément d'application vibre ou non au moment de la traversée de l'organe d'essorage.

Il s'avère en outre plus avantageux de faire vibrer l'applicateur que l'organe d'essorage, puisque les vibrations de l'applicateur peuvent être utiles pour l'application également.

L'organe d'essorage pourra présenter une ouverture nettement plus large de la tige éventuelle portant l'élément d'application.

Le procédé de maquillage ou de soin des cils selon l'invention peut comporter le réglage par l'utilisateur d'une fréquence de vibration et/ou le réglage d'une amplitude de vibration, par exemple en agissant sur un organe de réglage.

L'amplitude de vibration de l'élément d'application lors de l'application est par exemple inférieure ou égale à 5 mm, mieux inférieure ou égale à 3 mm, des microvibrations de l'élément d'application étant préférables à des vibrations de plus forte amplitude.

L'amplitude des vibrations est éventuellement plus grande lors du prélèvement de la composition dans un récipient ou lors de la traversée d'un organe d'essorage.

Selon un mode particulier de l'invention, l'amplitude maximale des vibrations (telle qu'imposée typiquement par l'amplitude du mouvement de la masselotte associée au moteur) est comprise entre 0,4 et 5mm, de préférence entre 0,5 et 3mm et par exemple, autour de 0,5mm.

Les vibrations peuvent être obtenues de diverses manières, par voie mécanique, hydraulique, pneumatique, électronique ou électromécanique.

La source vibrante peut comporter par exemple un moteur entraînant une masselotte, un excentrique, un électroaimant, un vibreur piézoélectrique ou mécanique.

Le contact entre la source vibrante et la partie applicatrice pourra être ponctuel ou étendu, selon par exemple l'amplitude, la fréquence et l'orientation des vibrations.

La source vibrante peut générer des vibrations, notamment sinusoïdales, par exemple de fréquence comprise entre 1 et 500 Hz, mieux 10 et 300 Hz, encore mieux 50 et 200 Hz.

La fréquence des vibrations est par exemple supérieure ou égale à 20 Hz.

La source vibrante peut comprendre une source électrique, par exemple une pile de 1,5 V. Le moteur peut être par exemple agencé de telle sorte que lorsqu'il est relié à une pile de 1,5 V, il tourne à une vitesse comprise entre 5 000 et 12 000 tr/min.

Selon un mode particulier, le moteur tourne à une vitesse comprise entre 5000 et 9000 tr/min.

Le système vibrant peut être configuré de manière à générer des vibrations qui sont soit principalement perpendiculaires à l'axe de la tige au bout de laquelle est monté l'applicateur (conformément à ce qui est décrit dans la demande de brevet US2006/0032512 évoquée précédemment), soit principalement longitudinalement à l'axe de la tige. Le sens des vibrations s'entend du sens des vibrations lorsque l'élément d'application (la brosse) est chargé en composition (mascara) et qu'il est mis en engagement avec les cils, c'est-à-dire lorsqu'il est en condition d'application.

Selon un mode préféré de l'invention, les vibrations sont orientées principalement longitudinalement à l'axe de la tige au bout de laquelle sont fixés les moyens d'application. Cette orientation s'avère bénéfique à la qualité du maquillage obtenu, notamment quant au lissage et à la séparation des cils. Un tel sens de vibration peut être obtenu en configurant le système vibrant de la manière représentée à la figure 35 de la demande de brevet FR2919476. Dans cette configuration, le moteur, sous forme d'un disque plat à la périphérie duquel sont réparties de manière équidistante une ou plusisurs masselottes, a son axe de rotation sensiblement perpendiculaire à l'axe de la tige au bout de laquelle sont fixés les moyens d'application. Avec cette configuration, le mouvement vibratoire de l'applicateur est sensiblement dans un plan (le plan du disque plat). A l'intérieur de ce plan, lorsque la brosse, chargée en mascara, est mise en engagement avec la frange de cils, le mouvement vibratoire de la brosse a sa composante majoritaire disposée dans l'axe de la tige.

Selon un mode de réalisation particulier, l'unité vibrante est montée de manière amovible relativement au reste de l'unité d'application. Cela permet d'utiliser une unité vibrante en association avec des parties applicatrices différentes, afin par exemple de traiter différemment selon les parties applicatrices sélectionnées.

La partie applicatrice peut comporter, éventuellement, un élément de fermeture d'un récipient contenant la composition selon l'invention.

Le procédé d'application de la composition selon l'invention sur les cils peut également comporter les étapes

suivantes :

i) former un dépôt de la composition cosmétique sur les cils,
ii) simultanément à la formation du dépôt ou postérieurement au dépôt, soumettre ce dernier à un mouvement vibratoire,
iii) laisser le dépôt sur les cils, le dépôt pouvant sécher.

L'élément d'application est agencé pour appliquer un produit sur les cils, et peut comporter par exemple une brosse ou un peigne.
La brosse peut comporter une âme torsadée et des poils pris entre les spires de l'âme, ou être réalisée autrement encore.
Le peigne est par exemple réalisé d'une seule pièce par moulage de matière plastique.
L'élément d'application peut être magnétique.
Dans certains exemples de réalisation, l'élément d'application est monté à l'extrémité d'une tige, laquelle peut être flexible, ce qui peut contribuer à augmenter l'amplitude des vibrations de l'élément d'application et/ou améliorer le confort à l'application.
L'applicateur peut comporter ou non un réservoir contenant la composition.
Lorsque l'applicateur ne comporte pas de réservoir contenant la composition, la composition est par exemple contenue dans un récipient et l'élément d'application est par exemple chargé en composition en étant introduit au moins partiellement dans ce récipient. Le récipient peut comporter ou non un organe d'essorage.
La source vibrante peut être présente à demeure sur l'applicateur ou, en variante, faire partie d'une unité vibrante pouvant être fixée de manière amovible sur une partie applicatrice de l'applicateur.
L'ensemble peut ainsi comporter une unité vibrante et plusieurs parties applicatrices associées à des produits ou éléments d'application différents.
L'ensemble comporte par exemple une unité vibrante et au moins deux parties applicatrices choisies parmi des parties applicatrices destinées au maquillage ou au soin des cils.
Le réservoir peut être fixé de manière amovible ou non sur l'applicateur. Lorsque le réservoir est fixé à demeure sur l'applicateur, pour alimenter celui-ci en produit, la paroi du réservoir sert par exemple à la préhension de l'applicateur.
L'ensemble selon l'invention peut également comporter au moins deux éléments d'application différents pouvant être sélectivement montés sur l'applicateur, ce dernier comportant une source vibrante.
L'ensemble comporte par exemple plusieurs parties applicatrices différentes et une unité vibrante amovible ou plusieurs éléments d'application agencés pour se monter sur une partie de l'applicateur contenant la source vibrante.
Des ensembles d'application et de vibration pouvant être utilisés pour la mise en oeuvre de l'invention sont décrits notamment dans la demande W02006/090343. D'autres sont décrits également dans les demandes WO2006/020577, WO2006/130644, WO2006/130643, WO 2006/130642, ou US2007/0272269. Le contenu de ces demandes est incorporé par référence à la présente demande.
L'ensemble pourra être mieux compris à la lecture de la description détaillée qui va suivre, d'exemples de mise en oeuvre non limitatifs de celle-ci, et à l'examen des dessins annexés, sur lesquels :

- la figure 1 représente de manière schématique, avec coupe longitudinale partielle, un dispositif de conditionnement et d'application conforme à un exemple de mise en oeuvre de l'invention,
- la figure 2 représente isolément l'applicateur de la figure 1, avec coupe longitudinale partielle et schématique.

Les figures 1 et 2 auxquelles il est fait référence illustrent un dispositif 1 de conditionnement et d'application conforme à un mode de réalisation décrit dans la demande W02006/090343. Selon ce mode de réalisation, l'ensemble de conditionnement et d'application comporte un applicateur 2 et un récipient 3 contenant une réserve d'un produit de maquillage P, en l'espèce une composition selon l'invention, par exemple du mascara.
Le récipient 3 est conventionnel dans l'exemple illustré et comporte un corps 5 pourvu d'un col 6 fileté extérieurement. Un organe d'essorage 7 est fixé dans le col 6.
L'applicateur 2 comporte un élément d'application 10 constitué dans l'exemple considéré par une brosse à mascara. L'élément d'application 10 est monté à l'extrémité d'une tige 11 dont l'autre extrémité est solidaire d'un boîtier 13 comportant une partie d'extrémité 14 agencée pour se visser sur le col 6 et fermer ainsi de manière étanche le récipient 3.
Le boîtier 13 loge une source vibrante permettant de faire vibrer l'élément d'application 10 lors de l'application du produit de maquillage et/ou lors du prélèvement de produit dans le récipient et/ou de l'extraction de l'élément d'application.
La source vibrante comporte dans l'exemple considéré un vibreur 16 composé d'un moteur 17 et d'une masselotte 18 entraînée en rotation par le moteur et dont le centre de gravité est excentré par rapport à l'axe de rotation.

Le moteur 17 est alimenté électriquement par une source d'énergie 20 telle que par exemple une pile bâton, logée dans le boîtier 13 dans l'exemple considéré et reliée électriquement au moteur par un interrupteur 19.

Le boîtier 13 comporte un couvercle amovible 22 permettant la mise en place et le remplacement de la pile 20 et pouvant assurer un contact électrique avec cette dernière.

Dans l'exemple considéré, l'axe de rotation du moteur 17 coïncide sensiblement avec l'axe longitudinal X de l'applicateur, de sorte que les vibrations sont produites sensiblement perpendiculairement à l'axe X.

Ces vibrations se propagent le long de la tige 11 et amènent l'élément d'application 10 à vibrer sensiblement perpendiculairement à l'axe X lors de l'application du produit sur les cils.

Sur cette figure, la brosse a été représentée de manière très schématique pour bien illustrer le fait que l'invention n'est pas limitée à un élément d'application particulier.

La brosse 10 comporte par exemple des poils dont les extrémités sont disposées comme des nappes hélicoïdales. L'oscillation de la brosse 10 permet d'obtenir un mouvement relatif des poils de la brosse 10 le long des cils C et ainsi de lisser le produit à la surface de ceux-ci et/ou d'orienter des fibres éventuelles contenues dans le produit P. Les vibrations de la brosse 10 peuvent également faciliter la séparation des cils.

Le moteur 17 peut être mis en marche lors de l'application du produit sur les cils, que ce soit lors de l'application initiale du produit ou après celle-ci, pour parfaire le maquillage.

L'utilisateur peut également faire vibrer la brosse au moment où elle est plongée dans le récipient 3, de façon par exemple à faciliter le chargement en produit de la brosse, par exemple obtenir une charge plus homogène.

L'utilisateur peut encore faire vibrer la brosse 10 au moment où elle franchit l'organe d'essorage 7.

La tige peut être réalisée avec une section transversale constante ou non.

De préférence, la tige 11 est flexible, ce qui peut accroître l'amplitude des vibrations de la brosse 10, l'homme du métier pouvant choisir les dimensions de la tige en fonction par exemple de la nature de l'élément d'application, du produit et du traitement à effectuer.

Lorsque l'organe d'application est configuré sous forme d'un peigne, ce dernier peut être conforme à ce qui est décrit notamment dans les publications US 2003-0089379-A1, US 6 655 390, US 6 814 084, US 6675 814, US 6 581 610, US 6 546 937, US 6 539 950, US 6 412 496 ou US 6 343 607, cette liste n'étant pas limitative.

L'invention n'est pas limitée à un élément d'application particulier et ce dernier peut notamment être muni d'un moyen permettant de chauffer le produit et/ou les cils lors de l'application.

Outre le fait de faire vibrer la composition, l'applicateur vibrant peut également permettre de chauffer la composition, notamment pour en modifier la réhéologie lors de l'application. Dans ce cas de figure, l'interrupteur peut par exemple prendre plusieurs positions, l'une d'entre elles correspondant par exemple au chauffage seul, et les autres aux vibrations seules ou au chauffage et vibrations simultanément.

Diverses modifications peuvent être apportées aux exemples de réalisation qui viennent d'être décrits sans que l'on sorte du cadre de la présente invention.

Par exemple, la source vibrante peut comporter un vibreur autre qu'un moteur électrique entraînant en rotation une masselotte et autre qu'un vibreur piézoélectrique. La source vibrante peut notamment comporter tout système électromécanique, pneumatique, hydraulique, mécanique, électronique ou électromécanique capable de produire des vibrations.

La source vibrante peut comporter des moyens de contrôle des vibrations autres qu'un simple interrupteur de marche/arrêt et notamment comporter des moyens de contrôle, mécaniques ou électroniques, permettant de régler l'amplitude et/ou la fréquence des vibrations. Les moyens de contrôle peuvent par exemple comporter un potentiomètre ou un commutateur, rotatif ou linéaire, permettant de sélectionner au moins deux vitesses de rotation du moteur électrique dans le cas où le vibreur comporte un tel moteur.

La source vibrante peut comporter plus d'un vibreur et par exemple deux vibreurs agencés pour produire des oscillations selon des directions différentes. Dans ce cas, la source vibrante peut également comporter par exemple un sélecteur permettant de sélectionner le ou les vibreurs que l'on souhaite mettre en fonctionnement.

La source vibrante peut, le cas échéant, être orientée par l'utilisateur de façon à faire vibrer l'élément d'application avec des vibrations d'orientation voulue.

La source vibrante peut comporter une source d'énergie qui peut être autre qu'une pile et notamment comporter un ou plusieurs accumulateurs ou condensateurs. Le cas échéant, la source vibrante peut être agencée de manière à pouvoir être rechargée en électricité en étant reposée sur un socle.

Le cas échéant, la source vibrante peut être alimentée par le secteur par l'intermédiaire d'un transformateur éventuel.

La source vibrante peut être montée de multiples manières dans un logement correspondant de l'applicateur, et le montage de la source vibrante est par exemple conçu de manière à favoriser le transfert de vibrations vers l'élément d'application.

La mise en fonctionnement de la source vibrante pourra s'effectuer d'autres manières encore que celles qui viennent d'être décrites.

Un interrupteur ayant la forme d'une pince de stylo pourra être utilisé ou tout autre contacteur disposé sur le côté ou en bout, suivant le type d'application.

Les éléments d'application pourront être de tous types, notamment à fentes capillaires ou autres encore.

Les éléments d'application pourront être réalisés de diverses manières, notamment par moulage, surinjection, agrafage, torsadage.

Les éléments d'application pourront être à usage unique, le cas échéant.

Les éléments d'application pourront être fixés par tous moyens sur la partie applicatrice, notamment par collage, soudage, matriçage, encliquetage, vissage, avec des aimants, par friction, par accrochage de type VELCRO®, par serrage entre des mors ou les branches d'une pince.

La tension d'alimentation du vibreur dans le cas où celui-ci est électrique, est par exemple comprise entre 1 V et 9 V.

L'élément d'application peut être entraîné en rotation, le cas échéant, par exemple comme décrit dans les brevets US 4 937 326, US 4 922 934 et US 6 565 276, dont les contenus sont incorporés à la présente par référence.

La mise en vibration d'une brosse à mascara lorsque celle-ci tourne peut réduire le risque que les cils ne se coincent dans les poils.

L'application du produit peut se faire, notamment lorsque l'invention est mise en oeuvre pour appliquer un produit sur les cils, après avoir chauffé le produit, par exemple par passage dans un four à micro-ondes.

La présente invention est illustrée par les exemples qui suivent. Sauf indication contraire, les quantités indiquées sont exprimées en pourcentage massique par rapport au poids total de la composition.

<u>Dans tous les exemples qui suivent, le protocole de mesure de la viscosité des compositions est le suivant :</u>

**<u>Préparation des échantillons :</u>**

La brosse vibrante selon l'invention est immergée dans le mascara puis, une fois sortie dudit mascara, subit :

- aucune vibration
- 3 minutes de vibration (brosse tenue par les moyens de préhension sans être en engagement avec les cils).
- 10 minutes de vibration (brosse tenue par les moyens de préhension sans être en engagement avec les cils).

On utilise une vibration ayant une amplitude d'environ 0,5mm, la vibration étant obtenue au moyen d'un moteur ayant une vitesse de rotation de 7000tr/min +/- 2000 sous forme d'un disque plat dont l'axe de rotation est sensiblement perpendiculaire à l'axe de la tige. La fréquence correspondante est de 117Hz+/-33.

Le mascara est alors prélevé sur la brosse à l'aide d'une spatule et placé sur le plan inférieur du rhéomètre (temps de repos entre la fin de la vibration et le début de la mesure ~2minutes).

**<u>Protocole de mesure de rhéologie :</u>**

Rhéomètre RS600
Mobile plan/plan 20mm - entrefer 0.2mm
Mesures faites à 25°C

Vitesse de montée du plan inférieur pour venir se positionner à un entrefer de 0.2mm: 1.25 mm/min.

Après positionnement de l'échantillon sur le mobile, 30secondes d'attente au repos.
Rampe croissante en 60secondes de 0 à 1000$s^{-1}$.
Rampe décroissante en 60secondes de 1000 à 0$s^{-1}$.

Pour chaque formule, la mesure est reproduite 3 fois (sur un nouvel échantillon à chaque fois).

**<u>Résultats :</u>**

Sur chaque courbe, les valeurs de viscosité sont prises en Poise à 100$s^{-1}$, 400$s^{-1}$ et 900$s^{-1}$ sur la montée en cisaillement. Pour un même échantillon, la valeur moyenne de viscosité est calculée à ces différents cisaillements.
Le pourcentage de variation de viscosité aux 3 différents cisaillements est calculé pour chaque formule ayant subi les vibrations par rapport à la même formule au repos.

Une variation de viscosité est dite significative (seuil de significativité défini par rapport à l'incertitude liée à la reproductibilité des essais, de sorte que le seuil est supérieur à toute variation qui pourrait être attribuée à la reproductibilité) dès lors qu'elle est supérieure ou égale à 10% en valeur absolue selon le protocole décrit ci-dessus, pour l'une au moins des mesures prises à $100s^{-1}$, $400s^{-1}$ et $900s^{-1}$ sur la montée en cisaillement, après 3 et/ou 10 minutes de vibration. Les compositions présentant une variation de viscosité supérieure ou égale à 10% en valeur absolue, de préférence supérieure ou égale à 15% en valeur absolue et mieux, supérieure ou égale à 20% en valeur absolue, seront préférées selon l'invention pour une utilisation avec un applicateur vibrant.

Selon un premier mode, lesdites compositions préférées pourront présenter une diminution de viscosité d'au moins 10% (soit une variation négative d'au moins 10% par rapport au témoin sans vibration) lorsqu'elles sont utilisées avec un applicateur vibrant.

Selon un autre mode, lesdites compositions préférées pourront présenter une augmentation de viscosité d'au moins 10% (soit une variation positive d'au moins 10% par rapport au témoin sans vibration) lorsqu'elles sont utilisées avec un applicateur vibrant.

Ce protocole permet donc de sélectionner ou détecter toute composition de mascara sensible au cisaillement et par conséquent toute indiquée pour être utilisée avec un applicateur vibrant selon l'invention.

La présente invention concerne donc également un ensemble de maquillage et/ou de soin des matières kératiniques, notamment des cils ou des sourcils comprenant:

- un récipient délimitant au moins un compartiment contenant au moins une composition de maquillage et/ou de soin desdites matières kératiniques ;
- au moins un applicateur comprenant un élément d'application pour appliquer la composition sur lesdites matières kératiniques ; et
- une source vibrante permettant, préalablement, simultanément ou postérieurement à l'application sur les matières kératiniques de la composition, de faire vibrer cette dernière,
- **caractérisé en ce que** ladite composition est telle que définie précédemment et présente une variation de viscosité d'au moins 10% en valeur absolue, lorsqu'elle est soumise à une source vibrante d'amplitude 0,5mm, la vibration étant obtenue au moyen d'un moteur ayant une vitesse de rotation de 7000tr/min +/- 2000 sous forme d'un disque plat dont l'axe de rotation est sensiblement perpendiculaire à l'axe de la tige (fréquence 117Hz+/-33), par rapport à la même composition au repos (sans vibration),
  la variation de viscosité de ladite composition étant mesurée à l'aide d'un Rhéomètre RS600 selon le protocole suivant :

    a) l'applicateur vibrant est immergé dans ladite composition, puis une fois sorti, soumis à aucune vibration (témoin), 3 ou 10 minutes de vibration
    b) on prélève ladite composition à l'aide d'une spatule et on la positionne sur le plan inférieur d'un Rhéomètre RS600 (Mobile plan/plan 20mm - entrefer 0.2mm; Vitesse de montée du plan inférieur pour venir se positionner à un entrefer de 0.2mm : 1.25 mm/min
    c) on réalise des courbes de cisaillement à partir de Rampe croissante en 60secondes de 0 à $1000s^{-1}$/ Rampe décroissante en 60secondes de 1000 à $0s^{-1}$
    d) on mesure les valeurs de viscosité en Poise à $100s^{-1}$, $400s^{-1}$ et $900s^{-1}$ sur la montée en cisaillement
    e) on calcule le pourcentage de variation de viscosité aux 3 différents cisaillements pour chaque formule ayant subi les vibrations par rapport à la même formule au repos.

La présente invention concerne également une méthode de sélection ou détection d'une composition utilisable avec un applicateur vibrant selon l'invention comprenant les étapes suivantes :

a) l'applicateur vibrant (source vibrante d'amplitude 0,5mm, la vibration étant obtenue au moyen d'un moteur ayant une vitesse de rotation de 7000tr/min +/- 2000 sous forme d'un disque plat dont l'axe de rotation est sensiblement perpendiculaire à l'axe de la tige soit une fréquence de 117Hz+/-33) est immergé dans ladite composition, puis une fois sorti, soumis à aucune vibration (témoin), 3 ou 10 minutes de vibration ;
b) on prélève ladite composition à l'aide d'une spatule et on la positionne sur le plan inférieur d'un Rhéomètre RS600 (Mobile plan/plan 20mm - entrefer 0.2mm; Vitesse de montée du plan inférieur pour venir se positionner à un entrefer de 0.2mm : 1.25 mm/min
c) on réalise des courbes de cisaillement à partir de Rampe croissante en 60secondes de 0 à $1000s^{-1}$/ Rampe décroissante en 60secondes de 1000 à $0s^{-1}$
d) on mesure les valeurs de viscosité en Poise à $100s^{-1}$, $400s^{-1}$ et $900s^{-1}$ sur la montée en cisaillement
e) on calcule le pourcentage de variation de viscosité aux 3 différents cisaillements pour chaque formule ayant subi les vibrations par rapport à la même formule au repos.
f) on sélectionne les compositions pour lesquelles on obtient une variation de viscosité d'au moins 10% en valeur absolue par rapport au témoin.

**Exemple 1: Mascara selon l'invention à phase continue huileuse avec argile lipophile plaquettaire**

On prépare la composition suivante :

| | |
|---|---|
| **CIRE DE CARNAUBA** | **4,7** |
| **CIRE D'ABEILLE** | **8,3** |
| **PARAFFINE** | **2,8** |
| **HUILE DE JOJOBA HYDROGENEE** | **0,1** |
| **CIRE DE SON DE RIZ** | **2,8** |
| **MICRO-DISPERSION DE CIRE DE CARNAUBA AVEC 10% D'ETHANOL(Mexoryl SAP de Chimex)** | **7** |
| **OXYDES DE FER NOIR** | **4,2** |
| **Conservateurs** | **Qs** |
| **CONDENSAT DIACIDE EN C36 HYDROGENE/ETHYLENE DIAMINE, ESTERIFIEPAR ALCOOL STEARYLIQUE (UNICLEAR 100 VG)** | **1** |
| **POLYLAURATE DE VINYLE (Mexomère PP de Chimex)** | **2,2** |
| **COPOLYMERE ACETATE DE VINYLE / STEARATE D'ALLYLE(65/35) (Mexomère PQ de Chimex)** | **3,3** |
| **COPOLYMERE VINYLPYRROLIDONE/EICOSENE (Antaron V220 d'ISP)** | **2** |
| **ETHANOL** | **2** |
| **CARBONATE DE PROPYLENE** | **1,9** |
| **ISODODECANE** | **Qsp 100** |
| **HECTORITE MODIFIEE DISTEARYL DIMETHYL AMMONIUM (Bentone 38 VCG)** | **5,8** |
| **TALC** | **1** |

**Exemple 2: Mesure de l'impact de la vibration sur le mascara de l'exemple 1**

Protocole : voir ci-avant

Résultats : on obtient les résultats suivants :

| Viscosité | Viscosité @ 100 $s^{-1}$ | Viscosité @ 400 $s^{-1}$ | Viscosité @ 900 $s^{-1}$ |
|---|---|---|---|
| (durant la montée) | Poise | Poise | Poise |
| Contrôle | 107,67 | 24,17 | 10,30 |
| 3 min vibration | 113,00 | 26,00 | 10,90 |
| % vs contrôle | 4,95 | 7,59 | 5,83 |
| 10 min vibration | 129 | 36,67 | 13,97 |
| % vs contrôle | 19,81 | 51,72 | 35,60 |

Le mascara selon l'exemple 1 est donc particulièrement sensible à la vibration (écarts minimums de 20% après 10 minutes de vibration) : sa viscosité se trouve significativement augmentée lorsque la durée des vibrations augmente.

**Exemple 3: Mesure de l'impact de la vibration sur le mascara Neutrogena Weightless Volume Wax Free**

Le mascara Neutrogena Weightless Volume Wax Free est commercialisé par Johnson & Johnson et comprend comme ingrédients, d'après le full ingredient labelling:

WATER
MICA
POLYISOBUTENE
CYCLOPENTASILOXANE
DIMETHICONE COPOLYOL POLYACRYLATE
GLYCERIN
POLYSORBATE 20
POLYURETHANE-1
HYDROXYETHYLCELLULOSE
DIMETHICONE
PROPYLENE GLYCOL
DIPROPYLENE GLYCOL
PANTOTHENIC ACID
ASCORBIC ACID
RETINYL PALMITATE
HYDROLYZED WHEAT PROTEIN
TOCOPHERYLACETATE
SIMETHICONE
MATRICARIA (CHAMOMILLA RECUTITA) EXTRACT
GERANIUM MACULATUM EXTRACT
POLYSILICONE-11
PVP/HEXADECENE COPOLYMER
POLYMETHYL METHACRYLATE
ETHANOL
CITRIC ACID
PHENOXYETHANOL
ISOPROPYLPARABEN
ISOBUTYLPARABEN
BUTYLPARABEN
METHYLPARABEN
May contain: IRON OXIDES

Ce mascara comprend donc notamment du mica comme charge plaquettaire, ainsi que du polysorbate-20 comme tensioactif non ionique.

Protocole : le protocole de mesure de la viscosité est identique à celui décrit ci-avant.

Résultats : on obtient les résultats suivants :

| Viscosité (durant la montée) | Viscosité @ 100 $s^{-1}$ Poise | Viscosité @ 400 $s^{-1}$ Poise | Viscosité @ 900 $s^{-1}$ Poise |
|---|---|---|---|
| Contrôle | 130,00 | 34,67 | 19,47 |
| 3 min vibration | 133,33 | 43,80 | 22,50 |
| % vs contrôle | 2,56 | 26,35 | 15,58 |
| 10 min vibration | 131,67 | 44,03 | 23,33 |
| % vs contrôle | 1,28 | 27,02 | 19,86 |

Le mascara Neutrogena Weightless Volume Wax Free est donc particulièrement sensible à la vibration (écarts minimums de 15% après 3 ou 10 minutes de vibration à 400 et 900 $s^{-1}$): sa viscosité se trouve significativement augmentée lorsque la durée des vibrations augmente.

**Exemple 4: Mascara selon l'invention à phase continue aqueuse avec structurant huileux (polymère siliconé polyamide)**

On prépare la composition suivante :

| | |
|---|---|
| **EDTA** | **0,2** |
| **HYDROXYSTEAROYL STEARATE D'ALCOOLS GRAS EN C18-C38 (Kester Wax K82P de Koster Keunen)** | **5** |
| **ISONONANOA TE D'ISONONYLE** | **10,46** |
| **OXYDES DE FER NOIR** | **7,14** |
| **DEHYDROACETATE DE SODIUM** | **0,2** |
| **Conservateurs** | **Qs** |
| **TALC** | **3** |
| **PHOSPHATE DE MONO-CETYLE MONO-POTASSIQUE NON STABILISE (Amphisol K)** | **3,21** |
| **TRISTEARATE DE SORBITANE (Span 65V de Croda)** | **0,96** |
| **PEG-40 STEARATE (Myrj 52 P de Croda)** | **2,25** |
| **SIMETHICONE** | **0,19** |
| **NYLON-611/DIMETHICONE COPOLYMER (and) PPG- 3 MYRISTYL ETHER (Dow Corning 2-8178 Gellant de Dow Corning)** | **9,26** |
| **COPOLYMERES ACRYLIQUE ET STYRÈNE/ACRYLIQUE EN EMULSION AQUEUSE A 40% DANS MELANGE EAU/BUTYLENE GLYCOL/SODIUM LAURYL ETHER SULFATE PROTEGE (Syntran 5760 d'Interpolymer)** | **5** |
| **HYDROXYETHYLCELLULOSE** | **0,88** |
| **GOMME ARABIQUE** | **3,38** |
| **1,3-BUTYLENE GLYCOL** | **2,5** |
| **EAU** | **Qsp 100** |

**Exemple 5: Mesure de l'impact de la vibration sur le mascara de l'exemple 4**

Protocole : le protocole de mesure de la viscosité est identique à celui décrit ci-avant.

Résultats : on obtient les résultats suivants :

| Viscosité (durant la montée) | Viscosité @ 100 $s^{-1}$ Poise | Viscosité @ 400 $s^{-1}$ Poise | Viscosité @ 900 $s^{-1}$ Poise |
|---|---|---|---|
| Contrôle | 139,33 | 34,13 | 20,67 |
| 3 min vibration | 141,00 | 34,67 | 21,27 |
| % vs contrôle | 1,20 | 1,56 | 2,90 |
| 10 min vibration | 166,67 | 38,30 | 24,27 |
| % vs contrôle | 19,62 | 12,21 | 17,42 |

Le mascara selon l'exemple 4 est donc sensible à la vibration (écarts minimums de 12% après 10 minutes de vibration) : sa viscosité se trouve significativement augmentée lorsque la durée des vibrations augmente.

**Exemple 6: Mascara selon l'invention à phase continue aqueuse avec structurant huileux (polymère semi-cristallin)**

Un mascara est préparé avec les proportions suivantes :

| | |
|---|---|
| Phase grasse | 35% |
| Acide stéarique | 5,82% |
| Neutralisants | 2,9 % |

(suite)

| | |
|---|---|
| Oxyde de fer noir | 8% |
| Hydroxyéthylcellulose | 0,91% |
| Gomme arabique | 3,45% |
| Additifs, conservateurs, eau | qsp |

Où la phase grasse est constituée du mélange suivant :
Phase grasse = mélange polybutène (1) / copolymère acrylate de stéaryle N-vinyl pyrrolidonne (2) (40/60) de point de fusion de 56°C.

(1) : Indopol H 100 de la société AMOCO
(2) : Polymère basique de point de fusion de 56°C préparé selon le mode opératoire suivant :

Dans un réacteur d'11 muni d'une agitation centrale avec ancre, d'un réfrigérant et d'un thermomètre, on introduit 120 g de cyclohexane que l'on chauffe de la température ambiante à 80°C en 45 min. A 80°C, on introduit en 2h le mélange $C_1$ suivant :

40 g de cyclohexane + 4 g de Triganox 141 [2,5 bis (2-éthyl hexanoyl peroxy) - 2,5 - diméthyl hexane].
30 min après le début de la coulée du mélange $C_1$, on introduit en 1h30 le mélange $C_2$ constitué de :
190 g d'acrylate de stéaryle + 10 g de N-vinyl pyrrolidone + 400 g de cyclohexane.

A la fin des deux coulées, on laisse agir 3h supplémentaires à 80°C puis on distille à pression atmosphérique la totalité du cyclohexane présent dans le milieu réactionnel.
On obtient alors le polymère à 100 % en poids en matière active.
Sa masse moléculaire moyenne en poids $M_w$ est de 38 000 exprimée en équivalent polystyrène et sa température de fusion pF est de 56°C, mesurée par D.S.C.

**Exemple 7: Mesure de l'impact de la vibration sur le mascara de l'exemple 6**

Protocole : le protocole de mesure de la viscosité est identique à celui décrit ci-avant.
Résultats : on obtient les résultats suivants :

| Viscosité (durant la montée) | Viscosité @ 100 $s^{-1}$ Poise | Viscosité @ 400 $s^{-1}$ Poise | Viscosité @ 900 $s^{-1}$ Poise |
|---|---|---|---|
| Contrôle | 97,67 | 35,87 | 17,20 |
| 3 min vibration | 102,00 | 36,10 | 17,67 |
| % vs contrôle | 4,44 | 0,65 | 2,71 |
| 10 min vibration | 99,33 | 39,93 | 21,30 |
| % vs contrôle | 1,71 | 11,34 | 23,84 |

Le mascara selon l'exemple 6 est donc sensible à la vibration (écart minimum de 20% après 10 minutes de vibration à 900 $s^{-1}$): sa viscosité se trouve significativement augmentée lorsque la durée des vibrations augmente.

**Exemple 8: Mesure de l'impact de la vibration sur le mascara Magnascopic**

Le mascara Magnascopic est commercialisé par Estée Lauder et comprend comme ingrédients, d'après le full ingredient labelling :

WATER
ISODODECANE
ETHYLENEDIAMINE/STEARYL DIMER TALLATE
COPOLYMER (UNICLEAR)
CYCLOMETHICONE
KAOLIN

STEARIC ACID
STEARAMIDE MEA STEARATE
AMMONIUM SHELLACATE
POLYSORBATE 20
SILICA
SORBITAN TRISTEARATE
GLYCERYLSTEARATE
PEG-100 STEARATE
MAGNESIUM ALUMINUM SILICATE
HYDROXYETHYLCELLULOSE
PVP
TOCOPHERYL ACETATE
ACACIA SENEGAL GUM
PTFE
COFFEE (COFFEA ARABICA) SEED EXTRACT
PANTOTHENIC ACID POLYPEPTIDE
HYDROLYZED JOJOBA PROTEIN
SILK AMINO ACIDS
AMINOMETHYL PROPANEDIOL
PROPYLENE GLYCOL
TETRADIBUTYL PENTAERYTHRITYL
HYDROXYHYDROCINNAMATE
ISOPROPYL ALCOHOL
DISODIUM EDTA
CHLORPHENESIN
SORBIC ACID
PHENOXYETHANOL
METHYLPARABEN
BUTYLPARABEN
ETHYLPARABEN
PROPYLPARABEN
ISOBUTYLPARABEN
May contain :
MICA
TITANIUM DIOXIDE
IRON OXIDES
BRONZE POWDER
BLUE 1
ALUMINUM POWDER
YELLOW 5
FERRIC FERROCYANIDE
YELLOW 5 LAKE
ULTRAMARINES
CARMINE
CHROMIUM HYDROXIDE GREEN
CHROMIUM OXIDE GREENS
BISMUTH OXYCHLORIDE
BLU E 1 LAKE

Ce mascara comprend donc notamment de l'Uniclear (polymère polyamide) comme agent rhéologique de phase grasse.
Protocole : voir ci-avant
Résultats : on obtient les résultats suivants :

| Viscosité | Viscosité @ 100 $s^{-1}$ (Poise) | Viscosité @ 400 $s^{-1}$ (Poise) | Viscosité @ 900 $s^{-1}$ (Poise) |
|---|---|---|---|
| Contrôle | 96,00 | 24,93 | 10,66 |

(suite)

| Viscosité | Viscosité @ 100 $s^{-1}$ (Poise) | Viscosité @ 400 $s^{-1}$ (Poise) | Viscosité @ 900 $s^{-1}$ (Poise) |
|---|---|---|---|
| 3 min vibration | 69,75 | 22,11 | 9,58 |
| % vs contrôle | -27,35 | -11,31 | -10,13 |
| 10 min vibration | 69,3 | 22,48 | 9,83 |
| % vs contrôle | -27,82 | -9,83 | -7,79 |

Le mascara Magnascopic est donc particulièrement sensible à la vibration (écarts supérieurs à 10% après 3 minutes de vibration, et après 10 minutes de vibration à 100 $s^{-1}$): sa viscosité se trouve significativement diminuée lorsque la durée des vibrations augmente.

**Exemple 9 : Mesure de l'impact de la vibration sur un mascara selon l'invention contenant des charges plaquettaires de type mica-dioxyde de titane-oxyde de fer noir**

| Composition de Mascara testée | |
|---|---|
| Mica-oxyde de titane-oxyde de fer noir (58/18/24) (CI :77019+77891+77499) COLORONA PATINA SILVER® de la société MERCK | 22% |
| Acrylates/Steareth-20 methacrylate copolymer (Aculyn 22 Polymer® de Rohm et Haas) | 35% |
| Microdispersion de cire de carnauba (Mexoryl SAP® de Chimex) | 20% |
| Conservateurs qs | |
| Eau qsp | 100% |

| Viscosité | Viscosité @ 100 $s^{-1}$ (Poise) | Viscosité @ 400 $s^{-1}$ (Poise) | Viscosité @ 900 $s^{-1}$ (Poise) |
|---|---|---|---|
| Contrôle | 194,00 | 2,03 | 0,41 |
| 3 min vibration | 105,00 | 0,64 | 0,23 |
| % vs contrôle | -45,88 | -68,64 | -43,90 |
| 10 min vibration | 117,33 | 0,79 | 0,28 |
| % vs contrôle | -39,52 | -61,25 | -32,52 |

Cette composition est donc particulièrement sensible à la vibration (écarts supérieurs à 30% à 3 et 10 min de vibration) : sa viscosité se trouve significativement diminuée.

## Revendications

**1.** Ensemble de maquillage et/ou de soin des matières kératiniques, notamment des cils ou des sourcils comprenant:

- un récipient délimitant au moins un compartiment contenant au moins une composition de maquillage et/ou de soin desdites matières kératiniques ;
- au moins un applicateur comprenant un élément d'application pour appliquer la composition sur lesdites matières kératiniques ; et
- une source vibrante permettant, préalablement, simultanément ou postérieurement à l'application sur les matières kératiniques de la composition, de faire vibrer cette dernière,
- **caractérisé en ce que** ladite composition comprend des particules de forme plaquettaire.

**2.** Ensemble selon la revendication 1, **caractérisé en ce que** les particules de forme plaquettaire sont choisies parmi les argiles lipophiles et hydrophiles, le talc, le mica, le sulfate de baryum, le kaolin, la lauroyl-lysine, l'amidon, le nitrure de bore, le carbonate de calcium précipité, le carbonate et l'hydro-carbonate de magnésium, l'hydroxyapatite, la montmorillonite, les particules de cire de polytétrafluoroéthylène, le sulfate de calcium, la poudre de pierre ponce, l'oxychlorure de bismuth, la poudre d'oxychlorure de bismuth et d'oxyde de zinc, la perlite, les particules de verre notamment de taille d'environ 10 microns et d'épaisseur d'environ 0,4 microns ou de taille d'environ 25 microns et d'épaisseur d'environ 0,4 microns, les particules sol/gel de silice et dioxyde de titane, les particules de mica et de dioxyde de titane, les savons métalliques dérivés d'acides organiques carboxyliques ayant de 8 à 22 atomes de carbone, et en particulier de 12 à 18 atomes de carbone, et les particules réfléchissantes multicouches plaquettaires, en particulier les particules réfléchissantes comportant un substrat de mica synthétique revêtu de dioxyde de titane et/ou d'oxyde de fer noir, et leurs mélanges.

**3.** Ensemble de maquillage et/ou de soin des matières kératiniques, notamment des cils ou des sourcils comprenant:

- un récipient délimitant au moins un compartiment contenant au moins une composition de maquillage et/ou de soin desdites matières kératiniques ;
- au moins un applicateur comprenant un élément d'application pour appliquer la composition sur lesdites matières kératiniques ; et
- une source vibrante permettant, préalablement, simultanément ou postérieurement à l'application sur les matières kératiniques de la composition, de faire vibrer cette dernière,
- **caractérisé en ce que** ladite composition comprend au moins un tensioactif non ionique et/ou au moins un tensioactif ionique et/ou au moins un tensioactif polymérique.

**4.** Ensemble selon la revendication 3, **caractérisé en ce que** la composition comprend au moins un tensioactif non ionique.

**5.** Ensemble selon la revendication 3 ou 4, **caractérisé en ce que** la composition comprend au moins un tensioactif non ionique et au moins un tensioactif anionique.

**6.** Ensemble de maquillage et/ou de soin des matières kératiniques kératiniques, notamment des cils ou des sourcils comprenant:

- un récipient délimitant au moins un compartiment contenant au moins une composition de maquillage et/ou de soin desdites matières kératiniques ;
- au moins un applicateur comprenant un élément d'application pour appliquer la composition sur lesdites matières kératiniques ; et
- une source vibrante permettant, préalablement, simultanément ou postérieurement à l'application sur les matières kératiniques de la composition, de faire vibrer cette dernière,
- **caractérisé en ce que** ladite composition comprend au moins une huile structurée par au moins un agent rhéologique de phase grasse choisi parmi :

  • les polymères cristallins, de préférence choisis parmi les polymères semi-cristallins, les esters de dextrine et d'acide gras, les polysaccharides modifiés hydrophobes, les copolymères d'oléfines cristallins et les polycondensats cristallins ;
  • les agents structurants lipophiles minéraux, comme les argiles lipophiles et les silices hydrophobes,
  • les polymères de type polyamide lipophiles,
  • les polymères de type polyurées ou polyuréthanes lipophiles,
  • les polymères siliconés comprenant le cas échéant au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons,
  • les organogélateurs,
  • les polymères blocs,
  • les agents cristaux liquides cholestériques,
  • et leurs mélanges.

**7.** Ensemble selon la revendication 6, **caractérisé en ce que** la composition contient moins de 10% en poids de cire, de préférence moins de 5% en poids, de préférence est exempte de cire.

**8.** Ensemble de maquillage et/ou de soin des matières kératiniques, notamment des cils ou des sourcils comprenant:

- un récipient délimitant au moins un compartiment contenant au moins une composition de maquillage et/ou de soin desdites matières kératiniques ;
- au moins un applicateur comprenant un élément d'application pour appliquer la composition sur lesdites matières kératiniques ; et
- une source vibrante permettant, préalablement, simultanément ou postérieurement à l'application sur les matières kératiniques de la composition, de faire vibrer cette dernière,
- **caractérisé en ce que** ladite composition comprend au moins une huile structurée par au moins un agent rhéologique de phase grasse et moins de 10% en poids de cire, de préférence moins de 5% en poids, de préférence est exempte de cire.

**9.** Ensemble selon l'une des revendications 6 à 8, **caractérisé en ce que** l'agent rhéologique de phase grasse est choisi parmi les polymères semi-cristallins, les polyamides et les polymères siliconés comprenant au moins un motif hydrocarboné comportant deux groupes amides.

**10.** Ensemble selon l'une des revendications précédentes, **caractérisé en ce que** la source vibrante est couplée audit élément d'application de manière à permettre la vibration de ce dernier avant application de la composition sur les fibres, lors de l'application de la composition sur les fibres, ou après.

**11.** Ensemble selon l'une quelconque des revendications 1 à 10, **caractérisé par le fait que** l'élément d'application comporte notamment une brosse (10) ou un peigne (30).

**12.** Ensemble selon l'une quelconque des revendications 1 à 11, **caractérisé par le fait que** l'élément d'application est monté à l'extrémité d'une tige (11).

**13.** Ensemble selon l'une quelconque des revendications précédentes **caractérisé en ce que** la source vibrante génère des vibrations de fréquence supérieure ou égale à 20Hz.

**14.** Ensemble selon l'une quelconque des revendications 1 à 13 **caractérisé en ce que** les vibrations sont d'amplitude inférieure ou égale à 5 mm, de préférence inférieure ou égale à 3 mm.

**15.** Ensemble selon l'une des revendications 1 à 14 **caractérisé en ce que** le récipient est en matériau thermoplastique.

**16.** Ensemble selon l'une des revendications 1 à 14 **caractérisé en ce que** le récipient est en matériau non thermoplastique.

**17.** Procédé de maquillage et/ou de soin non thérapeutique des matières kératiniques, notamment des cils ou des sourcils, consistant à :

- appliquer sur lesdites matières kératiniques au moins une couche d'une composition de maquillage et/ou de soin,
- faire vibrer ladite composition préalablement, et/ou simultanément et/ou postérieurement à son application sur lesdites matières kératiniques,
- ledit procédé étant **caractérisé en ce que** la composition est conforme à la composition selon l'une des revendications 1 à 9.

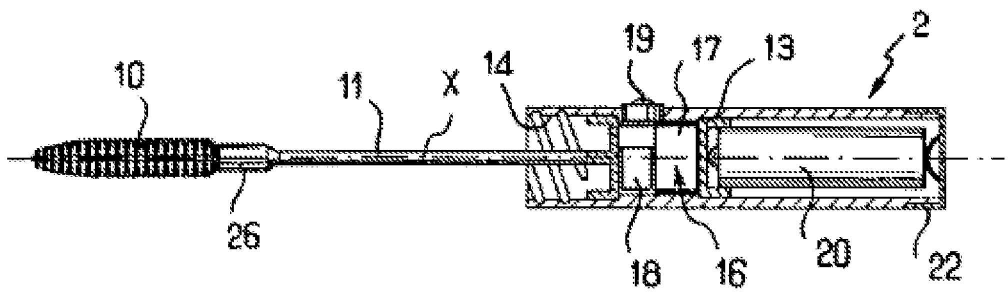


Fig.2

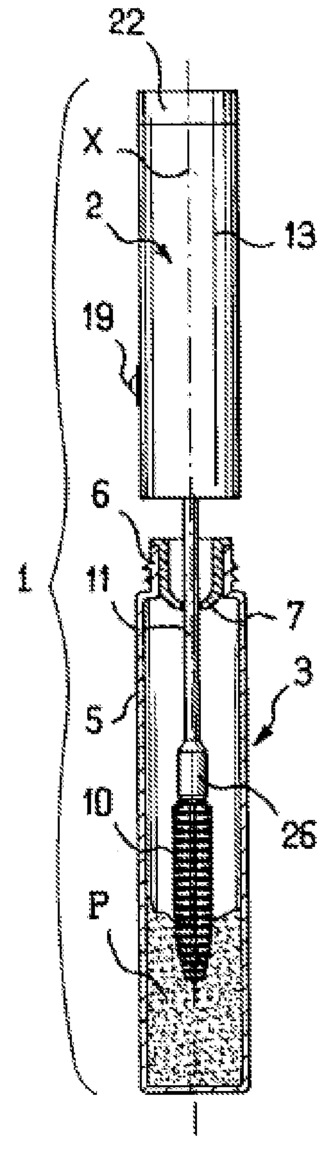


Fig.1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 20060032512 A
- EP 1307501 A
- EP 1355990 A
- EP 1355625 A
- FR 2856923
- EP 1493774 A
- WO 04006872 A
- US 6878754 B
- US 6689856 B
- EP 1407791 A
- EP 1416044 A
- FR 2788008
- WO 03008462 A
- FR 2694939
- EP 0629649 A
- US 6645476 B
- WO 9700275 A
- WO 9806438 A
- WO 982948 A
- WO 9848768 A
- WO 9850005 A
- WO 0007603 A
- WO 2076392 A
- FR 2820976
- WO 0035961 A
- WO 2032560 A
- EP 0692506 A
- US 6870012 B
- WO 03106536 A
- WO 0038651 A
- WO 0000222 A
- WO 0141735 A
- US 20030099709 A
- GB 2408510 A
- EP 692506 A
- FR 2840907 A
- US 2005175573 A
- US 5702717 A
- EP 0955039 A
- EP 0951897 A
- US 5156911 A
- WO 0119333 A
- US 5736125 A
- US 5519063 A
- EP 550745 A
- US 5783657 A
- US 3645705 A
- US 3148125 A
- US 5500209 A
- FR 2782723
- US 5874069 A
- US 5919441 A
- US 6051216 A
- US 5981680 A
- WO 2003106614 A
- EP 1068854 A
- EP 1086945 A
- WO 0247031 A
- WO 9323008 A
- FR 2910809
- EP 545409 A
- WO 94109086 A
- EP 709445 A
- GB 2282145 A
- GB 2276883 A
- WO 95132247 A
- WO 95132248 A
- EP 686674 A
- EP 711780 A
- US 5188899 A
- EP 0216479 A
- US 3915921 A
- US 4509949 A
- FR 2792190 A
- FR 2232303 A
- US 5162410 A
- WO 2004073626 A
- EP 749746 A
- WO 04055081 A
- FR 2792618
- FR 2919476
- WO 2006090343 A
- WO 2006020577 A
- WO 2006130644 A
- WO 2006130643 A
- WO 2006130642 A
- US 20070272269 A
- US 20030089379 A1
- US 6655390 B
- US 6814084 B
- US 6675814 B
- US 6581610 B
- US 6546937 B
- US 6539950 B
- US 6412496 B
- US 6343607 B
- US 4937326 A
- US 4922934 A

- US 6565276 B

**Littérature non-brevet citée dans la description**


- **S. Caillère ; S. Hénin ; M. Rautureau.** Minéralogie des argiles. Masson, 1982
- *J. Soc. Cosm. Chem.,* 1954, vol. 5, 249-256
- **KIRK-OTHMER.** Encyclopedia of Chemical Technology. WILEY, 1979, vol. 22, 333-432
- **D. HOURDET et al.** *Polymer,* 1994, vol. 35 (12), 2 624-2 630
- **F. L'ALLORET et al.** *Coll. Polym. Sci.,* 1995, vol. 273 (12), 1 163-1 173
- **F. L'ALLORET.** *l'Institut Français du Pétrole,* 1997, vol. 52 (2), 117-128
- **TAYLOR et al.** *Journal of Polymer Science, part A : Polymer Chemistry,* 1975, vol. 13, 2 551
- **J. BAILEY et al.** *Journal of Applied Polymer Science,* 1959, vol. 1, 56
- **HESKINS et al.** *Journal of Macromolecular Science, Chemistry A2,* 1968, vol. 8, 1441
- **S. Nojima.** Melting behavior of poly(ε-caprolactone)-block-polybutadiène copolymers. *Macromolécules,* 1999, vol. 32, 3727-3734
- **B. Boutevin et al.** Study ofmorphological and mechanical properties of PP/PBT. *Polymer Bulletin,* 1995, vol. 34, 117-123
- **P. Rangarajan et al.** Morphology of semi-crystalline block copolymers of ethylene-(ethylene-alt-propylene). *Macromolecules,* 1993, vol. 26, 4640-4645
- **P. Richter et al.** Polymer agregates with crystalline cores : the system poly(ethylene)-poly(ethylene-propylene). *Macromolécules,* 1997, vol. 30, 1053-1068
- **I.W. Hamley.** Cristallization in block copolymers. *Advances in Polymer Science,* 1999, vol. 148, 113-137
- **S. Bensason et al.** Elastomeric blends of homogeneous ethylene-octene copolymers (Mélanges élastomériques de copolymères homogènes éthylène-octène). *Polymer,* 1997, vol. 38 (15), 3913-19
- **S; Bensason et al.** Blends of homogeneous ethylene-octene copolymers (Mélanges de copolymères homogènes éthylène-octène). *Polymer,* 1997, vol. 38 (14), 3513-20
- Specialist Surfactants. 1997, 209-263
- Microemulsions Theory and Practice. Academic Press, 1977, 21-32